# EUROPEAN PATENT SPECIFICATION

(11) **EP 3 411 394 B1**
(45) Date of publication and mention of the grant of the patent: **18.11.2026**
(21) Application number: 17702860.2
(22) Date of filing: 01.02.2017
(51) Int. Cl.: C07K 14/705, C07K 16/32, A61K 39/00

(54) **ENGINEERED ANTIGEN PRESENTING CELLS AND USES THEREOF**
MANIPULIERTE ANTIGEN-PRÄSENTIERENDE ZELLEN UND DEREN VERWENDUNG
CELLULES PRÉSENTANT DES ANTIGÈNES MODIFIÉS ET LEURS UTILISATIONS

(30) Priority: 02.02.2016 EP 16153966
(43) Date of publication of application: 12.12.2018
(73) Proprietor: Ecole Polytechnique Fédérale de Lausanne (EPFL), 1015 Lausanne (CH)
(72) Inventor: DE PALMA, Michele, 1003 Lausanne (CH); SQUADRITO, Mario Leonardo, 20126 Milano (MI) (IT)
(74) Representative: reuteler & cie SA
(86) International application number: PCT/EP2017/052145
(87) International publication number: WO 2017/134100

(56) References cited:
- YANFANG LIU ET AL: "The exosomes in tumor immunity", ONCOIMMUNOLOGY, vol. 4, no. 9, 2 April 2015 (2015-04-02), pages e1027472, XP055354756, DOI: 10.1080/2162402X.2015.1027472
- ZACHARY C HARTMAN ET AL: "Increasing vaccine potency through exosome antigen targeting", VACCINE, ELSEVIER, AMSTERDAM, NL, vol. 29, no. 50, 30 September 2011 (2011-09-30), pages 9361 - 9367, XP028107148, ISSN: 0264-410X, [retrieved on 20111005], DOI: 10.1016/J.VACCINE.2011.09.133
- PAUL D. ROBBINS ET AL: "Regulation of immune responses by extracellular vesicles", THE JOURNAL OF IMMUNOLOGY, vol. 14, no. 3, 25 February 2014 (2014-02-25), GB, pages 195 - 208, XP055324647, ISSN: 1474-1733, DOI: 10.1038/nri3622
- KARSTEN MAHNKE ET AL: "The Dendritic Cell Receptor for Endocytosis, DEC-205, Can Recycle andEnhance Antigen Presentation via Major Histocompatibility ComplexClass II-positive Lysosomal Compartments", THE JOURNAL OF CELL BIOLOGY, 30 October 2000 (2000-10-30), pages 21 - 9525, XP055266759, Retrieved from the Internet <URL:http://www.ncbi.nlm.nih.gov/pmc/articles/PMC2185598/pdf/0007035.pdf> [retrieved on 20160418]
- KURT SCHÖNFELD ET AL: "Selective Inhibition of Tumor Growth by Clonal NK Cells Expressing an ErbB2/HER2-Specific Chimeric Antigen Receptor", MOLECULAR THERAPY, vol. 23, no. 2, 6 November 2014 (2014-11-06), GB, pages 330 - 338, XP055265087, ISSN: 1525-0016, DOI: 10.1038/mt.2014.219
- CLOTILDE THÉRY ET AL: "Membrane vesicles as conveyors of immune responses", NATURE REVIEWS IMMUNOLOGY, vol. 9, no. 8, 1 August 2009 (2009-08-01), pages 581 - 593, XP055018445, ISSN: 1474-1733, DOI: 10.1038/nri2567
- LAURA ANN MULCAHY ET AL: "Routes and mechanisms of extracellular vesicle uptake", JOURNAL OF EXTRACELLULAR VESICLES, vol. 11, no. 0, 4 August 2014 (2014-08-04), pages 1093, XP055266760, DOI: 10.1016/j.exphem.2010.01.002
- MARÍA YÁÑEZ-MÓ ET AL: "Biological properties of extracellular vesicles and their physiological functions", JOURNAL OF EXTRACELLULAR VESICLES, vol. 10, no. 0, 14 May 2015 (2015-05-14), pages 261, XP055266766, DOI: 10.3402/jev.v4.27066

## Description

### Field of the Invention

The present invention relates to a vaccine comprising cells expressing extra-cellular vesicle internalizing receptors and their use in the immunotherapy for prevention and/or treatment of cancers.

### Background of the Invention

Immunotherapy is gaining increasing importance for the treatment and prevention of various human diseases including infections, inflammatory and degenerative conditions, and cancers.

In cancer, immunotherapy includes stimulating the patient's own immune system to attack cancer cells or other cellular components of the tumor (Waldmann et al., 2003, Nat Med, 9: 269-277*;* Miller et al., 2015, Cancer Cell, 27(4):439-49*).* The main types of immunotherapy now being used to treat cancer include use of monoclonal antibodies, immune checkpoint inhibitors, and cancer vaccines (*Miller et al., 2015, supra*).

In cancer-related applications, immunotherapy is based on the assumption that cancer cells express on their surface molecules that are not expressed (or are expressed at lower levels) by normal cells and that can be detected by the immune system. These molecules are known as cancer antigens and are often proteins (normal or mutated) or other macromolecules such as carbohydrates and lipids. In the last decade, an increasing number of molecules derived from the processing of tumor proteins have been identified and classified as tumor-associated antigens (TAAs). So-called active immunotherapy is used to engage the immune system into attacking the tumor cells by targeting TAAs. TAAs can be recognized by different cell types including CD8+ cytotoxic T lymphocytes (CTLs). After cancer-cell recognition by CTLs, these can subsequently engage in eliminating cancer cells.

One of the main goals of current research on immunotherapy approaches is to elicit or enhance cancer-specific CTLs by vaccinating the patient against potential or known TAAs. Approaches developed and transferred to clinical trials include adoptive immunotherapy with *ex vivo* TAA-loaded antigen presenting cells (APCs), hereon referred to as APC-TAA. Indeed, the generation of potent and persisting anti-tumor immunity requires the presentation of TAAs by professional APCs, such as dendritic cells (DCs), to the CTLs (Palucka et al, 2012, Nat Rev Cancer, 12(4):265-77*).* DCs are generated *ex vivo* by culturing hematopoietic progenitors or monocytes, obtained from the patient, with specific cytokine/growth factor combinations. Once generated, the DCs are exposed to total tumor lysates or specific TAAs *ex vivo,* before reinfusion of the APC-TAA in the patient. During the past decade, preclinical studies in mice and several clinical trials have shown the safety of the procedure, its ability to induce the expansion of circulating CD4⁺ T cells and CD8⁺ T cells that are specific for tumor antigens, and objective clinical responses in some patients (*Palucka et al., 2012, supra*). Currently, only one APC-TAA vaccine, Sipuleucel-T (Provenge^{®}), is approved in the US to treat advanced prostate cancer that is no longer being helped by hormone therapy (Gardner et al., 2012, Hum Vaccin Immunother, 8(4): 534-9*).* This example demonstrates the feasibility of active immunization for the treatment of established cancer.

However, the use of *ex vivo* cultured APC-TAA is typically labour-intensive (the APCs need to be isolated, manipulated *ex vivo,* and then re-infused), expensive, and is generally not individualized to each patient (Kastenmueller et al., 2014, Nat Rev Immunol., 14(10):705-11). An alternative vaccination approach consists of targeting a selected TAA to APCs *in vivo,* without *ex vivo* manipulation (*Palucka et al., 2012, supra; Kastenmueller et al., 2014, supra*). This can be achieved by several means, e.g., by using chimeric proteins that are comprised of an antibody, specific for an APC receptor (e.g. CD205, CLEC9A, CD11c), that is also fused to a selected TAA. The chimeric protein can target APCs and promote TAA internalization, processing, and presentation by the APC to the T cells. However, these approaches are not very efficient and only one TAA can be delivered. Furthermore, the induction of CTLs by the targeted APCs also requires the provision of DC maturation and/or activation signals, as the absence thereof can induce antigen-specific tolerance (*Kastenmueller et al., 2014, supra*).

In one approach, DCs were exposed *ex vivo* to cancer cell lysates or extracellular vesicles (EVs) and then inoculated back into the subject to activate antigen-specific T cells and induce anti-tumor immune responses (Gu et al., 2015, Intern Journal of Cancer, 136, E74-84). Another approach used monocytes/macrophages engineered to express an anticarcinoembryonic antigen (CEA) chimeric antigen receptor (CAR), or DCs engineered to express an anti-HER2 (human epidermal growth factor receptor 2) CAR to directly target and lyse cancer cells *in vitro* and *in vivo (*Biglari et al., 2006, Gene therapy, 13: 602-610*;* Wei et al., 2008, Cancer research, 68: 3854-62*).* In a different type of approach, CTLs or natural killer (NK) cells were engineered to express a CAR designed against a specific TAA. The CAR-engineered T or NK cells then recognized cancer cells that expressed the specific antigen and killed them (Ahmed et al., 2015, Journal of clinical oncology, 33, 1688-1696*;* Schonfeld et al., 2015, Molecular therapy, 23, 330-338). However, the efficacy of the aforementioned approaches is limited by the ability of the engineered cells to traffic to solid tumors once re-infused.

Therefore, there is still a need for developing anti-tumor vaccines able to induce strong and broad T-cell responses that are specific for multiple known and unknown TAAs on a personalized manner, but at the same time applicable to a broad range of patients.

### Summary of the Invention

The invention is based on the design of new Extra-cellular Vesicle Internalizing Receptors (EVIRs) directed against a surface molecule expressed by cancer cells, and the genetic engineering of APCs such as monocytes, macrophages, DCs, or B cells, in order to stably express those new EVIRs. The invention is further based on the observation that APCs engineered to express an EVIR of the present invention efficiently take and/or internalize extra-cellular vesicles (EVs), comprising any cancer-cell derived particles or membranes, that are derived from surface molecule-positive cancer cells but not surface molecule-negative cells, independent of cell contact. It was further observed that EVs uptake by the EVIR-expressing APCs advantageously enhances the presentation of TAAs that are unrelated to the selected surface molecule but are related to the cancer cell of origin, and promotes the expansion of TAA-specific T cells, thereby offering a promising tool for cancer treatment or diagnosis.

One aspect of the invention relates to a recombinant EVIR directed against at least one cancer-cell surface molecule.

Another aspect of the invention relates to an isolated nucleic acid molecule encoding an EVIR according to the invention.

In another aspect, the invention provides a recombinant vector comprising a nucleic acid molecule encoding an EVIR according to the invention.

Another aspect of the invention relates to an isolated cell expressing at least one EVIR of the invention, in particular an APC and compositions thereof.*

Another aspect of the invention provides an *ex vivo* method (i.e., in culture) of inducing expression of at least one EVIR of the invention in an APC or a stem/progenitor cell thereof comprising the steps of:
(i) *ex vivo* transducing said cell with a vector according to the invention; and
(ii) optionally inducing APC differentiation, maturation or activation.

Another aspect of the invention provides an *ex vivo* method of preparing EVIR-expressing, TAAs-presenting cells, comprising the steps of:
(i) providing at least one cancer cell or at least one cancer cell-derived EV obtained from a cancer subject;
   * The invention is as defined in any one of claims 1 to 25.
(ii) providing an EVIR-expressing cell of the invention;
(iii) contacting, *ex vivo,* an EVIR-expressing cell provided under (ii) with said at least one cancer cell or EV provided under (i);
(iv) collecting cells obtained in step (iii);
wherein cells obtained under (iv) have an enhanced ability to present TAAs from said cancer subject as compared to a cell not expressing an EVIR and treated as in (iii), once administered to said subject.

Another aspect of the invention relates to an isolated EVIR-expressing cell, or an EVIR-expressing TAAs-presenting cell obtainable by a method according to the invention.

Another aspect of the invention relates to an EVIR-expressing cell, an EVIR-expressing TAAs-presenting cell or a recombinant vector according to the invention for use as a medicament.

Another aspect of the invention provides a pharmaceutical composition comprising cells of the invention or at least one recombinant vector according to the invention and at least one pharmaceutically acceptable carrier, diluent or excipient thereof.

Another aspect of the invention relates to an EVIR-expressing cell, an EVIR-expressing TAAs-presenting cell or a recombinant vector according to the invention for use in the prevention and/or treatment of a cancer.

Another aspect of the invention provides a kit for carrying out methods according to the invention comprising at least one EVIR, or at least one recombinant expression vector, or at least one EVIR-expressing cell according to the invention.

### Description of the figures

Statistical p values were calculated in Prism (GraphPad Software) and are indicated in the figures as follows. * p < 0.05, ** p < 0.01, *** p < 0.001.

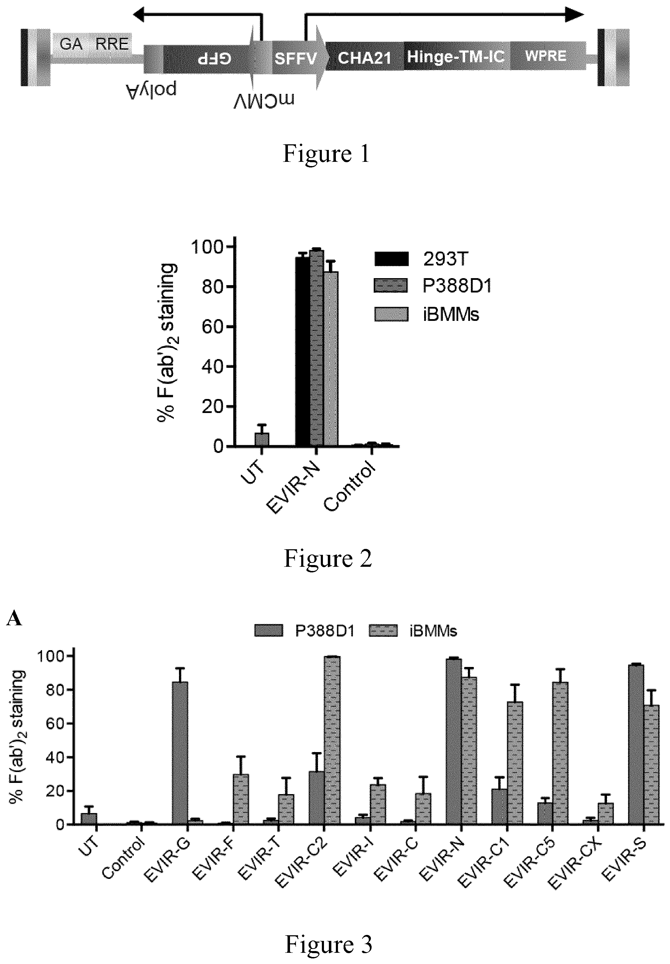

**Figure 1****. EVIR-expressing LV.** Schematic representation of the bidirectional proviral LV used to simultaneously express a representative EVIR (e.g., anti-HER2) and a second gene, in this case, Green Fluorescent Protein (GFP).
**Figure 2****. Expression of EVIRs after cell transduction.** Anti-HER2 EVIR-N expression measured as the percentage of F(ab')2-positive cells (mean ± SEM, n=5) analyzed by flow cytometry (anti-F(ab')2 staining) in 293T, P388D1 and iBMMs, either untransduced (UT; F(ab')2-positive cells are undetectable, and signal from P388D1 cells represents noise) or transduced with control or EVIR-N LVs (design described in Example 1).

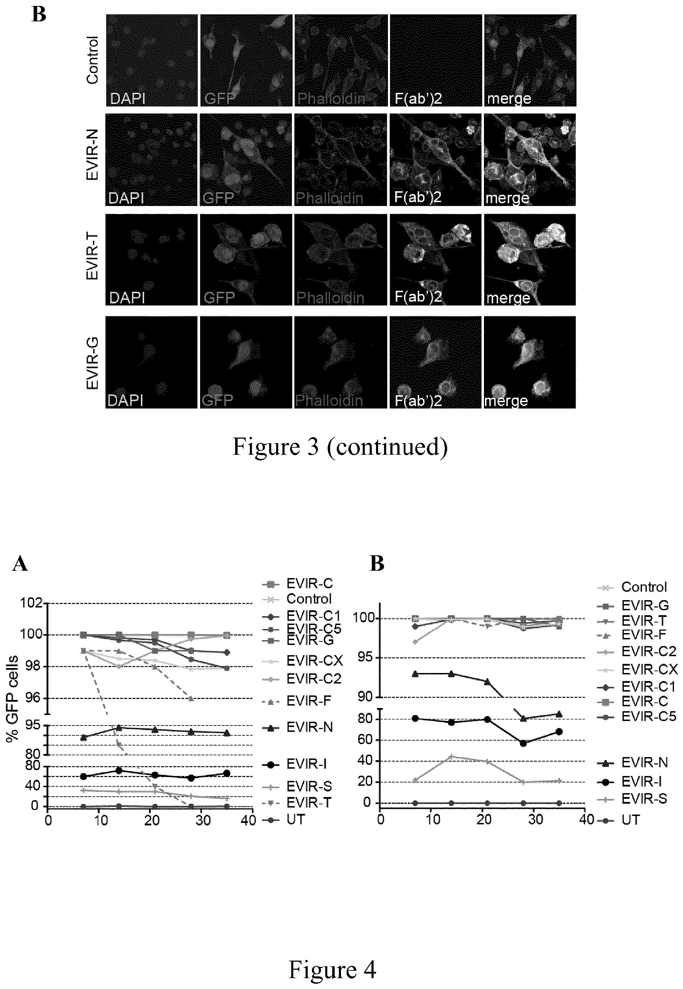

**Figure 3****. Expression of EVIRs with different signal domains after cell transduction. A:** Anti-HER2 EVIR expression measured as the percentage of F(ab')2-positive cells (mean ± SEM, n=5) analysed by flow cytometry (anti-F(ab')2 staining) in P388D1 and iBMMs. **B:** Representative images of immunofluorescence analysis of iBMMs transduced with control, EVIR-N, EVIR-T or EVIR-G LVs, stained with anti-F(ab')₂ antibody, as described in Example 2.
**Figure 4****. Persistence of EVIR-expressing cells.** Data show the percentage of GFP-positive cells (**A**: iBMMs, **B**: P388D1) over a period of 36 days from transduction with control or anti-HER2 EVIR LVs, measured by flow cytometry as described in Example 2.

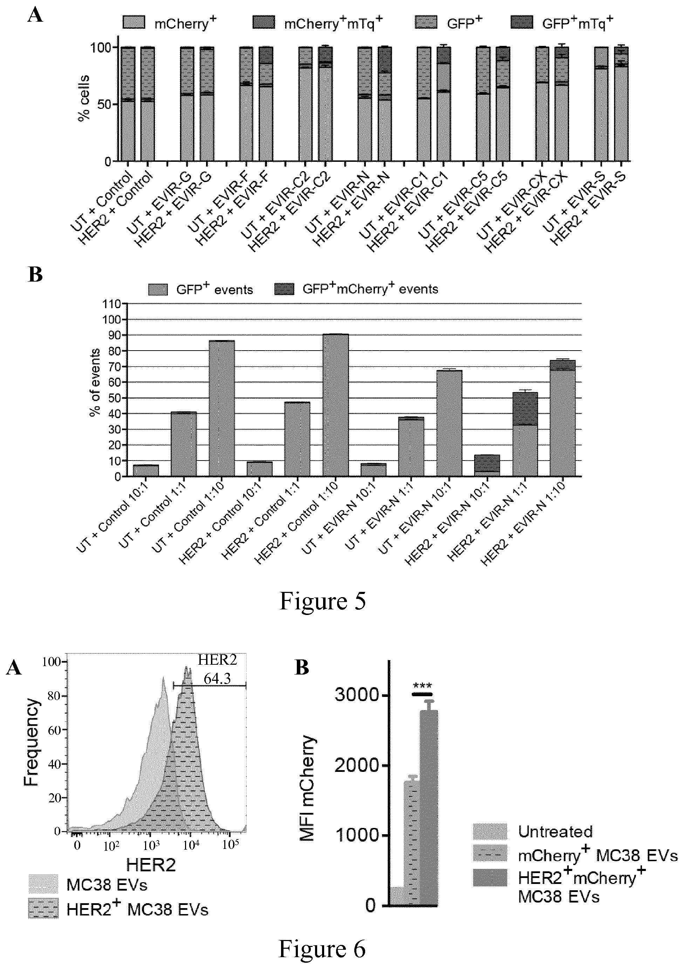

**Figure 5****. Co-culture assays. A:** Percentage (mean ± SEM, n=3) of mCherry⁺, GFP⁺, GFP⁺mTq⁺ or mCherry⁺mTq⁺ iBMMs within total iBMMs (indicated as 100%), measured by flow cytometry analysis of co-cultures of HER2⁺ or HER2⁻ (untransduced, UT) mTq+ MC38 cells. iBMMs were either transduced (EVIR) or not (control) with the anti-HER2 EVIR-expressing LVs, as described in Example 3. mCherry+mTq+ and GFP+mTq+ indicate events positive for mCherry and mTq, or GFP and mTq, which represent iBMMs binding to MC38 cells. **B:** Percentage (mean ± SEM, n=3) of binding events between EVIR-N or Controltransduced P388D1 cells and HER2-transduced or UT mCherry+ MC38 cells, at different ratios (10:1, 1:1 or 1:10).

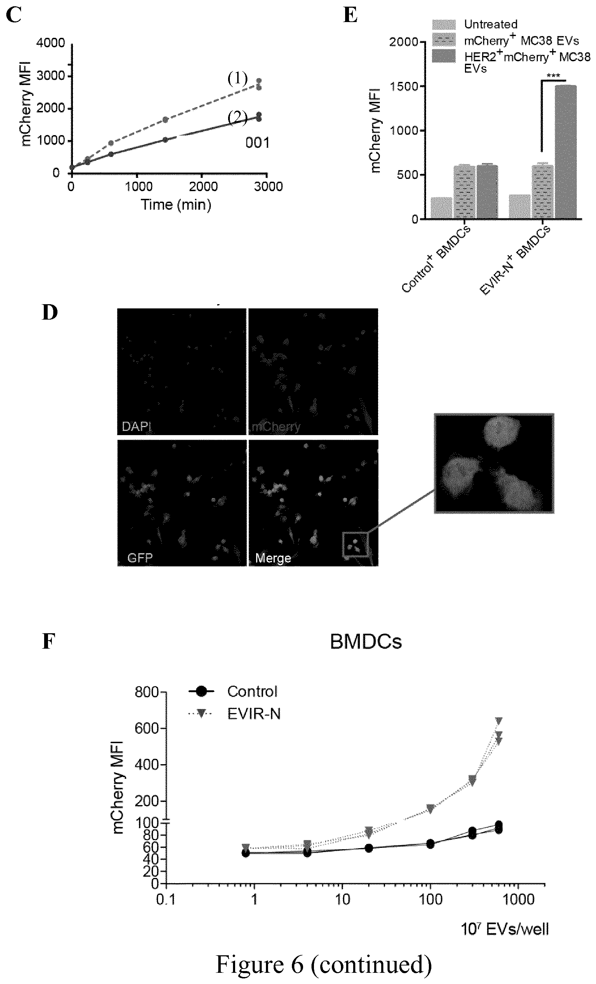

**Figure 6****. EV uptake by anti-HER2 EVIRs. A:** Representative histogram of flow cytometry analysis of EVs derived from HER2⁺ MC38 cells stained with an anti-HER2 antibody, as described in Example 4. **B:** Flow cytometry analysis of mCherry expression in anti-HER2 EVIR-N⁺ iBMMs untreated or treated with EVs isolated from HER2⁺ or HER2⁻ (UT) mCherry+ MC38 cells (median fluorescence intensity, MFI; mean ± SEM, n=2). Statistical analysis by one-way ANOVA with Tukey's multiple comparison test. **C:** Time course analysis of the MFI of mCherry in iBMMs expressing an anti-HER2 EVIR and treated with HER2⁺/mCherry⁺ MC38-derived EVs (1) or EVs isolated from HER2⁻/mCherry+ MC38 cells (2); p<0.001, by two-way ANOVA statistical analysis; **D:** Immunofluorescence imaging of anti-HER2 EVIR-N⁺ iBMMs treated with EVs isolated from HER2⁺/mCherry⁺ MC38 cells. **E:** mCherry MFI of BMDCs, either expressing a control EVIR (Control⁺ BMDCs) or an anti-HER2 EVIR (EVIR-N⁺ BMDCs), untreated or treated with EVs from HER2+/mCherry+ or HER2⁻/mCherry+ (indicated as mCherry+) MC38 cells. Statistical analysis by two-way ANOVA with Tukey's multiple comparison test. **F:** Flow cytometry analysis of mCherry fluorescence in anti-HER2 EVIR-N⁺ or Control EVIR⁺ BMDCs treated with increasing concentrations of EVs isolated from HER2⁺ mCherry+ MC38 cells (mean ± SEM, n=3).

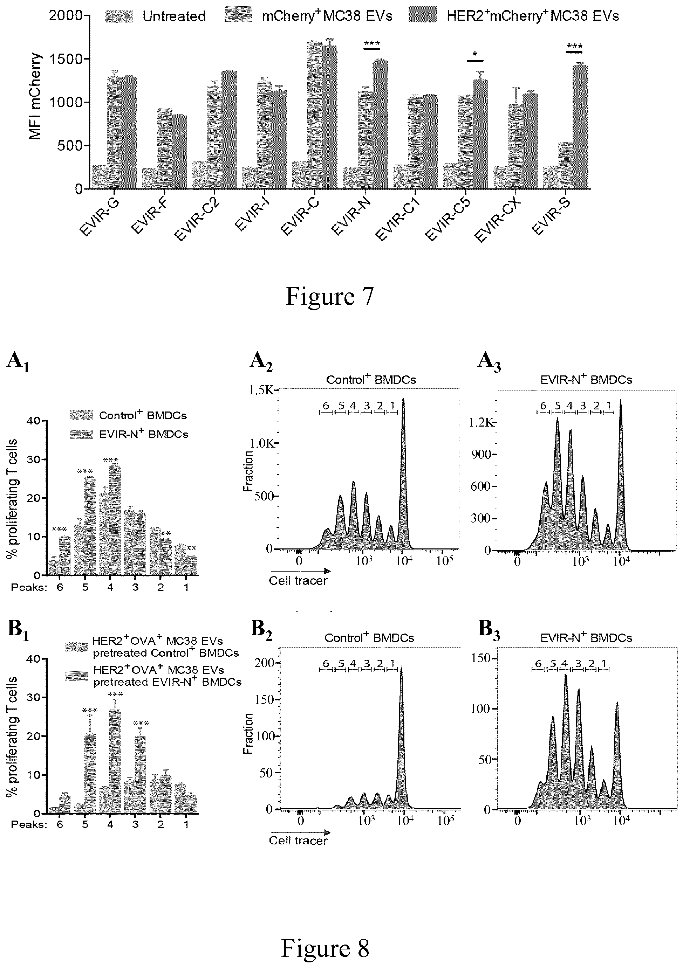

**Figure 7****. EVIRs with different intracellular signaling domains.** Cytometry analysis of mCherry MFI (mean ± SEM, n=2) of anti-HER2 EVIR-transduced iBMMs untreated or treated with EVs isolated from HER2+ or HER2⁻ (UT) mCherry+ MC38 cells as described in Example 4. Statistical analysis by two-way ANOVA with Tukey's multiple comparison test.

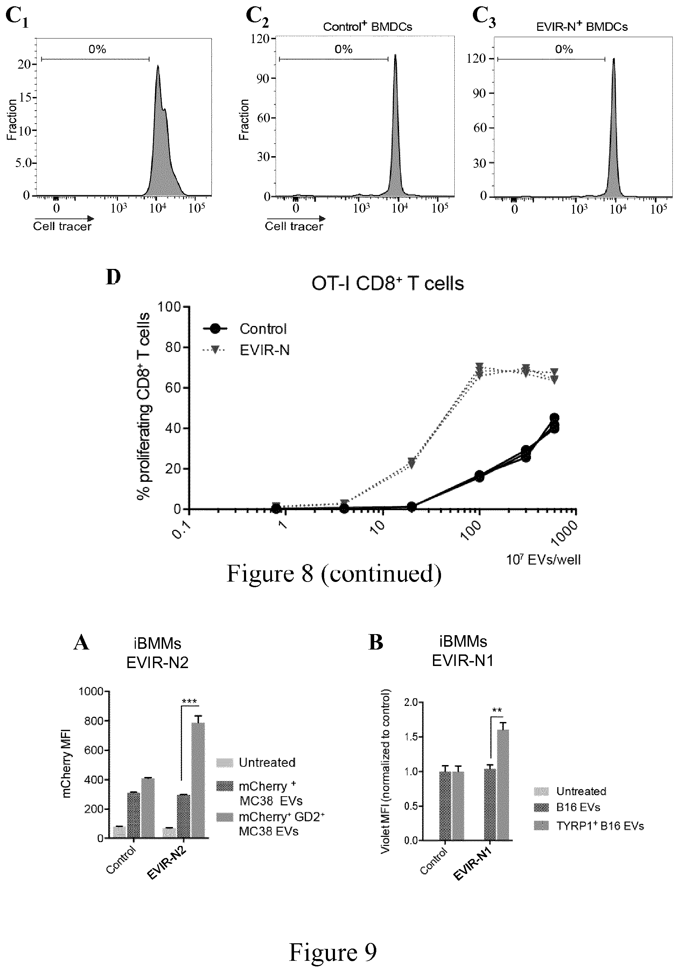

**Figure 8****. T cell proliferation assays** as described in Example 5. **A₁:** percentage of proliferating OT-I T cells labeled with cell tracer to measure the number of proliferation cycles ("peaks" in **A₂₋₃),** after culture with BMDCs either expressing a control EVIR (control⁺) or an anti-HER2 EVIR-N (EVIR-N⁺), in the presence of EVs isolated from HER2⁺OVA⁺ MC38 cells. The percentage of proliferating T cells was obtained by analyzing each "peak" (1-6) in Figures **A₂₋₃,** wherein each peak is indicative of one cell division. **B₁:** percentage of proliferating OT-I T cells labeled with cell tracer and cultured with BMDCs either expressing a control EVIR (control⁺) or an anti-HER2 EVIR-N (EVIR-N⁺), pre-treated for 24 h with EVs isolated from HER2⁺OVA⁺ MC38 cells (**A₁-B₁:** two-way ANOVA statistical analysis with Sidak post-test correction for multiple comparisons (mean ± SEM, n=3). **A₂₋₃:** representative histograms showing T cell proliferation cycles after culture with control+ **(A₂)** or anti-HER2 EVIR-N+ **(A₃)** BMDCs together with EVs; **B₂₋₃:** representative histograms showing T cell proliferation cycles after culture with control+ **(B₂)** or anti-HER2 EVIR-N+ **(B₃)** BMDCs pre-treated with EVs; Statistical analysis in A₁ and B₁ by two-way ANOVA with Sidak's multiple comparison test. **C₁₋₃:** representative histograms showing T cell proliferation after their exposure to HER2⁺OVA⁺ EVs **(C₁),** control+ BMDCs (C₂) or anti-HER2 EVIR-N+ BMDMs **(C₃)** without EVs. **D:** Proliferation of CD8⁺ OT-I T cells labeled with cell tracer after culture with BMDCs either expressing a control or an anti-HER2 EVIR-N, and treated with increasing concentrations of EVs isolated from HER2⁺ OVA⁺ MC38 cells. Data-points indicate 3 independent biological replicates.

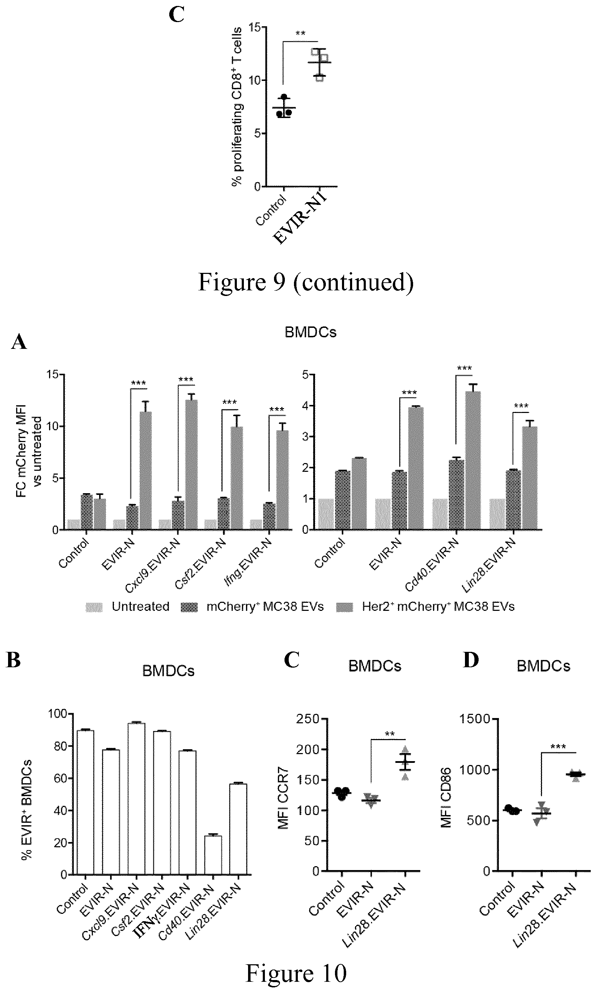

**Figure 9****. EVIRs directed against two distinct melanoma-specific surface antigens, DG2 and TYRP1** as described in Example 6. **A:** mCherry MFI of Control⁺ or EVIR-N2⁺ iBMMs (mean ± SEM, n=3) untreated or treated with EVs from GD2⁺/mCherry⁺ or GD2 /mCherry⁺ MC38 cells. **B:** MFI of CellTracker TM Blue in Control⁺ or EVIR-N1 iBMMs (mean ± SEM, n=3) untreated or treated with EVs isolated from TYRP1⁺ or TYRP1⁻ B16 melanoma cells. Statistical analysis by two-way ANOVA with Tukey's multiple comparison test. **C:** Number of proliferating CD8⁺ OT-I T cells labeled with cell tracer after culture with BMDCs either expressing a Control or an EVIR-N1 and treated with EVs isolated from TYRP1⁺ OVA⁺ B16 melanoma cells (mean ± SEM, n=3). Statistical analysis by Student's t test.
**Figure 10****. Transduction by bidirectional LVs of EVIRs together with a factor favoring APC differentiation, activation, and presentation, and/or T-cell recruitment** as described in Example 7. **A:** mCherry uptake of EVs isolated from HER2⁺ or HER2⁻ mCherry⁺ MC38 cells by EVIR⁺ BMDCs after cell transduction with LVs encoding a Control EVIR alone, an EVIR-N alone, or a bidirectional LV encoding both the EVIR-N and one of the indicated proteins (CXCL9, CSF2, IFNy, CD40, LIN28). Data represent the mean of 3 transduction replicates per EVIR (biological replicates; mean ± SEM, n=3). Statistical analysis by one-way ANOVA with Tukey's multiple comparison test. **B:** Expression of EVIR⁺ in BMDCs after transduction of cells with LVs encoding a Control EVIR alone, an EVIR-N alone, or EVIR-N along with one of the indicated proteins (CXCL9, CSF2, IFNy, CD40, LIN28). Data represent technical replicate (mean ± SEM, n=3). **C-D:** Expression of CCR7 (C) or CD86 (D) in BMDCs (mean ± SEM, n=3) transduced with Control EVIR alone, EVIR-N alone, or EVIR-N with LIN28. Data represent the mean of 3 transduction replicates per EVIR (biological replicates; mean ± SEM, n=3). Statistical analysis by one-way ANOVA with Tukey's multiple comparison test.

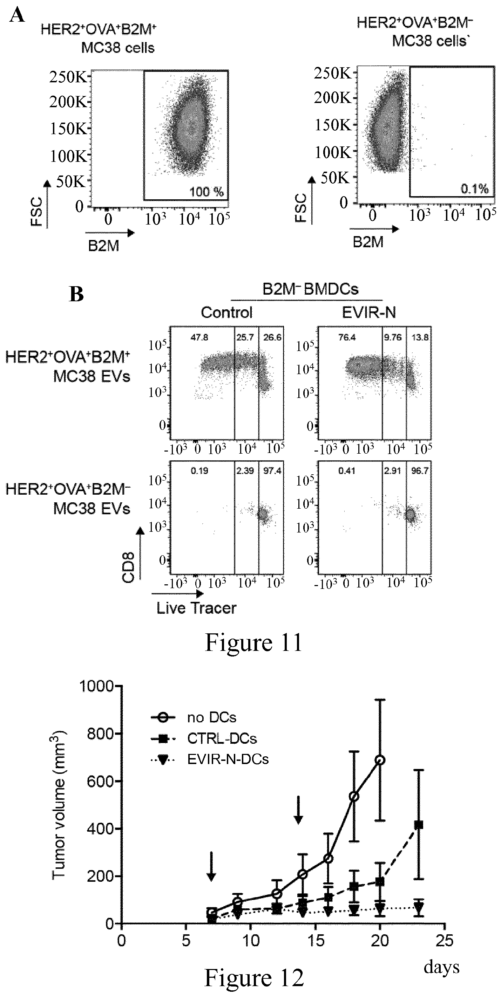

**Figure 11****. T-cell proliferation assays** as described in Example 11. **A₁₋₂:** Data show flow cytometry analysis of the indicated MC38 cells in which the B2M gene was either intact **(A₁)** or disrupted by CRISPR transduction **(A₂). B:** Data show flow cytometry analysis of CD8⁺ OT-I T cells labeled with cell tracer after culture with BMDCs isolated from MHCI-deficient B2M⁻ mice and expressing either a Control EVIR or EVIR-N. Cultures were treated with EVs isolated from either B2M- proficient (B2M⁺) or deficient (B2M⁻) HER2⁺ OVA⁺ MC38 cancer cells.
**Figure 12****. Tumor vaccination study** as described in Example 12. HER2⁺ MC38 tumor growth in syngeneic mice injected subcutaneously with PBS (no DCs; n=4) or vaccinated with either DCs expressing a Control EVIR (CTRL DCs; n=7) or an anti-HER2 EVIR (EVIR-DCs; n=9) at day 7 and 14 post-MC38 tumor injection.

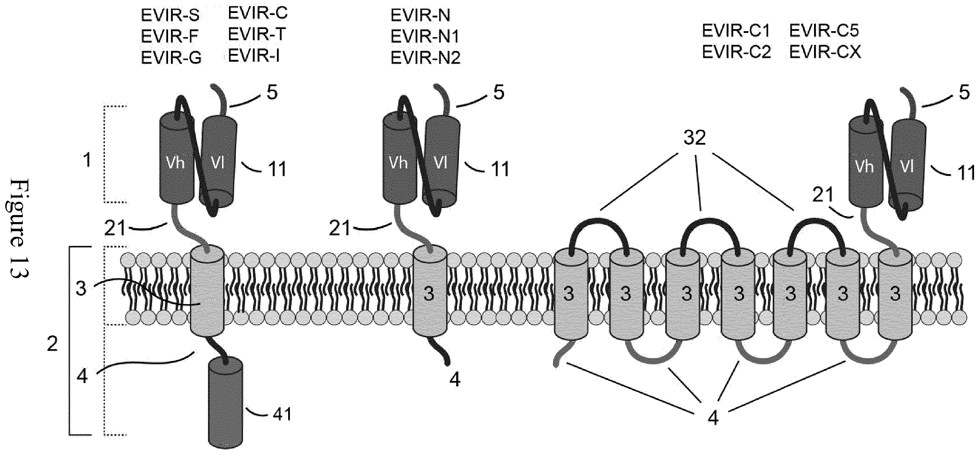

**Figure 13****.** The illustration represents schematically and generically some examples of constructs for the EVIR of the invention comprising an extracellular antibody domain specific for a membrane-associated molecule of a cancer cell (1) such as a scFv (11), a proteinic domain (2) comprising at least one transmembrane domain (3) and at least one intracellular domain (4). The proteinic domain may comprise a hinge domain (21) connecting the transmembrane domain (3) to the extracellular antibody domain (1). The proteinic domain (2) can be varied to either comprise one transmembrane domain (3) and one intracellular domain (4) such as the example represented on the left and the middle or more than one of each, wherein the transmembrane domains (3) are linked together on the extracellular side by extracellular non-hinge domains (32) and by the more than one intracellular domains (4) on the intracellular side, as in the example represented on the right. Further, the EVIR comprises a cell membrane export domain (5) linked to the extracellular antibody domain (1). The intracellular domain (4) may also comprise a signalling sequence (41). The specific names of the constructs of the invention are indicating under which type of constructs of the invention those are falling.

### Detailed description

The term "antibody" as referred to herein designates a polypeptide that binds to an antigen. This includes whole antibodies and any antigen binding fragments. The term "antibody" is used in its broadest sense and includes monoclonal antibodies, polyclonal antibodies, human antibodies, humanized antibodies, chimeric antibodies and the like as long as the characteristic properties of the invention are retained, in particular the ability of binding to the target antigen, more specifically to the membrane-associated molecules of cancer cells. Examples of antibodies and fragments thereof include a variable domain fragment ("Fv", consisting of the VH and VL domains of a single arm of an antibody), Fab fragment (monovalent fragment consisting of the VH, VL, CH1 and CL domains), Fab2 fragment (bivalent), Fab3 fragment (trivalent), Fab' fragment (Fab with hinge region), F(ab')2 fragment (bivalent fragment including two Fab fragments linked by a disulfide bridge at the hinge region), Fd fragment (consisting of the VH and CH1 domains), rIgG (reduced IgG or half-IgG), diabodies, triabodies, tetrabodies, minibodies, monovalent antibodies, divalent or multivalent antibodies comprising a fragment of more than one antibody, single chain variable fragment (ScFv), bis-scFv (bispecific), and derivatives of antibodies such as disulfide stabilized Fv fragments, CDR-comprising peptides, as well as epitope-binding fragments of any of the above (Holliger et al., 2005, Nature Biotechnology, 23(9): 1126-1136).

The term "a membrane-associated molecule" or "surface molecule" as used herein refers to any molecule that is physically embedded in the lipid bilayer or bound or anchored to a cell membrane permanently or transiently under specific conditions. The molecule may be associated with any membrane of the cancer cell, including the plasma membrane or intracellular membranes. These molecules could perform a variety of functions and belong to different functional groups including, but not limited to glycoproteins, membrane receptor proteins, transport proteins, membrane enzymes, cell adhesion molecules, and their mutated forms. These molecules can be expressed either on the cancer cell's plasma membrane or any membrane associated with cancer-cell derived particles, such as extra-cellular vesicles (EVs). The term includes known and unknown cancer cell membrane-associated molecules. Examples of membrane-associated molecules include, but are not limited to, human epidermal growth factor receptor 2 (HER2), tyrosinase-related protein-1 (TYRP1), carcinoembryonic antigen (CEA), mesothelin, PMEL (gp100), gangliosides (GD2, GD3), and mucins.

The term "extra-cellular vesicle internalizing receptor" or "EVIR" refers to a recombinant receptor directed against a surface molecule expressed by a cancer cell or any cancer-cell derived particle/vesicle. An EVIR according to the invention comprises the following elements that are referred to as "an extracellular antibody domain", "proteinic domain" and optionally "a domain to increase EVIR export to the cellular membrane".

The term "extracellular antibody domain" refers to any antibody domain with specificity for any membrane-associated molecule expressed by a cancer cell or cancer-cell derived particle/vesicle. Examples of antibody domains according the invention include, but are not limited to: **(i)** anti-HER2 scFv, such as CHA21 (Zhou et al., 2011, The Journal of Biological Chemistry, 286: 31676-31683*),* a trastuzumab-based scFv (Morgan et al., 2010, Mol Ther., 18(4):843-51*),* a pertuzumab-based scFv *(*Franklin et al., 2004, Cancer Cell, 5(4):317-28) and a FRP5-based scFv *(*Ahmed et al., 2009, Mol Ther., 17(10):1779-87*);* **(ii)** anti-GD2 scFv *(*Newik et al., 2016, Mol Ther Oncolytics, 68:139-152*);* or **(iii)** anti-TYRP1 scFv (Saenger et al., 2008, Cancer Res, 68(23); 9884-91); among others.

The terms "transmembrane domain" and "intracellular domain" refer to portions of the protein fragments of the EVIR of the invention comprising polypeptides that anchor the "extracellular antibody domain" of the EVIR to the cell surface and extend to the cell cytoplasm. According to a particular aspect, the transmembrane domain of the EVIR according to the invention can allow the EVIR to anchor to the antigen presenting cell membrane. According to a particular aspect, the intracellular domain of the EVIR according to the invention can have a signalling or non-signalling capacity.

In particular, those "transmembrane domains" and "intracellular domains" forming the proteinic domain can be the corresponding domains from the native membrane associated protein they are derived from or domains that are derived from those through some truncations and/or homologous sequence modifications that would not affect their anchoring ability to the cell membranes, such as for example the removal of the endogeneous signal peptides. For example, according to a particular aspect, the proteinic domain may comprise more than one of transmembrane domains that are linked together by extracellular "non hinge" domains (peptidic fragments that bridges two "transmembrane domains" in the native membrane protein), such as found in certain membrane proteins such as CCR1, -2, -4 and -5. When the intracellular domain of the EVIR has a signalling ability (e.g. comprising a signalling peptidic portion) it may be referred as a "signalling domain" and when the intracellular domain of the EVIR does not have a signalling capacity, it may be referred as "an inert intracellular domain". According to a particular aspect, the proteinic domain may comprise more than one of transmembrane domains that are linked by an intracellular domain. Various examples of constructs for the EVIRs and in particular various types of proteinic domains are illustrated on Figure 13. The proteinic domain of the EVIRs according to the invention can comprise "transmembrane domain", "intracellular domain" and optionally further "extracellular non hinge domain" linking the transmembrane domains derived from the same or different proteins. The transmembrane domains, intracellular domains and optional extracellular non hinge domains can be derived from any protein that induces at least one of the following functions: cell survival, differentiation, proliferation, activation, maturation, phagocytosis, endocytosis, antigen-processing and presentation, T-cell recruitment, among other functions. Preferably, that includes any protein fragment that induces monocyte and/or macrophage and/or DC cell survival, differentiation, proliferation, activation, maturation, phagocytosis, endocytosis, antigen-processing and presentation, T-cell recruitment, among other functions (*Holliger et al., 2005, supra; Palucka et al., 2012, supra; Kastenmueller et al., 2014, supra).* Examples of proteinic domains or the transmembrane/intracellular and optional extracellular non hinge domains of those include domains from a membrane associated protein such as growth factor receptors, s, C-C chemokine receptors, as defined in the claims, either native or recombinant, that are expressed on the surface of any cell and that can promote monocyte and/or macrophage and/or DC cell survival, differentiation, proliferation, activation, maturation, phagocytosis, endocytosis, antigen-processing and presentation, T-cell recruitment, among other functions. Proteinic domains are selected from C-C chemokine receptor type 2 (CCR2), C-C chemokine receptor type 5 (CCR5), and P-selectin glycoprotein-1 ligand receptor (SELPLG), or the human nerve growth factor receptor (NGFR).

The proteinic domain may further comprise a "hinge region", which is a peptidic fragment that bridges the "transmembrane domain" to the "extracellular antibody domain" of the EVIR of the invention, thereby providing flexibility to the recombinant receptor *(*Sadelain et al., 2013, Cancer Discov, 3(4):388-98). In a particular embodiment, the proteinic domain containing a transmembrane domain, an intracellular domain and a hinge region is obtained by simply removing the extracellular domain from the membrane associated protein from which the transmembrane and intracellular domains are derived as exemplified herein. In this case, the hinge region corresponds to a peptidic region naturally linking the transmembrane and the original extracellular domain of the membrane associated protein.

An EVIR of the invention may also contain a "cell membrane export domain", which refers to any protein fragment, either cellular or viral, that increases sorting of the EVIR to the cell membrane. A non-limiting example is an IgK domain (von Heijne et al., 2006, Nat Rev Mol Cell Biol., 7:909-18*),* for example inserted at the N-terminus of the EVIR.

The term "an antigen-presenting cell" or "APC" as referred to herein, refers to a cell that displays foreign antigens complexed with major histocompatibility complexes (MHCs) on its surface; this process is known as antigen presentation. Those cells are also sometimes referred to as or "accessory cell". T-cells may recognize these complexes using their T-cell receptors (TCRs), so APCs process antigens and present them to T-cells. Examples of APCs include, but are not limited to, dendritic cells (DCs), monocytes, macrophages, certain B-cells, and certain activated epithelial cells.

The term "hematopoietic cells" refers to cells having the ability to differentiate into mature blood cells, including monocytes, macrophages and dendritic cells and includes hematopoietic stem cell (HSCs) and hematopoietic progenitor cells (HPCs).

The term "EVIR-expressing, TAA-presenting cell" refers herein to a cell expressing an EVIR according to the invention and, optionally, a protein capable of inducing APC differentiation, survival, activation and/or cross-presentation, or attracting and/or activating T cells, which after being contacted with cancer cells and/or cancer-cell derived particles, such as EVs, has internalized the cancer cell and/or cancer-cell derived particles and processed TAAs, so that TAAs presentation was achieved within the antigen presenting cell expressing the EVIR.

The term "extracellular vesicles" or "EVs" refers herein to any membrane-containing particles or fragments derived from cancer cells. EVs may comprise exosomes, microvesicles, microparticles, apoptotic bodies, cell debris, membrane fragments and similar subcellular material of tumor origin that, therefore, may be associated with known and unknown tumor antigens. After fusion of the EV with the engineered APC, the EV-associated tumor antigens are presented by the engineered APCs to T cells in order to initiate an immune response against cancer. EVs can be isolated as described (Squadrito et al., 2014, Cell Rep, 8(5):1432-46*;* Thery et al., 2006, Curr Protoc Cell Biol, Chapter 3; Unit 3:22*).* Presentation of the tumor antigens by the APCs may occur after processing and loading of the antigens on the APC's MHCI or MHCII molecules (conventional and cross-presentation) as described in Villadangos et al., 2014, Immunity, 29(3):352-61*,* but also by direct presentation of EV-derived antigen/MHC complexes via cross-dressing as described in Schölzel et al., 2014, J Hepatol., 61(3):600-8*.*

The terms "cancers" or "tumors" as defined herewith are diseases involving abnormal cell growth with the potential to invade or spread to other parts of the body. Term "cancers" designate diseases exemplified by, but not limited to, carcinomas (such as breast, prostate, lung, pancreas, and colon cancers), melanomas, sarcomas (such as bone, cartilage, nerve cancer), lymphomas and leukemias (hematopoietic cancers), germ cell tumors (such as seminoma and dysgerminoma) and blastomas.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a cancer in a mammal, particularly a human, and includes inhibiting the disease, i.e., arresting its development; or relieving the disease, i.e., causing regression of the disease and/or its symptoms or conditions such as improvement or remediation of damage. In particular, the cells, methods, uses, formulations and compositions according to the invention are useful in the treatment of cancer and/or in the prevention of evolution of a cancer into an advanced or metastatic stage in patients with early stage cancer, thereby improving the cancer staging and patient prognosis. In particular, prevention and/or treatment of a cancer may include administration of cells according to the invention.

The term "efficacy" of a treatment or method according to the invention can be measured based on changes in the course of disease or condition in response to a use or a method according to the invention. For example, the efficacy of a treatment or method according to the invention can be measured by its impact on signs or symptoms of illness. A response is achieved when the patient experiences partial or total alleviation, or reduction of unwanted symptoms of illness. According to a particular embodiment, the efficacy can be measured through the measuring of the elicited immune response against cancer cells such as by analyzing tumor-specific T cells or by assessing cancer cell death and/or inhibition of tumor growth, progression and dissemination.

The term "effective amount" as used herein refers to an amount of at least one cell according to the invention, or a pharmaceutical formulation thereof, that elicits a detectable reduction of the symptoms of the disease in a subject that is being administered said cells, these symptoms can include, for instance decrease in solid tumor mass.

The term "subject" as used herein refers to mammals. For examples, mammals contemplated by the present invention include human, primates, domesticated animals such as cattle, sheep, pigs, horses, laboratory rodents, other pets and the like.

The term "variant" as used herein means a polypeptide substantially homologous to the original peptide sequence, but which has at least one an amino acid sequence different from that of the original sequence because of one or more deletions, insertions or substitutions. Substantially homologous means a variant amino acid sequence that is at least 90%, at least 95%, at least 96%, at least 97%, at least 98% or at least 99% identical to the original amino acid sequences, as disclosed above. The percent identity of two amino acid sequences can be determined by visual inspection and/or mathematical calculation, or more easily by comparing sequence information using known computer program used for sequence comparison such as Clustal package version 1.83. A variant may comprise a sequence having at least one conservatively substituted amino acid, meaning that a given amino acid residue is replaced by a residue having similar physiochemical characteristics. Generally, substitutions for one or more amino acids present in the original polypeptide should be made conservatively. Examples of conservative substitutions include substitution of one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known (Kyte, et al, 1982, J. Mol. Biol., 157: 105-131*).* For example, a "conservative amino acid substitution" may involve a substitution of a native amino acid residue with a non-native residue such that there is little or no effect on the polarity or charge of the amino acid residue at that position. Desired amino acid substitutions (whether conservative or non-conservative) can be determined by those skilled in the art at the time such substitutions are desired.

### EVIRs according to the invention

Extra-cellular vesicle internalizing receptors (EVIRs) of the invention comprise:
(i) an extracellular antibody domain specific for a membrane-associated molecule of a cancer cell wherein said membrane molecule is a cancer-associated antigen;
(ii) a proteinic domain to anchor the EVIR to the membrane of the entigen-presing cellwhen expressed in said cell, said proteinic domain comprising at least one transmembrane domain and at least one intracellular domain; and
(iii) optionally a cell membrane export domain increasing the export of the EVIR to the cellular membrane of antigen-presenting cells.

Extra-cellular vesicle internalizing receptors of the invention comprise:
(i) an extracellular antibody domain specific for a membrane-associated molecule of a cancer cell;
(ii) a proteinic domain to anchor the EVIR to the membrane of the entigen-presing cellwhen expressed in said cell, said proteinic domain comprising one transmembrane domain and one intracellular and being selected from the nerve growth domain;
   factor receptor (NGFR), C-C chemokine receptor type 2 (CCR2), C-C-chemokine receptor type 5 (CCR5) and p-selecting glycoprotein-1 ligand receptrot (SELPLG); and
(iii) optionally a cell membrane export domain increasing the export of the EVIR to the cellular membrane of antigen-presenting cells when expressed in said cell.

In a particular embodiment, is provided an EVIR according to the invention wherein the proteinic domain further comprises a hinge region linking the extracellular antibody domain to the transmembrane domain.

In a particular embodiment, is provided an EVIR according to the invention wherein the proteinic domain comprises at least two, at least three, at least four, at least five, at least six or at least seven transmembrane domains (for example seven transmembrane domains).

In a further particular embodiment, is provided an EVIR according to the invention wherein the proteinic domain comprises at least two, at least three, at least four, at least five, at least six or at least seven transmembrane domains linked together by intracellular domains of the EVIR of the invention and/or extracellular non hinge domains.

In a particular embodiment, is provided an EVIR according to the invention wherein the proteinic domain comprises at least two, at least three or at least four intracellular domains (for example four intracellular domains).

In a further particular embodiment, is provided an EVIR according to the invention wherein the proteinic domain comprises at least two, at least three, at least four, at least five, at least six or at least seven transmembrane domains linked together by at least one, at least two, or at least three intracellular domains and optionally further linked together by extracellular at least one, at least two, or at least three non-hinge domains.

In a particular embodiment, is provided an EVIR according to the invention that further comprises an amino acid sequence that facilitates DNA engineering (e.g. a cloning site) such as TG.

In a particular embodiment, is provided an EVIR according to the invention wherein an amino acid sequence that facilitates DNA engineering (e.g. a cloning site) is of SEQ ID NO: 104.

In a particular embodiment, is provided an EVIR according to the invention wherein an amino acid sequence that facilitates DNA engineering is present between the transmembrane domain and extracellular antibody domain.

In a particular embodiment, is provided an EVIR according to the invention wherein the extracellular antibody domain is a scFv.

In a further particular embodiment, is provided an EVIR according to the invention wherein the extracellular antibody domain comprises a sequence of an antibody, or a fragment thereof specific for human epidermal growth factor receptor 2 (HER2).

In another further particular embodiment, is provided an EVIR according to the invention wherein the extracellular antibody domain comprises a sequence of an antibody specific for at least one cancer cell membrane-associated molecule corresponding to a tumor-associated antigen, including but not limited to, human epidermal growth factor receptor 2 (HER2), tyrosinase-related protein-1 (TYRP1), carcinoembryonic antigen (CEA), mesothelin, PMEL (gp100), gangliosides (GD2, GD3), and mucins.

In a more particular embodiment, is provided an EVIR according to the invention wherein the extracellular antibody domain comprises a sequence of an antibody specific for an anti-human epidermal growth factor receptor 2 (HER2).

In another more particular embodiment, is provided an EVIR according to the invention wherein the extracellular antibody domain comprises a sequence of an antibody specific for a tyrosinase-related protein-1 (TYRP1).

In another more particular embodiment, is provided an EVIR according to the invention wherein the extracellular antibody domain comprises a sequence of an antibody specific for a ganglioside GD2 (GD2).

In a more particular embodiment, is provided an EVIR according to the invention wherein the sequence of an anti-human epidermal growth factor receptor 2 (HER2) comprises the sequence of CHA21 (SEQ ID NO: 27), or a variant thereof.

In another more particular embodiment, is provided an EVIR according to the invention wherein the sequence of an anti-human epidermal growth factor receptor 2 (HER2) comprises the sequence of a trastuzumab-based scFv (SEQ ID NO: 28), or a variant thereof.

In another more particular embodiment, is provided an EVIR according to the invention wherein the sequence of an anti-human epidermal growth factor receptor 2 (HER2) comprises the sequence of a pertuzumab-based scFv (SEQ ID NO: 29), or a variant thereof.

In another more particular embodiment, is provided an EVIR according to the invention wherein the sequence of an anti-human epidermal growth factor receptor 2 (HER2) comprises the sequence of a FRP5-based scFv (SEQ ID NO: 30), or a variant thereof.

In another more particular embodiment, is provided an EVIR according to the invention wherein the sequence of an anti-tyrosinase-related protein-1 (TYRP1) comprises the sequence of TA99-based anti-TYRP1 scFv (SEQ ID NO: 113), or a variant thereof.

In another more particular embodiment, is provided an EVIR according to the invention wherein the sequence of an anti- ganglioside GD2 (GD2) comprises the sequence of 14G2abased anti-GD2 scFv (SEQ ID NO: 114), or a variant thereof.

In a particular embodiment, is provided an EVIR according to the invention wherein the proteinic domain is a fragment of a transmembrane receptor expressed by myeloid cells.

In a particular embodiment, is provided an EVIR according to the invention wherein the proteinic domain comprises a G protein-coupled receptor, a fragment or a variant thereof, expressed by myeloid cells.

In a particular embodiment, is provided an EVIR according to the invention wherein the proteinic domain comprises a seven-transmembrane domain receptor, a fragment or a variant thereof, expressed by myeloid cells.

In a more particular embodiment, is provided the EVIR according to the invention wherein it comprises a proteinic domain comprising at least one transmembrane and at least one intracellular domain, and optionally at least one extracellular non-hinge domain, and optionally a hinge domain from a receptor selected from a growth factor receptor, to anchor the EVIR to the membrane of the entigen-presing cellwhen expressed in said cell, said proteinic domain, C-C chemokine receptor, or other signalling receptor as defined in claim 1 and that that can promote monocyte and/or macrophage and/or DC cell survival, differentiation, proliferation, activation, maturation, phagocytosis, endocytosis, antigen-processing and presentation, T-cell recruitment, among other functions.

In a more particular embodiment, is provided an EVIR according to the invention wherein the proteinic domain comprises a hinge domain, at least one transmembrane and at least one intracellular domain from a receptor selected from the human nerve growth factor receptor (proteinic domain of SEQ ID NO: 31), the C-C chemokine receptor type 2 (proteinic domain of SEQ ID NO: 35), the chemokine receptor CCR5 (S proteinic domain of EQ ID NO: 39), and the P-selectin glycoprotein-1 ligand receptor (proteinic domain of SEQ ID NO: 41), or variants thereof.

In a more particular embodiment, is provided an EVIR according to the invention wherein the proteinic domain is selected from SEQ ID NO: 35, SEQ ID NO: 38, SEQ ID NO: 39 and SEQ ID NO: 41, or a fragment or variant thereof.

In a more particular embodiment, is provided an EVIR according to the invention comprising proteinic domains derived from isoforms of the proteins described herein.

In a more particular embodiment, is provided an EVIR according to the invention wherein the proteinic domain derived from a receptor selected from the human nerve growth factor receptor, the chemokine receptor CCR5, and the P-selectin glycoprotein-1 ligand receptor.

In a further more particular embodiment, is provided an EVIR according to the invention wherein the proteinic domain comprises a sequence selected from SEQ ID NO: 31, 39 and 41 or a variant thereof.

According to a particular embodiment, an EVIR according to the invention comprises a cell membrane export domain, for example at the N-terminus of the EVIR sequence.

According to a more particular embodiment, an EVIR according to the invention comprises a cell membrane export domain comprising an IgK domain (*von Heijne, 2006, supra*). According to a further particular embodiment an EVIR according to the invention comprises a cell membrane export domain of SEQ ID NO: 42 or a variant thereof.

EVIRs according to the invention can be obtained by any known methods of molecular cloning for polypeptide expression, as described in the following examples.

According to a further particular embodiment EVIRs of the invention comprise:
(i) an anti-HER2 CHA21 comprising an amino acid sequence of SEQ ID NO: 27;
(ii) a proteinic domain comprising an amino acid sequence selected from: SEQ ID NO: 31, SEQ ID NO: 35, 8, SEQ ID NO: 39, and SEQ ID NO: 41;
(iii) optionally an amino acid sequence that facilitates DNA engineering of SEQ ID NO: 104; and
(iv) a cell membrane export domain IgK of SEQ ID NO: 42.

According to a further particular embodiment an EVIR of the invention comprises:
(i) an anti-TYRP1 comprising an amino acid sequence of SEQ ID NO: 113;
(ii) a proteinic domain comprising an amino acid sequence of SEQ ID NO: 31;
(iii) optionally an amino acid sequence that facilitates DNA engineering of SEQ ID NO: 104; and
(iv) a cell membrane export domain IgK of SEQ ID NO: 42.

According to a further particular embodiment EVIRs of the invention comprise:
(i) an anti-GD2 comprising an amino acid sequence of SEQ ID NO: 114;
(ii) a proteinic domain comprising an amino acid sequence of SEQ ID NO: 31;
(iii) optionally an amino acid sequence that facilitates DNA engineering of SEQ ID NO: 104; and
(iv) a cell membrane export domain IgK of SEQ ID NO: 42.

According to a further particular embodiment, an EVIR according to the invention has an amino acid sequence selected from SEQ ID NO: 54, SEQ ID NO: 62, and SEQ ID NO: 64, or variants thereof.

According to a further particular embodiment, an EVIR according to the invention has an amino acid sequence of SEQ ID NO: 132, or variants thereof.

According to a further particular embodiment, an EVIR according to the invention has an amino acid sequence of SEQ ID NO: 133 or variants thereof.

### Nucleic acids of the invention

Isolated nucleic acid encoding an EVIR according to the invention may be, for instance, natural DNA or RNA or a recombinant or synthetic DNA, RNA or LNA or a recombinant nucleic acid molecule comprising any of the nucleic acid molecules according to the invention either alone or in combination. In a particular embodiment, the nucleic acid molecules according to the invention are cDNA.

In a particular embodiment, is provided an isolated nucleic acid molecule encoding an EVIR according to the invention, wherein the extracellular antibody domain comprises a sequence of an antibody specific for at least one cancer cell membrane-associated molecule corresponding to a tumor-associated antigen, including but not limited to, human epidermal growth factor receptor 2 (HER2), tyrosinase-related protein-1 (TYRP1), carcinoembryonic antigen (CEA), mesothelin, PMEL (gp100), gangliosides (GD2, GD3), and mucins.

In a more particular embodiment, is provided an isolated nucleic acid molecule encoding an EVIR according to the invention comprising an extracellular antibody domain consisting of CHA21 sequence, wherein the said nucleic acid molecule comprises SEQ ID NO: 1 or a variant thereof.

In a more particular embodiment, is provided an isolated nucleic acid molecule encoding an EVIR according to the invention comprising an extracellular antibody domain consisting of CHA21 sequence, wherein the said nucleic acid molecule comprises SEQ ID NO: 1.

In a more particular embodiment, is provided an isolated nucleic acid molecule encoding an EVIR according to the invention comprising an extracellular antibody domain consisting of CHA21 sequence, wherein the said nucleic acid molecule comprises SEQ ID NO: 128.

In another more particular embodiment, is provided an isolated nucleic acid molecule encoding an EVIR according to the invention comprising an extracellular antibody domain consisting of a trastuzumab-based scFv, wherein the said nucleic acid molecule comprises SEQ ID NO: 76.

In another more particular embodiment, is provided an isolated nucleic acid molecule encoding an EVIR according to the invention comprising an extracellular antibody domain consisting of a pertuzumab-based scFv sequence, wherein the said nucleic acid molecule comprises SEQ ID NO: 77.

In another more particular embodiment, is provided an isolated nucleic acid molecule encoding an EVIR according to the invention comprising an extracellular antibody domain consisting of a FRP5-based scFv sequence, wherein the said nucleic acid molecule comprises SEQ ID NO: 78.

In another more particular embodiment, is provided an isolated nucleic acid molecule encoding an EVIR according to the invention comprising an extracellular antibody domain consisting of an anti-TYRP1 scFv sequence, wherein the said nucleic acid molecule comprises SEQ ID NO: 111.

In a further particular embodiment, is provided an isolated nucleic acid molecule encoding an EVIR according to the invention comprising an extracellular antibody domain consisting of an anti-GD2 scFv sequence, wherein the said nucleic acid molecule comprises SEQ ID NO: 112.

In a more particular embodiment, is provided an isolated nucleic acid molecule encoding an EVIR according to the invention comprising at least one transmembrane and at least one intracellular domain from a receptor selected from the human nerve growth factor receptor (SEQ ID NO: 43), the C-C chemokine receptor type 2 (SEQ ID NO: 47), the chemokine receptor CCR5 (SEQ ID NO: 51), ) and the P-selectin glycoprotein-1 ligand receptor (SEQ ID NO: 53).

In another embodiment, is provided an isolated nucleic acid molecule encoding an EVIR according to the invention comprising a cell membrane export domain comprising an IgK domain, wherein the said nucleic acid molecule comprises SEQ ID NO: 2, in particular SEQ ID NO: 129, wherein an isolated nucleic acid molecule encoding said EVIR is further comprising a nucleic acid sequence encoding an extracellular antibody domain consisting of CHA21 (SEQ ID NO: 1).

In another embodiment, is provided an isolated nucleic acid molecule encoding an EVIR according to the invention comprising a cell membrane export domain comprising an IgK domain, wherein the said nucleic acid molecule comprises SEQ ID NO: 129, wherein an isolated nucleic acid molecule encoding said EVIR is further comprising a nucleic acid sequence encoding an extracellular antibody domain consisting of CHA21 (SEQ ID NO: 128).

In another embodiment, is provided an isolated nucleic acid molecule encoding an EVIR according to the invention comprising a cell membrane export domain comprising an IgK domain, wherein the said nucleic acid molecule comprises SEQ ID NO: 105 wherein an isolated nucleic acid molecule encoding said EVIR is further comprising an extracellular antibody domain consisting of a trastuzumab-based scFv of SEQ ID NO: 76 or an extracellular antibody domain consisting of a FRP5-based scFv sequence of SEQ ID NO: 78. In another embodiment, is provided an isolated nucleic acid molecule encoding an EVIR according to the invention comprising a cell membrane export domain comprising an IgK domain, wherein the said nucleic acid molecule comprises SEQ ID NO: 106, wherein an isolated nucleic acid molecule encoding said EVIR is further comprising an extracellular antibody domain consisting of a pertuzumab-based scFv sequence of SEQ ID NO: 77.

In a particular embodiment, is provided an isolated nucleic acid molecule encoding an EVIR according to the invention comprising an amino acid sequence that facilitates DNA engineering (e.g. cloning site).

In a particular embodiment, is provided an isolated nucleic acid molecule encoding an EVIR according to the invention comprising an amino acid sequence that facilitates DNA engineering (e.g. cloning site), wherein the said nucleic acid molecule comprises SEQ ID NO: 107 or SEQ ID NO: 108.

According to another embodiment, is provided an isolated nucleic acid molecule encoding an EVIR according to the invention comprising a nucleic acid sequence encoding a functional protein that promotes in cells, preferably in monocytes, macrophages and/or DCs, survival, differentiation, proliferation, activation, maturation, phagocytosis, endocytosis, M1-polarization, antigen-processing and presentation, T-cell recruitment, among other functions, or a protein capable of inducing APC differentiation, survival, activation and/or cross-presentation (for example CD40 (cluster of differentiation 40), GM-CSF (CSF2, colony stimulating factor 2), Type I and II interferon (e.g. interferon gamma (IFNy)), LIN28, or Rab34), or attracting and/or activating T cells (for example IL-2 or CXCL9 (chemokine (C-X-C motif) ligand 9)).

According to another embodiment, is provided an isolated nucleic acid molecule encoding an EVIR according to the invention further comprising a nucleic acid sequence encoding a protein capable of inducing APC differentiation, survival, activation and/or cross-presentation (for example CD40, GM-CSF (CSF2), Type I and II interferon (e.g. IFNy), or Rab34), or attracting and/or activating T cells (for example IL-2 or CXCL9).

According to another embodiment, is provided an isolated nucleic acid molecule encoding an EVIR according to the invention comprising a nucleic acid sequence encoding a functional protein capable of inducing APC differentiation, survival, activation and/or cross-presentation such as LIN28 (protein encoded by the *Lin28* gene).

According to another embodiment, is provided an isolated nucleic acid molecule encoding an EVIR according to the invention further comprising a nucleic acid sequence encoding a protein capable of inducing APC differentiation, survival, activation and/or cross-presentation selected from: SEQ ID NO: 117 (CXCL9), SEQ ID NO: 120 (GM-CSF), SEQ ID NO: 123 (IFN*γ*), SEQ ID NO: 126 (LIN28) and SEQ ID NO: 127 (CD40).

According to a further particular embodiment, is provided an isolated nucleic acid encoding an EVIR according to the invention selected from SEQ ID NO: 65, SEQ ID NO: 69, SEQ ID NO: 73, and SEQ ID NO: 75.

According to a further particular embodiment, is provided an isolated nucleic acid encoding an EVIR according to the invention selected from SEQ ID NO: 130 and SEQ ID NO: 131.

In a more particular embodiment, is provided an isolated nucleic acid encoding an EVIR according to the invention comprising orthologous human sequences encoding proteinic domain of the invention.

### Vectors and methods for cell transduction

In one embodiment, the invention provides a recombinant expression vector comprising a nucleic acid molecule according to the invention, wherein the vector optionally comprises an expression controlling sequence, allowing expression in eukaryotic host cells of the encoded sequence, operably linked to said nucleic acid molecule.

Numerous expression systems can be used, including without limitation chromosomes, episomes, plasmids, and virus-derived vectors. More particularly, the recombinant vectors used can be derived from bacterial plasmids, transposons, yeast episomes, insertion elements, yeast chromosome elements, viruses such as baculovirus, papilloma viruses such as SV40, vaccinia viruses, adenoviruses, fox pox viruses, pseudorabies viruses, retroviruses, lentiviruses, adeno-associated viruses (AAV). These recombinant vectors can equally be cosmid or phagemid derivatives.

In one embodiment, the recombinant vectors are any viral vectors selected from retroviral vectors (both replication-competent and replication-defective retroviral vectors), lentiviral vectors, in particular bidirectional lentiviral vectors, adenoviral vectors and adeno-associated vectors.

In a particular embodiment, the recombinant vector is a retroviral vector.

In one embodiment, the invention provides a recombinant expression vector comprising nucleic acid molecules encoding for one or more than one EVIR sequence of the invention.

In one embodiment, the invention provides a recombinant expression vector comprising at least one nucleic acid molecule encoding for a functional protein that promotes in cells, preferably in monocytes, macrophages and/or DCs, survival, differentiation, proliferation, activation, maturation, phagocytosis, endocytosis, antigen-processing and presentation, T-cell recruitment, among other functions.

In one embodiment, the invention provides a recombinant expression vector comprising nucleic acid molecules encoding for one or more than one EVIR sequence of the invention further comprising at least one nucleic acid molecule encoding for a functional protein that promotes in cells, preferably in monocytes, macrophages and/or DCs, survival, differentiation, proliferation, activation, maturation, phagocytosis, endocytosis, antigen-processing and presentation, T-cell recruitment, among other functions.

According to a particular embodiment, the expression vectors according to the invention may also encode for a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9). According to a particular embodiment, the invention provides a recombinant expression vector comprising nucleic acid molecules encoding for a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

In another embodiment, a bidirectional or bicistronic expression vector can be used to coexpress at least one EVIR according to the invention together with a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

The nucleic acid sequence can be inserted in the recombinant expression vector by methods well known to a person skilled in the art such as, for example, those that are described in Molecular Cloning: A Laboratory Manual, Sambrook et al., 4th Ed., Cold Spring Harbor Laboratory Press, Cold Spring Harbor, N.Y., 2001*.*

Recombinant vectors can include nucleotide sequences that allow, control or regulate the expression and the transcription of a polynucleotide of the invention as well as the translation of an EVIR of the invention, these sequences being selected according to the host cells that are used. For example, an appropriate secretion signal can be integrated in the recombinant vector so that the EVIR, encoded by the nucleic acid molecule of the invention, will be directed to the membrane.

In a further embodiment, is provided a host cell comprising a recombinant vector according to the invention.

The introduction of the recombinant vector in a host cell can be carried out according to methods that are well known to a person skilled in the art, such as those described in Basic Methods in Molecular Biology, Davis et al., 2nd ed., McGraw-Hill Professional Publishing, 1995*, and Molecular Cloning: A Laboratory Manual,* supra, such as transfection by calcium phosphate, transfection by DEAE dextran, transfection, microinjection, transfection by cationic lipids, electroporation, transduction or infection.

In another embodiment, the invention provides a process for producing APCs capable of expressing an EVIR, optionally along with a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9), comprising contacting cells with a vector or a nucleic acid according to the invention.

According to an embodiment, EVIRs according to the invention are optionally co-expressed with the said protein or alternatively, the expression of said protein is achieved in APCs expressing EVIRs by using an independent vector.

According to a particular aspect is provided an *ex vivo* method (i.e., in culture) of inducing expression of at least one EVIR of the invention in an APC or a stem/progenitor cell thereof comprising the steps of:
(i) *ex vivo* transducing said cell with a vector according to the invention; and
(ii) optionally inducing APC differentiation, maturation or activation;
wherein an EVIR according to the invention is expressed in said APC cells with a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

Another aspect of the invention provides a method of inducing *in vivo* the expression of at least one EVIR of the invention in an APC or a stem/progenitor cell thereof in a subject in need thereof, said method comprising the steps of:
(i) administering a vector encoding an EVIR according to the invention to said subject under suitable conditions for inducing transduction of the subject's APCs or stem/progenitor cell thereof *in vivo* with said vector; and
(ii) optionally inducing APC differentiation, maturation or activation *in vivo,*
wherein an EVIR according to the invention is expressed *in vivo* in said APC cells with a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

The EVIRs can be delivered to APCs using a lentiviral vector (or alternative viral or non-viral vectors), either *ex vivo* on isolated APCs (or precursors thereof) or *in vivo* via systemic (e.g., intravenous) or local (e.g., intra-tumoral, peri-tumoral, lymphnodal, etc.) delivery of a vector of the invention encoding said EVIRs.

In particular, the invention provides a process for producing an antigen-presenting cell or any stem of progenitor cell thereof, expressing at least one EVIR according to the invention, comprising contacting said APCs or stem of progenitor cell thereof, in particular DCs, monocytes or macrophages, either *ex vivo* or *in vivo* with a vector or a nucleic acid according to the invention.

### EVIR-expressing APCs

According to an embodiment, the invention provides an antigen-presenting cell expressing at least one EVIR according to the invention.

According to an embodiment, the invention provides a cell expressing one EVIR according to the invention.

According to a further embodiment, the invention provides a cell expressing at least an EVIR along with a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

According to a particular embodiment, the invention provides a cell expressing at least 2, at least 3, at least 4 different EVIRs of the invention.

According to a particular embodiment, the invention provides a cell expressing at least one EVIR according to the invention, for example from about 1 to about 3 different EVIRs of the invention.

According to an embodiment, is provided a cell composition comprising APCs expressing at least one EVIR of the invention, wherein at least 1%, at least 10%, at least 20%, at least 30%, at least 40%, at least 50%, at least 60%, at least 70%, at least 80%, or at least 90% of the APCs, in particular monocytes, macrophages or DCs, express at least one EVIR of the invention.

According to a further embodiment, is provided a cell composition of the invention, wherein at least 1% of the cell population expresses at least one EVIR of the invention.

According to another embodiment, is provided a cell composition comprising APCs expressing at least an EVIR along with a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

According to an embodiment, is provided a cell composition of the invention, wherein expression of the EVIR persists for at least several hours after delivery with a vector of the invention.

According to an embodiment, is provided a cell composition of the invention, wherein at least 1% of the cell population expresses at least one EVIR of the invention and said expression persists for at least several hours after delivery with a vector of the invention.

According to an embodiment, is provided a cell composition of the invention, wherein expression of a protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9), persists for at least several hours after delivery with a vector of the invention.

According to one embodiment, the expression level and time of EVIRs can be measured by methods such as flow cytometry, protein analysis, or nucleic acidic amplification.

According to another embodiment, the invention provides a cell according to the invention, wherein said cells is a hematopoietic cell with the ability to differentiate into monocytes, macrophages or dendritic cells, including hematopoietic stem/progenitor cells.

According to another embodiment, the invention provides a cell according to the invention, wherein said cell is selected from hematopoietic stem cell and progenitor cell.

According to another embodiment, the invention provides a cell according to the invention, wherein said cell is an antigen-presenting cell (APC).

According to another embodiment, the invention provides a cell according to the invention, wherein said cell is an APC selected from a monocyte, a macrophage or a dendritic cell.

In another embodiment, the invention provides a cell expressing at least one EVIR, wherein said cell further expresses at least one protein capable of inducing APC differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9).

It is understood that, when co-expressed in a cell expressing at least one EVIR of the invention, CD40 can act to enhance antigen-presenting cell and T-cell activation; Type I and II interferon (e.g. IFNγ) can act to enhance antigen-presenting cell and T-cell maturation and activation; LIN28 blocks the maturation of the microRNA Let-7, promoting activation and antigen presentation by macrophages and DCs (Baer et al., 2016, Nat Cell Biol., 18(7):790-802); Rab34 can act to enhance antigen cross-presentation; GM-CSF (CSF2) can act to increase antigen-presenting cell and dendritic cell differentiation, maturation and activation; IL-2 can act to increase T-cell proliferation; CXCL9 can act to increase T-cell recruitment.

In another embodiment, the invention provides EVIR-expressing cells that are able to internalize EVs to the cell cytoplasm.

In another embodiment, the invention provides EVIR-expressing cells with enhanced internalization capabilities of cancer-cell derived EVs, as compared to the same cells not expressing an EVIR of the invention, which property is independent of a contact with the cancer cells.

In another embodiment, the invention provides EVIR-expressing cells with faster internalization of cancer-cell derived EVs, as compared to cells not expressing EVIR, which property is independent of contact with cancer cells.

According to one embodiment, the internalization level and kinetics of EVs by EVIR-expressing cells can be measured by methods such as flow cytometry and protein analysis.

In another embodiment, the invention provides EVIR-expressing cells that are able to uptake, process, and present to T-cells tumor associated antigens (TAAs) without the need of a preidentification of those TAAs.

In another embodiment, the invention provides EVIR-expressing cells that facilitate TAAs cross-presentation to CD8⁺ T cells.

In another embodiment, the invention provides EVIR-expressing cells with enhanced presentation (in terms of repertoire of TAAs and in terms of quantity of each TAA) to T-cells of EV-associated TAAs as compared to cells not expressing EVIR.

According to one embodiment, the level of presentation of TAAs to T cells achieved by cells of the invention can be measured by methods such as flow cytometry, protein analysis, and T-cell proliferation/activation assays.

In another embodiment, the invention provides cells according to the invention that induce T-cells proliferation.

In another embodiment, the invention provides cells according to the invention with enhanced ability to induce T-cell proliferation as compared to cells not expressing EVIR.

According to one embodiment, T-cell proliferation can be measured by methods such as flow cytometry, cell cycle analysis, T-cell suppression, and mixed leukocyte reactions (MLR).

### Methods and uses according to the invention

The invention provides a method of inducing expression of EVIRs, optionally along with a protein capable of inducing differentiation, survival, activation and/or cross-presentation (for example CD40, GM-CSF (CSF2), Type I and II interferon (e.g. IFNy), LIN28, or Rab34), or attracting and/or activating T cells (for example IL-2 or CXCL9), in an antigen-presenting cell (APC) or a stem or progenitor cell thereof according to the invention.

In one particular method of the invention, APC differentiation can be conducted according to methods involving exposing APC precursors such as monocytes under cell culture conditions well-known to those skilled in the art.

In a particular embodiment, the invention provides a method of inducing expression of EVIRs in APCs, said method comprising the step of transfecting or transducing said cells with a vector according to the invention.

In another embodiment, is provided an *ex vivo* method of preparing EVIR-expressing, TAA-presenting cells according to the invention, wherein the provided EVs are tumor-derived particles, such as exosomes and other vesicles, isolated from either a tumor or blood sample.

In another embodiment, is provided an *ex vivo* method of preparing EVIR-expressing, TAA-presenting cells according to the invention, wherein the APC and cancer cells or EVs are co-cultured and the EVs derive from the *ex-vivo* cultured cancer cells.

According to a particular embodiment, the EVIR-expressing, TAAs-presenting cells according to the invention can be injected to cancer subject, while and optionally inducing APC differentiation, maturation or activation *in vivo* and can be useful in a method of treatment as described herein.

In a particular embodiment, the cancer cells or cancer-cell derived EVs are autologous, i.e. originating from the patient to be treated.

In a particular embodiment, the APCs or stem/progenitor cells are autologous, i.e. originating from the patient to be treated.

In another aspect, cells according to the invention are useful for the preparation of a vaccine for treating and/or preventing a cancer.

According to a particular aspect, the EVIRs of the invention are useful for uptaking cancer cell-derived EVs that contain free (unloaded) TAAs.

According to another particular aspect, the EVIRs of the invention are useful for uptaking cancer cell-derived EVs that contain TAAs already loaded on MHCI or MHCII molecules.

Another aspect of the invention relates to an isolated cell expressing at least one EVIR of the invention and capable of presenting unloaded TAAs via MHCI or MHCII molecules.

Another aspect of the invention provides an isolated cell expressing at least one EVIR of the invention and capable of presenting TAAs already loaded on MHCI or MHCII molecules.

It is worth pointing that currently existing approaches using APC-TAA are not designed to instruct the APC to uptake and present TAAs endogenously, i.e., in the body of the patient and from the tumor of the patient, but instead rely on the *ex vivo* exposure to tumor-derived material. Therefore, the EVIR-expressing vectors or cells according to the invention and methods using thereof are particularly advantageous over the existing approaches in immunotherapy and/or in the prevention and/or treatment of cancers.

### Compositions according to the invention

Pharmaceutical compositions or formulations according to the invention may be administered as a pharmaceutical formulation, which contains EVIR-expressing vectors or cells as described herewith.

Another aspect of the invention provides a pharmaceutical composition comprising cells of the invention and at least one pharmaceutically acceptable agent able to promote APC differentiation, maturation and/or activation *in vivo.*

The invention provides pharmaceutical or therapeutic cells as compositions and methods for treating a subject, preferably a mammalian subject, and most preferably a human patient who is suffering from a cancer.

Cells of the invention or formulations thereof may be administered as a pharmaceutical formulation, which can contain one or more co-agents according to the invention in any form described herein. The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended dosage range to be employed.

Compositions of this invention may be liquid formulations including, but not limited to aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methylcellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween^{®}, Span^{®}.

Compositions of this invention may also be formulated as a depot preparation, which may be administered by implantation or by intramuscular injection.

The compounds of this invention can also be administered in sustained release forms or from sustained release drug delivery systems.

According to a particular embodiment, compositions according to the invention are for intravenous use.

According to a particular embodiment, compositions according to the invention are for intratumoral use.

According to a particular embodiment, compositions according to the invention are for subcutaneous use.

According to a particular embodiment, compositions according to the invention are for intralymphnodal use.

According to a particular aspect, compositions of the invention are vaccine compositions.

According to a particular aspect, vaccine compositions may comprise one or more co-agents selected among CpG oligonucleotides (short single-stranded synthetic DNA molecules that contain a cytosine triphosphate deoxynucleotide ("C") followed by a guanine triphosphate deoxynucleotide ("G"), "p" refers to the phosphodiester link between consecutive nucleotides) diphtheria toxin, growth factors (non-limiting examples are: M-CSF (macrophage colony-stimulating factor), GM-CSF, granulocyte-macrophage colony-stimulating factor), FLT3 (FMS-like tyrosine kinase-3),) and/or cytokines (non-limiting examples are: IFNy (Interferon gamma), CXCL9 (Chemokine (C-X-C motif) ligand 9), IL12 (interleukin 12), IL2 (interleukin 2), IL-4 (interleukin 4)).

In another particular aspect, compositions according to the invention are adapted for delivery by single administration.

According to a particular embodiment, compositions of the invention are veterinary compositions.

Further materials as well as formulation processing techniques and the like are set out in Part 5 of Remington's "The Science and Practice of Pharmacy ", 22nd Edition, 2012, University of the Sciences in Philadelphia, Lippincott Williams & Wilkins*.*

In another aspect, the invention provides compositions comprising vectors according to the invention.

In another aspect, the invention provides compositions comprising EVIR-expressing cells according to the invention.

### Mode of administration

Cells and formulations thereof according to this invention may be administered in any manner including parenterally, intravenously, intratumorally, subcutaneously, intra-dermally, rectally, direct tissue perfusion during surgery, or combinations thereof. Parenteral administration includes, but is not limited to, intravenous, intra-arterial, intra-peritoneal, subcutaneous and intramuscular. The compositions of this invention may also be administered in the form of an implant, which allows slow release of the compositions as well as a slow controlled i.v. infusion.

### Combination

According to the invention, the vectors and cells according to the invention, and pharmaceutical formulations thereof, can be administered alone or in combination with a coagent useful in the prevention and/or treatment of a cancer such as therapeutic antibodies that enhance the adaptive immune system's activity against the tumor (such as anti-PD1, anti-PDL1, anti-CTLA4 antibodies), therapeutic antibodies or TLR agonists that enhance the innate immune system's activity against the tumor (such as anti-CD40 antibodies), therapeutic antibodies or small molecule inhibitors that deplete endogenous monocytes, macrophages or dendritic cells (for example anti-CSF1R inhibitors), thus favoring the engraftment of and uptake of EVs by EVIR expressing cells of the invention.

The cells according to the invention might also be combined with known chemo-, radiotherapeutics that enhance cancer cell killing and release of cancer-cell derived EVs, as defined in the application.

The invention encompasses the administration of vectors or cells, pharmaceutical formulations thereof, or composition according to the invention, wherein said vectors or cells or compositions are administered to an individual prior to, simultaneously or sequentially with other therapeutic regimens, co-agents useful in the prevention and/or treatment of a cancer, in a therapeutically effective amount.

Cells or composition according to the invention, or the pharmaceutical formulation thereof, that are administered simultaneously with said co-agents can be administered in the same or different composition(s) and by the same or different route(s) of administration.

### Kits

According to another aspect of the invention, is provided a kit comprising at least one recombinant expression vector and/or at least one cell according to the invention, and optionally instructional material.

According to another further embodiment, the kit according to the invention comprises at least one recombinant expression vector and further comprises at least one agent for transducing an antigen-presenting cell or a stem/progenitor cell thereof with said recombinant expression vector.

According to another further embodiment, the kit according to the invention comprises at least one cell according to the invention and further comprises at least one agent for the preservation of said cells and/or culture of said cells with cancer cells of cancer derived EVs.

### Patients

In an embodiment, patients according to the invention are suffering from any type of cancer. In an embodiment, patients according to the invention are suffering from any type of cancer at any stage, including non-metastatic and metastatic.

In a particular embodiment, patients according to the invention are suffering from carcinomas, sarcomas, melanomas, brain tumors, hematological cancers, or any pre-malignant or malignant neoplasm.

### EXAMPLES

The following abbreviations refer respectively to the definitions below:
**293T cells** (human embryonic kidney cells); **B4GALNT1** (Beta-1,4-N-Acetyl-Galactosaminyltransferase 1, GD2 synthase); **B16 cells** (murine melanoma tumor cell line); **BM** (bone marrow); **BMDC** (bone marrow derived dendritic cell); **BMDM** (bone marrow derived macrophage); **CCR1** (chemokine receptor type 1); **CCR2** (C-C chemokine receptor type 2); **CCR5** (chemokine receptor type 5); **CSF2** (colony stimulating factor 2); **CSF2RB** (colony-stimulating factor-2 receptor B); **CXCR4** (chemokine receptor CXCR4); **CD40** (cluster of differentiation 40); **DC** (dendritic cell); **CXCL9** (chemokine (C-X-C motif) ligand 9); **EC** (extracellular domain); **EV** (extracellular vesicle); **dLNGFR** (truncated low-affinity human nerve growth factor receptor); **EVIR** (extra-cellular vesicle internalizing receptor); **FLT3** (receptor tyrosine kinase); **GD2** (ganglioside GD2); **GFP** (green fluorescent protein); **iBMM** (immortalized murine bone marrow derived macrophages); **IC** (intracellular domain); **IFNγ** (interferon gamma); **ITGB2** (integrin beta chain beta 2 receptor); LIN28 (protein encoded by the *LIN28* gene); **LV** (lentiviral vector); **MC38** (a colon carcinoma cell line); **mTq** (turquoise fluorescent protein); **OVA** (ovalbumin); **MFI** (mean fluorescence intensity); **P388D1** (murine monocytic cell line isolated from lymphoma); **PM** (Peritoneal macrophages); **SELPLG** (P-selectin glycoprotein-1 ligand receptor); **SFFV** (spleen forming focus virus); **ST8SIA1** (ST8 Alpha-N-Acetyl-Neuraminide Alpha-2,8-Sialyltransferase 1, GD3 synthase); **TLR4** (toll-like receptor 4); **TM** (transmembrane domain); **TYRP1** (tyrosinase-related protein-1); **UT** (untransduced).

### Example 1: Design of extra-cellular vesicle internalizing receptor

11 different EVIR molecules were designed as follows.

### Cloning design of EVIRs

11 transmembrane receptors were selected and their intracellular (IC) domain, transmembrane (TM) domain, and a short extracellular (EC) domain were cloned in combination with the DNA coding sequence for the HER2-specific scFv termed CHA21 (SEQ ID NO: 128) (extracellular antibody domain specific for a surface molecule of a cancer cell).

DNA coding sequences from the selected transmembrane receptors were: the human nerve growth factor receptor (dLNGFR, SEQ ID NO: 43), FcyRIIIA receptor (a member of the Fcy receptor family expressed by cells of the innate immune system, SEQ ID NO: 44), the receptor tyrosine kinase (FLT3, SEQ ID NO: 45), the toll-like receptor 4 (TLR4, SEQ ID NO: 46), the C-C chemokine receptor type 2 (CCR2, SEQ ID NO: 47), the integrin beta chain beta 2 receptor (ITGB2, SEQ ID NO: 48), the colony-stimulating factor-2 receptor B (CSF2RB, SEQ ID NO: 49), the chemokine receptor CCR1 (SEQ ID NO: 50), the chemokine receptor CCR5 (SEQ ID NO: 51), the chemokine receptor CXCR4 (SEQ ID NO: 52), the P-selectin glycoprotein-1 ligand receptor (SELPLG, SEQ ID NO: 53).

A mouse-optimized CHA21 coding DNA sequence (SEQ ID NO: 128) was obtained from GeneArt^{®} (LifeTechnologies). A coding DNA sequence of IgK signal domain (SEQ ID NO: 129) was incorporated to increase the export of a receptor to membrane. A linker sequence containing a high efficiency Kozak sequence and restriction sites for cloning (SEQ ID NO: 3) was incorporated at the 5' end of the EVIR coding sequence. Restriction enzyme sites and a stop codon (SEQ ID NO: 4) were incorporated at the 3'end of the scFv sequence for cloning, before the STOP codon, the DNA sequence of the transmembrane and intracellular domains of the EVIR.

The DNA coding sequence of FcyRIIIa was obtained from GeneArt^{®} (Life Technologies). The linker sequences were added to the sequence at the 5' (SEQ ID NO: 5) and 3' (SEQ ID NO: 6) end of the FcyRIIIa coding sequence.

The DNA coding sequence of dLNGFR was obtained by PCR from a lentiviral vector (LV) that expresses the dLNGFR and GFP (Amendola et al., 2005, Nat biotechnol, 23(1): 108-16). Primers that contain restriction sites for AgeI and MluI were used as specified in **Table 1.** The DNA coding sequence of FLT3 was obtained by PCR from cDNA of BM-derived DCs as described in Example 2 and primers that contain restriction sites for AgeI and XhoI were used. The DNA coding sequences of the mouse *Tlr4, Ccr2, Itgb2, Csf2rb, Ccr1, Ccr5, Cxcr4, Selplg* receptors were obtained by PCR from cDNA of peritoneal macrophages as described in Example 2 and primers that contain restriction sites for for AgeI, XmaI, MluI and SalI were used.

**Table 1**

| **Gene name** | **Forward primer** | **Reverse Primer** |
|---|---|---|
| dLNGFR | AAAAAACCGGTCTTCTGGGGGTGTCCCTTG (SEQ ID NO: 7) | AAAAAACGCGTAGTTAGCCTCCCCCAT CTCC (SEQ ID NO: 8) |
| *Flt3* | AAAAAACCGGTCCAGGCCCCTTCCCTTTCA TC (SEQ ID NO: 9) | AAAAACTCGAGAGAGGCGAGGCTAATC TTGG (SEQ ID NO: 10) |
| *Tlr4* | AAAAAACCGGTCAGCTGTATTCCCTCAGC ACT (SEQ ID NO: 11) | AAAAAGTCGACTGGGTTTAGGCCCCAG AGTT (SEQ ID NO: 12) |
| *Ccr2* | AAAAAACCGGTATGGAAGACAATAATATG TTACCTC (SEQ ID NO: 13) | AAAAAACGCGTATGTACAAACTGCTCC CTCC (SEQ ID NO: 14) |
| *Itgb2* | AAAAAACCGGTAATGCACGGCTGGTAGAG TG (SEQ ID NO: 15) | AAAAAACGCGTGGGGTCACATCTGCT TGAT (SEQ ID NO: 16) |
| *Csf2rb* | AAAAAACCGGTACTCAGAAGATGGCTTAC TCATTCA (SEQ ID NO: 17) | AAAAAACGCGTTGGTGAGATTGGGAGG AGAC (SEQ ID NO: 18) |
| *Ccr1* | AAAAAACCGGTACTCCATGCCAAAAGACT GCT (SEQ ID NO: 19) | AAAAAACGCGTACCTTCCTTGGTTGAC ACCTATG (SEQ ID NO: 20) |
| *Ccr5* | AAAAAACCGGTATGTCAGCACCCTGCCAA AAA (SEQ ID NO: 21) | AAAAAACGCGTCATTCCTACTCCCAAG CTGCAT (SEQ ID NO: 22) |
| *Cxcr4* | AAAAACCCGGGTTCCGGGATGAAAACGTC CA (SEQ ID NO: 23) | AAAAAACGCGTTGCATAAGTGTTAGCT GGAGTG (SEQ ID NO: 24) |
| *Selplg* | AAAAAACCGGTATTGCCACCACTGACCCT A (SEQ ID NO: 25) | AAAAAACGCGTGCAAAGGTCTCGCTTA GGTG (SEQ ID NO: 26) |

The synthetic DNA sequence encoding for CHA21 was inserted in an LV containing the spleen forming focus virus (SFFV) promoter and the WPRE stabilizing sequence (Squadrito et al., 2012, Cell Rep, 1(2): 141-54*).* To this aim, the plasmid containing CHA21 with BamHI and XhoI, and the plasmid containing the SFFV.miR-511-3p.OFP.WPRE (*Squadrito et al., 2012, supra),* were digested with BamHI and SalI. The IC, TM and EC domains of the selected receptors were then inserted by digesting the PCR products indicated above with the restriction enzymes present in the corresponding primers.

In order to trace EVIR expression, the resulting SFFV.EVIR.WPRE sequence was cloned into a bidirectional LV (*Amendola et al., 2005, supra*) by replacing the hPGK.dLNGFR cassette with the SFFV.EVIR cassette with EcoRV and AvrII restriction sites. In this bidirectional LV, the GFP is expressed under the transcriptional control of the minimal cytomegalovirus promoter (mCMV).

Total RNA obtained from either bone marrow derived macrophages (BMDMs) or peritoneal macrophages was isolated by using the miRNeasy RNA kit (Qiagen) as indicated by the manufacturer. cDNA was then obtained by using Vilo reverse transcriptase (Life Technologies) as indicated by the manufacture. cDNA or plasmids were then amplified by PCR using the Pfu ultra II (Agilent Technologies) polymerase as indicated by the manufacture. Primers are described above. PCR was run in SensoQuest GmbH labcycler and purified using High Pure PCR product purification kit (Roche). After running the amplicons in 1% agarose gel, they were extracted using Jetquick gel extraction spin kit (Genomed). MiniPrep were performed using NucleoSpin Plasmid kit (Macherey-Nagel).

To express the EVIRs, a bidirectional lentiviral vector (LV) was used that was expressing a GFP sequence in antisense orientation, under the transcriptional control of a minimal cytomegalovirus (mCMV) promoter, and the anti-HER2 EVIR in sense orientation, under the transcriptional control of the spleen focus forming virus (SFFV) promoter **(****Figure 1****).** The resulting LVs were identified and named according to the intracellular component of the EVIR (being the extracellular domain, CHA21, constant), as follows: EVIR-N (dLNGFR-CHA21), EVIR-G (FcγRIIIa-CHA21), EVIR-F (FLT3-CHA21), EVIR-T (TLR4-CHA2), EVIR-C2 (CCR2-CHA21), EVIR-I (ITGB2-CHA21), EVIR-C (CSF2RB-CHA21), EVIR-C1 (CCR1-CHA21), EVIR-C5 (CCR5-CHA21), EVIR-CX (CXCR4-CHA21), EVIR-S (SELPLG-CHA21).

### Example 2: Expression and stability of an anti-HER2 EVIRs

The stability and expression profile of EVIRs in different cells was tested.

*Immortalized murine bone marrow (BM) derived macrophages (iBMM)* were described previously *(*Squadrito et al., 2014, Cell Rep., 8(5):1432-46*).*

Briefly, the cells were obtained by transducing mouse BM cells with a LV expressing the proto-oncogene SV40 large T-antigen. iBMMs were cultured in macrophage serum free medium (SFM medium, Life Technologies), supplemented with macrophage colony-stimulating factor (M-CSF, 50 ng/ml). iBMMs were then cultured in Iscove's Modified Dulbecco's Medium (IMDM, Sigma-Aldrich), supplemented with M-CSF (50 ng/ml), 20% fetal bovine serum (FBS, EuroClone Group), 5.5 mL L-glutamine (Life Technologies) and 5.5 ml penicillin-streptomycin (Life Technologies).

*Murine monocytes cell line (P388D1) and colon carcinoma cell line (MC38)* were cultured in IMDM supplemented with 10% FBS, glutamine and penicillin-streptomycin, as described above.

*Peritoneal macrophages (PMs), bone marrow derived dendritic cells (BMDCs) and bone marrow derived macrophages (BMDM)* were isolated from 5-6 weeks BL6C57 mice.

PMs were obtained by flushing the peritoneum of euthanized mice with phosphate-buffered saline (PBS). Cells were then seeded in plates with IMDM supplemented with M-CSF (50 ng/ml), after 1h non-adherent cells were discarded. BMDMs and BMDCs were obtained by flashing femurs and tibias with PBS. BM cells were then cultured for 8 days in IMDM or Roswell Park Memorial Institute (RPMI) medium respectively supplemented with M-CSF (50 ng Preprotech) for the BMDMs or GM-CSF (100 ng, Preprotech) for the BMDCs.

### Lentiviral vector production by transfection of 293T cells and cell transduction

Vesicular stomatitis virus (VSV)-pseudotyped, third-generation lentiviruses were produced by transient four-plasmid co-transfection into human embryonic kidney cells (293T) as described previously *(*De Palma et al., 2002, Methods in enzymology, 346: 514-529*).* Briefly, 9 million 293T cells were seeded in a 15 cm dish 24h before transfection in 20 ml of medium. 2h before transfection, medium was changed. Per plate, the plasmid DNA mix was prepared with envelope ENV plasmid (VSV-G, 9 µg), pMDLg/pRRE plasmid (12.5 µg), REV plasmid (6.25 µg), pADVANTAGE (15 µg) and transfer plasmid (32 µg). 125 µl of 2.5M CaCl₂ were added to the plasmid mix and 0.1 TE/dH20 (2:1) was used to have a final volume of 112 5µl. While vortexing at full speed this solution, 1125 µl of 2X HBS solution (pH 7.12) was added dropwise. The final HBS and plasmid solution was rapidly transferred on the cells. The medium was changed 12-14h later and 16 ml of fresh media was added per dish. The cell supernatant was collected and filtered (0.22 µm) 30 hours after and concentrated by ultracentrifugation using a Beckman ultracentrifuge equipped with a SW31Ti rotor, at 22'000rpm for 2h, at 20°C. Reagent compositions were as follows: 2X HBS (281 mM NaCl, 100 mM of 4-(2-hydroxyethyl)-1-piperazineethanesulfonic acid (HEPES buffer), 1.5 mM Na₂HPO₄), 0.1X TE buffer (10 mM Tris (pH 8.0), 1 mM EDTA (pH 8.0) diluted 1:10 with distilled H₂O). The stocks of LVs were kept at -80°C.

LVs (all expressing fluorescent proteins) were titered cells by dilutions ranging from 10-3 to 10-7 on 100'000 293T cells seeded in a 6-well plate the day before transduction. The percentage of positive cells was measured by flow cytometry 4 - 7 days after transduction. The titer was calculated applying the formula: TU/ml = 'number of cells' * 'percentage positive cells'/100/'dilution'. The 'percentage positive cells' used in the formula was always the lowest dilution with a value lower than 15%. 293T, P388D1 and iBMMs were transduced with LV doses ranging from 10⁶ to 10⁷ transducing units (TU)/ml. In experiments with double transduction (HER2-expressing LV and mCherry-expressing LV for instance), sequential transduction was performed i) transducing the cells with HER2-expressing LV, ii) washing and replating the cells and iii) transducing the cells with mCherry-expressing LV) 5-7 days after the first transduction.

### Immunofluorescence analysis of iBMMs

EVIR/Control-transduced iBMMs were seeded in a glass cover slide coated with fibronectin (200 µg/ml, Peprotech). The day after the medium was removed, the cells were washed 3 times for 1min with PBS. iBMMs were then fixed in 4% paraformaldehyde (PFA) for 15min at room temperature in the dark. After removing the PFA, 3 washes with PBS for 1 min were performed. The cells were then incubated for 30 min in blocking solution (0.1% Triton and 10% normal goat serum (NGS) in PBS). Then, a staining solution containing an anti-F(ab')2 antibody conjugated with Alexa Fluor 647 (Jackson ImmunoResearch) was prepared in blocking solution. 200 µl of staining solution were added per well for 4h. The staining solution was removed and the slides were washed with PBS 3x for 3 min. Phalloidin-Alexa Fluor 546 (Life Technologies) in blocking solution was added for 20 min. Then slides were washed 3x with PBS and finally 4',6-diamidino-2-phenylindole (DAPI) was added for 10 min and washed 5x for 3min. DAKO mounting medium was added and samples were dried overnight at RT. Images were acquired by confocal microscopy (Zeiss LSM 700 INVERT).

### Flow cytometry analysis

After transduction and cell culture, cells were detached by trypsin and stained with the appropriate antibodies before flow cytrometry analysis.

To validate the expression of EVIR-N, human kidney cells (293T cells), immortalized bonemarrow macrophages (iBMMs) and monocytes (P388D1 cells) were transduced with the EVIR-N LV or, as a control LV, a bidirectional LV that expresses GFP and dLNGFR (without the extracellular scFv domain). To measure EVIR-N expression, transduced cells were stained with an anti-F(ab')2 antibody, which recognizes the scFv domain of the EVIR. A robust surface expression of EVIR-N in all cell types was observed **(****Figure 2****).** Immunofluorescence staining analysis of iBMMs confirmed robust EVIR-N expression at the cell surface **(****Figure 3B****).**

The stability of EVIR-N-expressing monocytes/iBMMs over an extended period of time (5 weeks post-transduction) was analysed. GFP expression, which is indicative of the persistence of transduced cells, was stable in both P388D1 and iBMMs cells during the 5-week time window **(****Figure 4****).**

Additional EVIRs (as listed above) featuring a repertoire of distinct proteinic domains comprising transmembrane and intracellular signaling domains were tested. To investigate whether anti-HER2 EVIRs can be expressed in a sustained manner, we transduced iBMMs and P388D1 monocytes were transduced with the various GFP/EVIR and the control GFP/dLNGFR LVs. Flow cytometry showed heterogeneous expression of the different EVIRs at the cellular membrane, which was dependent on the EVIR or cell type tested **(****Figure 3A****).** Whereas EVIR-N was robustly expressed by either cell type, other EVIRs, such as EVIR-C, EVIR-CX and EVIR-I, were expressed less efficiently. Some EVIRs displayed differential expression among the two cell types, e.g., EVIR-G was highly expressed in P388D1 cells but much less so in iBMMs. Immunofluorescence analysis of iBMMs transduced with EVIR-N, EVIR-G, EVIR-T or control LVs further indicated that these EVIRs were detectably expressed, albeit to varying levels **(****Figure 3B****).** The stability of EVIR-expression over an extended period of time (5 weeks post-transduction) was investigated. For most EVIRs, GFP expression was stable in the iBMM and P388D1 cell lines during the 5-week time window **(****Figure 4****).** It is noted that a good expression of EVIRs is achieved when at least 1% of population of any cell type, preferentially monocytes, macrophage or dendritic cell (DC) expresses EVIRs and when this expression is detectable for a few weeks post-transduction.

Together, these results indicate that LVs can be used to stably express the anti-HER2 EVIRs on membranes of several cell types, including monocyte and macrophage-lineage cells.

### Example 3: Anti-HER2 EVIRs enhance monocyte/macrophage binding to cancer cells

EVIRs expression in cells on binding to monocytes/macrophages was tested.

### Binding assay in co-culture

Turquoise fluorescent protein (mTq) positive MC38 either expressing HER2 were obtained by transducing mTq⁺ MC38 with a HER2 expressing LV, which was obtained by substituting the GFP coding sequence of a PGK. GFP LV as described in Amendola et al., 2005 (Nat biotechnol, 23(1): 108-16*)* with a HER2 coding sequence as described in Leto et al., 2015 (Clin Cancer Res. 21(24):5519-31*).* mTq+ MC38, HER2 transduced or untransduced, were detached according to standard protocol and seeded in 24-well plates (Corning Costar), 20,000 cells/well. After 24h, iBMMs, transduced with either mCherry or GFP (EVIR)-expressing LVs, were detached according to standard protocols. 20,000 mCherry+ and GFP+ cells were mixed to a 1:1 ratio approx. and seeded on top of the mTq+ MC38 cells. After 24 h of co-culture, both cell types were detached with trypsin, stained with 7-AAD and analyzed by flow cytometry (LSRII, BD).

### Binding assay in suspension

P388D1 transduced with either control or EVIR-N LVs and mCherry MC38 (transduced with HER2-LV or UT) were detached and washed according to standard protocol and re-suspended in IMDM to a concentration of 5'000 cells/µl in a final volume of 30 µl. In a 0.5 ml tube (Eppendorf), the cells were mixed at different ratios (1:10, 1:1 and 10:1) and put on a rotating wheel for 3 hours at 20 rpm. The mix of cells was kept at 4°C in the dark to avoid internalization of the receptors and fluorescence squelching. After the incubation time, 30 µl 7-AAD 2X was added and cell suspensions were analyzed by flow cytometry (LSRII, BD).

It was tested whether EVIR-N expression facilitates monocyte/macrophage binding to HER2-expressing cancer cells. To this aim, colon cancer MC38 cells were transduced with a turquoise fluorescent protein (mTq)-expressing LV, with or without a HER2-expressing LV. Double-transduced MC38 cells, hereon termed HER2⁺mTq⁺ MC38 cells, were 69% HER2/mTq double-positive, whereas control mTq+ MC38 cells were 79% mTq-positive. In parallel, iBMMs were transduced with either EVIR-N/GFP or mCherry-expressing LVs (hereon termed EVIR⁺ GFP⁺ and mCherry+ iBMMs, respectively). The transduction efficiency of iBMMs was 78% and 97% for the EVIR-N/GFP and mCherry-expressing LVs, respectively. Next, a mixed population of EVIR-N⁺GFP⁺ and mCherry⁺ iBMMs (1:1) with either HER2⁺ mTq⁺ or mTq⁺ MC38 cells at high density was co-cultured. In this assay, the presence of mTq+GFP+ or mTq+mCherry+ double-positive cells is indicative of binding/aggregation between iBMMs and MC38 cancer cells. It was found that EVIR-N⁺GFP⁺ iBMMs could bind efficiently to HER2⁺mTq⁺ MC38 cells. The occurrence of mTq⁺GFP⁺ events was only present when EVIR-N-expressing iBMMs and HER2⁺ MC38 cancer cells were present in the co-culture **(****Figure 5A****).** Conversely, mCherry+ iBMMs did not bind to either mTq⁺ or HER2⁺mTq⁺ MC38 cells, indicating that macrophages bind MC38 cancer cells specifically through the anti-HER2 EVIR-N. Furthermore, EVIR-N⁺GFP⁺ iBMMs did not bind to HER2-negative MC38 cells, indicating that binding occurs only when HER2 is expressed on the surface of the cancer cells. Retro-gating of the mTq⁺GFP⁺ double-positive events showed that they fell in the non-singlet cell region of the physical parameter plot, thus proving binding/aggregation and excluding cell phagocytosis as a source of double fluorescence.

Next, it was investigated whether EVIR-N-expressing P388D1 cells could rapidly bind to HER2⁺ MC38 cells also in a cell suspension assay. To this aim, first HER2⁺ or HER2⁻ MC38 cells were transduced with an LV expressing mCherry. Next, P388D1 cells were transduced with the EVIR-N or control LV (which both express GFP). Several days after transduction mCheery⁺ MC38 cells either expressing HER2 or not, were co-incubated with the GFP+ P388D1 cells, either expressing EVIR-N or dLNGFR, for 2h at 4°C to avoid phagocytosis or nonspecific binding. In agreement with data obtained with iBMMs in co-culture, it was found that expression of the EVIR-N greatly enhanced the binding of P388D1 cells to HER2-positive cancer cells, both at low and high monocyte concentration **(****Figure 5B****).** Confocal analysis of the cells reveled that most of the double-positive events measured by flow cytometry were indeed aggregates containing both cell types.

Next, the co-culture experiments were performed to assay the binding of EVIR-expressing iBMMs to HER2-expressing MC38 cells, as described above. It was found that EVIR⁺GFP⁺ iBMMs could efficiently bind to HER2+mTq+ MC38 cells, in particular when EVIR-N, EVIR-C2, EVIR-F, EVIR-C1 or EVIR-C5 were used **(****Figure 5A****).**

These results show that anti-HER2 EVIRs expressed by monocytes/macrophages promote their binding to HER2-cancer cells.

### Example 4: Anti-HER2 EVIRs enhance tumor antigen uptake via EV internalization

Next it was tested if anti-HER2 EVIRs expressed by monocytes, macrophages or other APCs, could specifically enhance the uptake of tumor-associated antigens (TAAs) contained in cancer-cell derived extracellular vesicles (EVs), independent of contact with cancer cells.

### EV purification, measurement, and cell treatment

Five million HER2⁺/UT mCherry⁺ MC38 cells or HER2⁺/UT ovalbumin (OVA)⁺ MC38 were seeded in 15 cm plates in 16 ml of IMDM medium supplemented with 10% FBS (LifeTechnologies) previously ultracentrifuged for 16h at 4°C at 28,000 rpm in a Beckman ultracentrifuge equipped with a SW32Ti rotor. After 72 h medium was collected, debris were discarded by three steps differential centrifugation at 500 g for 5 min, 2000g for 5 min and finally 4600g x 20 min. Supernatants were then ultracentrifuged as described above, but for 1 to 10h. Pellets were re-suspended in 80 µl of PBS for 36 ml medium. EVs were then diluted 1:1000 in PBS and quantified by using a NanoSight apparatus (Malvern Instrument) using the standard protocol. Concentration of EVs ranged from 0.8x10⁷ to 6x10⁹ particles/µl.

In experiments to measure mCherry transfer, 20,000 iBMMs or BMDCs per well were seeded in 24 wells plate (Corning) in 500 µl their respective culture medium. 2x10⁹ EV-particles/500 µl were then added to the cells in the medium. After incubation (ranging from 5min to 48h) cells were analysed by flow cytometry (LSRII, BD). In experiments to measure mCherry by immunofluorescence, iBMMs were seeded as described in the methods and treated with 1x10⁹ EV-particles/250 µl. In experiments that measure EV uptake at increasing EV concentrations, BMDCs were seeded in flat-bottom 96 well plates (10,000 cells/200 µl medium/well) and treated with 0.8x10⁷, 4x10⁷, 2x10⁸, 1x10⁹, 3x10⁹, or 6x10⁹ mCherry⁺ EVs.

It is increasingly appreciated that cancer cells release EVs, encompassing exosomes or microvesicles, which may contain immunogenic TAAs (Zeelenberget al., 2011, Journal of Immunology (Baltimore, Md.: 1950), 187: 1281-1288). Based on the ability of the anti-HER2 EVIR-N to bind efficiently to HER2-positive cancer cells, it was hypothesized that EVIR-N-expressing APCs would also bind to cancer cell-derived EVs, internalize them, and present EV-derived TAAs. EVs released from mCheery⁺, HER2-positive or negative MC38 cells were isolated. Nanosight analysis using a NS3000 device (Malvern) confirmed the presence of EVs (diameter: 100-400 nm, mean ~150 nm) in medium conditioned by MC38 cells. Furthermore, flow cytometry analysis of the EVs showed well detectable expression of HER2 in EVs derived from HER2⁺ MC38 cells **(****Figure 6A****).** Next EVIR-N⁺GFP⁺ iBMMs were treated with matched amounts of EVs isolated from HER2⁺mCheery⁺ or HER2⁻mCheery⁺ MC38 cells. Twenty-four hours after treatment, the EVIR-N⁺GFP⁺ iBMMs displayed higher mCherry mean fluorescence intensity (MFI) when they had been exposed to HER2⁺mCheery ⁺ MC38 cell-derived EVs as compared to HER2⁻mCheery⁺ EVs **(****Figure 6B****).** It was also found that EVIR-N-expressing iBMMs did uptake HER2+mCheery+ MC38 cell-derived EVs more rapidly than control (EVIR-N⁻) iBMMs, without evidence for saturation within 48 hours **(****Figure 6C****).** In order to investigate whether the EVs were internalized or remained associated with the iBMM's cell surface, the EV-treated iBMMs were analyzed by confocal microscopy. It was found that the mCherry signal largely co-localized with the cell cytoplasm, indicating internalization of HER2+ EVs by the EVIR-N-expressing iBMMs **(****Figure 6D****).** Of note, EV internalization was also observed in co-culture experiments using mTq+ MC38 cells.

Next, it was investigated whether EVIR-N could enhance the internalization of cancer cell-derived EVs by dendritic cells (DCs). BMDCs were transduced with either the EVIR-N or the control LV. The transduced BMDCs were then treated with EVs isolated from either mCheery⁺HER2⁺ or mCheery⁺HER2⁻ MC38 cells. In agreement with findings in iBMMs, EVIR-N⁺ BMDCs internalized greater amounts of cancer-cell derived HER2⁺ EVs than control cells **(****Figure 6E****),** across a broad range of EV doses **(****Figure 6F****).** Furthermore, other EVIR-expressing iBMMs internalized EVs derived from HER2⁺mCheery⁺ MC38 cells more rapidly than EVs derived from control HER⁻mCheery⁺ MC38 cells, in particular when the EVIR-N, EVIR-C5 and EVIR-S were used **(****Figure 7****).**

Taken together, these results indicate that anti-HER2 EVIRs enhance the uptake of cancer-cell derived EVs by macrophages or DCs.

### Example 5: Anti-HER2 EVIRs enhance TAA presentation by APCs

It was tested whether enhanced EV internalization by EVIR-N-expressing DCs was associated with increased presentation of an unrelated TAA.

### OT-I T cell proliferation assay

OT-I CD8⁺ T cells were obtained from OT-I TCR transgenic mouse line, which produces MHC class I-restricted, ovalbumin-specific, CD8⁺ T cells (OT-I cells) *(Hogquist, et al., 1994, supra).* CD8⁺ OT-I T cells were purified from spleens obtained from OT-I BL6/C57 mice. First, we depleted CD11c⁺ cells by using an automatic MACS-separator with anti-CD11c microbeads (Miltenyi biotech). Subsequently, we positively selected CD8⁺ T cells using anti-CD8 microbeads (Miltenyi biotech). OT-I CD8⁺ T cells were stained with Cell Tracer-violet (Life Technologies) following the manufacturer's instructions. 2x10⁵ purified OT-I T cells were co-cultured in flat-bottom 96-well plate together with 2x10⁴ EVIR/Control-transduced BMDCs. EVs isolated from OVA MC38 cells transduced with HER2 or UT were added to the wells together with BMDCs and T cells at day zero and kept for 3 days. In the pre-incubation experiments BMDCs were incubated for 24h with the EVs isolated from MC38 cells, then washed with PBS and added to the OT-I T cells. Proliferation of CD8 T cells was measured at day 3 by flow cytometry (LSRII, BD) gating on CD8⁺ CD11b⁻7AAD⁻ T cells. Used antibodies were as follows: goat anti-F(ab')2- Alexa Fluor 647 (Jackson ImmunoResearch), anti-HER2- Alexa Fluor 647 (BioLegend, 24B2), anti-CD8 - PE (BioLegend, 53-6.7), anti-CD11b- APC-Cy7 (BioLegend, M1/70), anti-Fc(BD, 2.4G2). In the experiments to measure OT-I CD8 T cell proliferation, 8x10⁹/ml EV-particles were added to the BMDCs or BMDCs + T cells as described in the methods of Example 4. In experiments that measure OT-I CD8 T cell proliferation at different concentrations of EVs, the EVs were added as described in Example 4.

MC38 cells expressing OVA were transduced with a HER2-expressing LV. In parallel, CD8⁺ T cells from OT-I mice were isolated, which express an MHCI-restricted, anti-OVA TCR. HER2⁺OVA⁺ EVs isolated from MC38 were co-cultured for 72 h in the presence of, (i) GFP⁺ BMDCs, either expressing EVIR-N or dLNGFR and (ii) cell tracer-stained OT-I CD8⁺ T cells. Remarkably, significantly greater T cell proliferation was observed when the OT-I T cells were co-cultured together with EVIR-N-expressing BMDCs and HER2⁺OVA⁺ EVs, compared to other co-culture conditions **(Figure 8A).**

In order to understand whether EVIR-N+ BMDCs acquire the OVA antigen from cancer cell-derived EVs, the following cells were co-cultured for 72h (i) GFP+ BMDCs, either expressing EVIR-N or dLNGFR, pretreated with HER2⁺OVA⁺ EVs, and (ii) tracer-stained OT-I CD8+ T cells. Greater OT-I CD8⁺ T-cell proliferation in the presence of pretreated-EVIR-N⁺ BMDMs, compared to other conditions was observed **(Figure 8B).** Remarkably, OT-I CD8+ T-cell proliferation was not observed when OT-I CD8+ T cells were treated with HER2+OVA+ EVs in the absence of BMDCs, or when OT-I CD8+ T cells were co-cultured with GFP⁺ BMDCs, either expressing EVIR-N or dLNGFR **(Figure 8C).** Furthermore, EVIR-expressing BMDCs stimulated OT-I CD8⁺ T proliferation was observed also when low EV doses were employed in a dose-response assay **(****Figure 8D****).**

These results indicate that EVIR-N expression by DCs and BMDCs greatly enhances their ability to uptake, process, and present to T-cells, EV-associated TAAs.

### Example 6: EVIR-N2 and EVIR-N1 enhance EV uptake by APCs and promote cancer-specific T cell proliferation

Further EVIRs directed against two distinct melanoma-specific surface antigens, DG2 and TYRP1 were designed. An EVIR-N2 was designed, which is specific to the ganglioside GD2 expressed on melanoma cells. GD2 has been previously employed as a target of melanoma immunotherapy, for example, for the design of GD2-specific CAR-T cells *(*Yvon et al., Clin Cancer Res. 2009, 15(18):5852-60*).* Next, an EVIR-N1 was designed, which is specific to the melanoma antigen TYRP1 expressed on melanoma cells *(*Saenger et al., 2008, Cancer Res, 68(23); 9884-91*).* It was tested whether EVIR-N1 and EVIR-N2 enhance EV uptake by APCs and promote cancer-specific T cell proliferation.

### Cloning design of EVIR-N1 and EVIR-N2

Mouse-optimized TA99 scFv (SEQ ID NO: 111; anti-TYRP1) and mouse-optimized 14G2a scFv (SEQ ID NO: 112; anti-GD2) coding DNA sequences were obtained from GeneArt^{®} (LifeTechnologies). For both scFv sequences, a coding DNA sequence of IgK signal domain (SEQ ID NO: 109 for EVIR-N1 and SEQ ID NO: 110 for EVIR-N2) was incorporated to increase the export of the chimeric receptor to the cell membrane. A linker sequence containing a high efficiency Kozak sequence and restriction sites (SEQ ID NO: 3) was incorporated at the 5' end of the EVIR coding sequence. A restriction enzyme site for AgeI was incorporated at the 3'end of the scFv sequence for cloning (in frame with the rest of the DNA sequence) the transmembrane and intracellular domains of the EVIR. The IgK-anti-HER2 scFv CHA21 sequence was then replaced in the EVIR-N lentiviral with the IgK-anti-TYRP1 scFv TA99 or the IgK-anti-GD2 scFv 14G2a sequences, respectively and the DNA coding sequences from the truncated human nerve growth factor receptor (dLNGFR, SEQ ID NO: 43), as described in Example 1, was used in both constructs.

### Preparation of TYRP1⁺ cells and GD2⁺ cells

TYRP1⁺ B16 were obtained by transducing B16 cells (murine melanoma tumor cell line) with a mouse TYRP1-expressing LV. TYRP1 sequence was obtained by PCR using cDNA from B16 cells as template and the specific primers (TYRP1 Fw of SEQ ID NO: 134 and TYRP1 Rv of SEQ ID NO: 135). *GD2⁺* mCherry⁺MC38 cells were obtained by transducing mCherry⁺ MC38 cells with LVs expressing B4GALNT1 (Beta-1,4-N-Acetyl-Galactosaminyltransferase 1, GD2 synthase) and ST8SIA1 (ST8 Alpha-N-Acetyl-Neuraminide Alpha-2,8-Sialyltransferase 1, GD3 synthase), enzymes involved in GD2 synthesis *(*Dall'Olio et al., 2014, Biochim Biophys Acta, 1840(9):2752-64*).* Mouse optimized GD2 and GD3 synthase DNA sequences were obtained from GeneArt^{®} (LifeTechnologies). EVs derived from *GD2⁺* mCherry⁺MC38 and TYRP1⁺ B16 were obtained as described in the Example 4.

*Measurements and cell treatment* as described in the Example 4.

### OT-I T cell proliferation assay as described in the Example 5.

As shown in **Figure 9A-B,** the GD2 and TYRP1-specific EVIRs (EVIR-N2 and EVIR-N1, respectively) enhanced the uptake by engineered iBMMs of EVs expressing the respective bait antigens since EVIR-N2⁺ and EVIR-N1⁺ iBMMs internalized greater amounts of cancer-cell derived GD2⁺ EVs and TYRP1⁺ EVs than control cells **(****Figure 9A****,** **B).** Furthermore, BMDCs engineered to express an EVIR-N1 increased the proliferation of OT-I CD8⁺ T cells when co-incubated with EVs obtained from OVA⁺TYRP1⁺ melanoma cells (OVA⁺TYRP1⁺ B16), compared to BMDCs engineered to express a control EVIR **(****Figure 9C****).**

These data indicate that EVIRs can be designed against a variety of surface antigens expressed by cancer cells, including melanoma cells.

### Example 7: EVIRs can be co-delivered to APCs along with APC-stimulating factors

The co-expression of EVIRS with APC-stimulating factors has been tested as follows.

The DNA coding sequences of the APC-stimulating factors: *Cxcl9* (SEQ ID NO: 117), *Csf2* (SEQ ID NO: 120) and *IFNγ* (SEQ ID NO: 123) were obtained by PCR from cDNA of peritoneal macrophages as described in Example 2, and primers that contain restriction sites for XmaI, Sal and NheI were used **(Table 2).** The DNA-coding sequence for *Lin28* (SEQ ID NO: 126) was obtained by PCR from cDNA from mouse trophoblasts *(Baer et al., 2016, supra),* and primers that contain restriction sites for XmaI, Sal and NheI were used **(Table 2).** The DNA-coding sequence for CD40 (SEQ ID NO: 127) was obtained from GeneArt^{®} (LifeTechnologies). The DNA coding sequences of these APC-stimulating factors were then cloned under the transcriptional control of a minimal CMV promoter by replacing the GFP coding sequence in the bidirectional LV *(Amendola et al., 2005, supra)* described in the Example 1.

*EV purification, measurement, and cell treatment* as described in the Example 4.

*Measurement of EVIR expression as* described in the Example 2.

*Flow cytometry analysis of expression of surface molecules CD86 and CCR7* as described in Example 2.

**Table 2**

| **Gene name** | **Forward primer** | **Reverse Primer** |
|---|---|---|
| *Cxcl9* | AAAAACCCGGGTCACTCCAACACAGT GACTC (SEQ ID NO: 115) | AAAAAGTCGACGCTAGCCAGGGTGCTTGTTG GTAAAGT (SEQ ID NO: 116) |
| *Csf2* | AAAAACCCGGGCAGAGAGAAAGGCTA AGGTCC (SEQ ID NO: 118) | AAAAAGTCGACGCTAGCAGTCTGAGAAGCTG GATT (SEQ ID NO: 119) |
| *IFNy* | AAAAACCCGGGAGTTCTGGCTTCTCC TCCT (SEQ ID NO: 121) | AAAAAGTCGACGCTAGCGACAATCTCTTCCC CACCCC (SEQ ID NO: 122) |
| *Lin28* | AAAAAGGATCCCTTTGCCTCCGGACTT CTCTGG (SEQ ID NO: 124) | AAAAAGTCGACAAAGACAGGGTGACACTGG GA (SEQ ID NO: 125) |

As shown in **Figure 10A-B,** the co-expression of proteins that potentiate APC differentiation, activation, and presentation, and/or T-cell recruitment, did not compromise the expression of the EVIR or its ability to promote EV uptake in BMDCs. Further **(****Figure 10C-D),** it was found that the co-transduction of LIN28, a protein that blocks Let-7 miRNA activity, increased the expression in the BMDCs of surface molecules indicative of APC activation, e.g., CD86 (cluster of differentiation 86) and CCR7 (C-C chemokine receptor type 7). Together, these data indicate that the co-delivery of accessory proteins along with the EVIR not only does not impair their EV-internalizing activity, but can also help enhance APC functions.

### Example 8: EVIRs can promote the transfer of MHC-antigen complexes from the cancer cells to the APCs

Potential EVIR-mediated transfer of MHCI-antigen complexes from cancer cells to APCs was tested as follows.

### Disruption of MHCI expression in cancer cells

In order to disrupt *B2m* and abrogate MHCI expression in cancer cells, we generated a self-inducible LV based on the CRISPR/Cas9 system. We obtained a TetO-CAS9 LV also expressing a reverse tTA (rtTA) and a selection marker (puromycin). This LV was further modified to include a U6 promoter-driven anti-B2m single guide RNA (sgRNA) sequence (SEQ ID NO: 136), which was cloned upstream to the TetO-CAS9 expression cassette to obtain the doxycycline-self-inducible LV: U6-sgRNA.TetO-CAS9.Pgk-PURO/2A/rtTA LV. B2M-deficient EVs were obtained by transducing HER2⁺OVA⁺MC38 cancer cells with the above LV. Transduced HER2⁺OVA⁺MC38 cells were cultured in a cell medium containing puromycin (2µg/ml) and doxycycline (10µg/ml) for 3 days, in order to activate the CRISPR/Cas9 system and select a clonal population of cells with disrupted *B2m* gene **(****Figure 11A****).** EVs were then purified as described in the Example 4.

In the experiment, the BMDCs were isolated from the BM of B2M^{-/-} mice, which lack the ability of presenting MHCI/antigen complexes *(*Koller et al., 1990, Science, 248(4960):1227-30*).* The MHCI-deficient B2M⁻ BMDCs were then transduced with either a control EVIR or an EVIR-N and assayed in T-cell proliferation assays as those described in Example 5.

Under specific experimental conditions whereby the APCs are genetically modified to lack the ability of cross-presenting, the direct transfer of MHC complexes from cancer cell-derived EVs to EVIR-expressing APCs was sufficient to promote T-cell proliferation **(****Figure 11B****).** The co-incubation of the MHCI-deficient BMDCs and OT-I T cells with OVA+HER2+ EVs led to increased T cell proliferation in the presence of the EVIR-N (versus control EVIR), suggesting enhanced MHCI cross-dressing **(****Figure 11B****, top panels).** On the other hand, disruption of B2M in the cancer cells using the LV described above (*U6-sgRNA.TetO-CAS9.Pgk-PURO*/*2A*/*rtTA)* completely blocked cross-dressing from purified EVs, proving the specificity of this phenomenon **(****Figure 11B****, bottom panels).**

These results support that direct transfer of MHC complexes from cancer cell-derived EVs to EVIR-expressing APCs may be sufficient to promote T-cell proliferation in the absence of endogenous MHCI in the APCs.

### Example 9: EVIR-engineered APCs inhibit tumor growth in mice

The action of EVIR was tested in *in vivo* with EVIR-N DC-based vaccination experiment.

The DCs were obtained from the BM of syngenic mice, transduced with LVs, and activated with LPS (lipopolysaccharides, 10ng/ml) prior to their inoculation (two sequential DC doses of 10⁷ cells, one week apart) in mice carrying small, established tumors (n=4 in no DCs, 7 in CTRL-DCs and 9 in EVIR DCs mice/group).

It was found that the subcutaneous, peri-tumoral deployment of EVIR-N transduced DCs inhibited the growth of MC38-HER2 tumors, compared to control EVIR-transduced DCs **(****Figure 12****).** Although the control EVIR-transduced DCs also delayed tumor growth, the EVIR-N-transduced DCs significantly improved tumor control, demonstrating the superior anti-tumoral activity of the EVIR engineered DCs.

Those data demonstrate that EVIR-engineered APCs can enhance inhibition of tumour growth *in vivo.*

### Sequence listing

Nucleic acid sequence of anti-HER2 scFv CHA21
   SEQ ID NO: 1: 
GGGGATATTGTCCTCACACAGACTCCCAGCTCCCTGCCTGTGTCCGTCGGAGAGAAAGTGACCATGAC
ATGCAAGTCTAGTCAGACACTGCTCTACTCTAACAATCAGAAGAACTACCTCGCATGGTATCAGCAGA
AACCAGGACAGAGCCCCAAGCTGCTCATCTCCTGGGCTTTCACCCGGAAATCCGGGGTGCCTGACCGC
TTCACAGGTAGCGGCTCCGGAACTGATTTTACTCTGACCATTGGATCTGTGAAGGCAGAGGACCTCGC
CGTCTACTATTGCCAGCAGTACAGTAATTATCCATGGACTTTTGGCGGAGGGACCAGGCTGGAAATCA
AGAGAGGTGGAGGAGGGTCCGGTGGAGGAGGGTCTGGTGGAGGAGGGAGTGGTGGAGGAGGGTCAGAG
GTGCAGCTGCAGCAGTCTGGCCCCGAAGTGGTCAAAACTGGAGCTTCAGTCAAAATCAGCTGTAAGGC
ATCTGGGTACAGCTTCACCGGCTACTTCATCAACTGGGTGAAGAAAAATTCAGGGAAGAGCCCTGAGT
GGATCGGCCACATTTCAAGCTCCTACGCCACAAGCACTTACAACCAGAAGTTCAAAAATAAGGCCGCT
TTTACCGTGGACACATCTAGTTCAACCGCCTTCATGCAGCTGAACTCCCTCACATCTGAAGATAGTGC
TGTGTACTATTGTGTCAGGAGCGGCAACTACGAAGAATATGCTATGGATTACTGGGGGCAGGGGACCT
CCGTGACTGTCTCAAGC
Nucleic acid sequence of IgK domain
   SEQ ID NO: 2:
   ATGGATTTTCAGGTCCAGATTTTCTCCTTCCTCCTCATTTCAGCCAGCGTCATTATGTCTCGG
Nucleic acid sequence of S5_BamHI_Kozak
   SEQ ID NO: 3: GGATCCGCCACC
Nucleic acid sequence of S3_BamHI_AgeI_MluI_SalI_stop_XhoI
   SEQ ID NO: 4: ACCGGTACGCGTGTCGACTGACTCGAG
Nucleic acid sequence of S5_FCgRIIIa_BamHI_Kozak.start_AgeI
   SEQ ID NO: 5: GGATCCGCCACCATGACCGGT
Nucleic acid sequence of S3_FCgRIIIa_MluI_SalI_stop_XhoI
   SEQ ID NO: 6: ACGCGTGTCGACTGACTCGAG
Nucleic acid sequence of dLNGFR_Fw_AgeI
   SEQ ID NO: 7: AAAAAACCGGTCTTCTGGGGGTGTCCCTTG
Nucleic acid sequence of dLNGFR_Rv_MluI
   SEQ ID NO: 8: AAAAAACGCGTAGTTAGCCTCCCCCATCTCC
Nucleic acid sequence of FLT3_Fw_AgeI
   SEQ ID NO: 9: AAAAAACCGGTCCAGGCCCCTTCCCTTTCATC
Nucleic acid sequence of FLT3_Rv_XhoI
   SEQ ID NO: 10: AAAAACTCGAGAGAGGCGAGGCTAATCTTGG
Nucleic acid sequence of Tlr4_Fw_AgeI
   SEQ ID NO: 11: AAAAAACCGGTCAGCTGTATTCCCTCAGCACT
Nucleic acid sequence of Tlr4_Rv_SalI
   SEQ ID NO: 12: AAAAAGTCGACTGGGTTTAGGCCCCAGAGTT
Nucleic acid sequence of Ccr2_Fw_AgeI
   SEQ ID NO: 13: AAAAAACCGGTATGGAAGACAATAATATGTTACCTC
Nucleic acid sequence of Ccr2_Rv_MluI
   SEQ ID NO: 14: AAAAAACGCGTATGTACAAACTGCTCCCTCC
Nucleic acid sequence of Itgb2_Fw_AgeI
   SEQ ID NO: 15: AAAAAACCGGTAATGCACGGCTGGTAGAGTG
Nucleic acid sequence of Itgb2_Rv_MluI
   SEQ ID NO: 16: AAAAAACGCGTGGGGGTCACATCTGCTTGAT
Nucleic acid sequence of Csf2rb_Fw_AgeI
   SEQ ID NO: 17: AAAAAACCGGTACTCAGAAGATGGCTTACTCATTCA
Nucleic acid sequence of Csf2rb_Rv_MluI
   SEQ ID NO: 18: AAAAAACGCGTTGGTGAGATTGGGAGGAGAC
Nucleic acid sequence of Ccr1_Fw_AgeI
   SEQ ID NO: 19: AAAAAACCGGTACTCCATGCCAAAAGACTGCT
Nucleic acid sequence of Ccr1_Rv_MluI
   SEQ ID NO: 20: AAAAAACGCGTACCTTCCTTGGTTGACACCTATG
Nucleic acid sequence of Ccr5_Fw_AgeI
   SEQ ID NO: 21: AAAAAACCGGTATGTCAGCACCCTGCCAAAAA
Nucleic acid sequence of Ccr5_Rv_MluI
   SEQ ID NO: 22: AAAAAACGCGTCATTCCTACTCCCAAGCTGCAT
Nucleic acid sequence of Cxcr4_Fw_XmaI
   SEQ ID NO: 23: AAAAACCCGGGTTCCGGGATGAAAACGTCCA
Nucleic acid sequence of Cxcr4_Rv_MluI
   SEQ ID NO: 24: AAAAAACGCGTTGCATAAGTGTTAGCTGGAGTG
Nucleic acid sequence of Selplg_Fw_AgeI
   SEQ ID NO: 25: AAAAAACCGGTATTGCCACCACTGACCCTA
Nucleic acid sequence of Selplg_Rv_MluI
   SEQ ID NO: 26: AAAAAACGCGTGCAAAGGTCTCGCTTAGGTG
Amino acid sequence of anti-HER2 CHA21
   SEQ ID NO: 27: 
DIVLTQTPSSLPVSVGEKVTMTCKSSQTLLYSNNQKNYLAWYQQKPGQSPKLLISWAFTRKSGVPDRF
TGSGSGTDFTLTIGSVKAEDLAVYYCQQYSNYPWTFGGGTRLEIKRGGGGSGGGGSGGGGSGGGGSEV
QLQQSGPEVVKTGASVKISCKASGYSFTGYFINWVKKNSGKSPEWIGHISSSYATSTYNQKFKNKAAF
TVDTSSSTAFMQLNSLTSEDSAVYYCVRSGNYEEYAMDYWGQGTSVTVSS
Amino acid sequence of anti-HER2 trastuzumab-based scFv
   SEQ ID NO: 28: 
DIQMTQSPSSLSASVGDRVTITCRASQDVNTAVAWYQQKPGKAPKLLIYSASFLYSGVPSRFSGSRSG
TDFTLTISSLQPEDFATYYCQQHYTTPPTFGQGTKVEIKRTGSTSGSGKPGSGEGSEVQLVESGGGLV
QPGGSLRLSCAASGFNIKDTYIHWVRQAPGKGLEWVARIYPTNGYTRYADSVKGRFTISADTSKNTAY
LQMNSLRAEDTAVYYCSRWGGDGFYAMDVWGQGTLVTVSSGTGTRX
Amino acid sequence of anti-HER2 pertuzumab-based scFv
   SEQ ID NO: 29: 
DIQMTQSPSSLSASVGDRVTITCKASQDVSIGVAWYQQKPGKAPKLLIYSASYRYTGVPSRFSGSGSG
TDFTLTISSLQPEDFATYYCQQYYIYPYTFGQGTKVEIKRTVAAPGGGGSGGGGSGGGGSGGGGSEVQ
LVESGGGLVQPGGSLRLSCAASGFTFTDYTMDWVRQAPGKGLEWVADVNPNSGGSIYNQRFKGRFTLS
VDRSKNTLYLQMNSLRAEDTAVYYCARNLGPSFYFDYW
Amino acid sequence of anti-HER2 FRP5-based scFv
   SEQ ID NO: 30: 
QVQLQQSGPELKKPGETVKISCKASGYPFTNYGMNWVKQAPGQGLKWMGWINTSTGESTFADDFKGRF
DFSLETSANTAYLQINNLKSEDMATYFCARWEVYHGYVPYWGQGTTVTVSSGGGGSGGGGSGGGGSDI
QLTQSHKFLSTSVGDRVSITCKASQDVYNAVAWYQQKPGQSPKLLIYSASSRYTGVPSRFTGSGSGPD
FTFTISSVQAEDLAVYFCQQHFRTPFTFGSGTKLEIKRX
Amino acid sequence of proteinic domain derived from dLNGFR
   SEQ ID NO: 31: 
LLGVSLGGAKEACPTGLYTHSGECCKACNLGEGVAQPCGANQTVCEPCLDSVTFSDVVSATEPCKPCT
ECVGLQSMSAPCVEADDAVCRCAYGYYQDETTGRCEACRVCEAGSGLVFSCQDKQNTVCEECPDGTYS
DEANHVDPCLPCTVCEDTERQLRECTRWADAECEEIPGRWITRSTPPEGSDSTAPSTQEPEAPPEQDL
IASTVAGVVTTVMGSSQPVVTRGTTDNLIPVYCSILAAVVVGLVAYIAFKRWNRGIL
Amino acid sequence of proteinic domain derived from FcγRIIIA
   SEQ ID NO: 32: 
HENSELLIPKATHNDSGSYFCRGLIGHNNKSSASFRISLGDPGSPSMFPPWHQITFCLLIGLLFAIDT
VLYFSVRRGLQSPVADYEEPKIQWSKEPQDKTRVD
Amino acid sequence of proteinic domain derived from FLT3
   SEQ ID NO: 33: 
PGPFPFIQDNISFYATIGLCLPFIVVLIVLICHKYKKQFRYESQLQMIQVTGPLDNEYFYVDFRDYEY
DLKWEFPRENLEFGKVLGSGAFGRVMNATAYGISKTGVSIQVAVKMLKEKADSCEKEALMSELKMMTH
LGHHDNIVNLLGACTLSGPVYLIFEYCCYGDLLNYLRSKREKFHRTWTEIFKEHNFSFYPTFQAHSNS
SMPGSREVQLHPPLDQLSGFNGNLIHSEDEIEYENQKRLAEEEEEDLNVLTFEDLLCFAYQVAKGMEF
LEFKSCVHRDLAARNVLVTHGKVVKICDFGLARDILSDSSYVVRGNARLPVKWMAPESLFEGIYTIKS
DVWSYGILLWEIFSLGVNPYPGIPVDANFYKLIQSGFKMEQPFYATEGIYFVMQSCWAFDSRKRPSFP
NLTSFLGCQLAEAEEAMYQNMGGNVPEHPSIYQNRRPLSREAGSEPPSPQAQVKIHGERS
Amino acid sequence of proteinic domain derived from TLR4
   SEQ ID NO: 34: 
QLYSLSTLDCSFNRIETSKGILQHFPKSLAFFNLTNNSVACICEHQKFLQWVKEQKQFLVNVEQMTCA
TPVEMNTSLVLDFNNSTCYMYKTIISVSVVSVIVVSTVAFLIYHFYFHLILIAGCKKYSRGESIYDAF
VIYSSQNEDWVRNELVKNLEEGVPRFHLCLHYRDFIPGVAIAANIIQEGFHKSRKVIVVVSRHFIQSR
WCIFEYEIAQTWQFLSSRSGIIFIVLEKVEKSLLRQQVELYRLLSRNTYLEWEDNPLGRHIFWRRLKN
ALLDGKASNPEQTAEEEQETATWT
Amino acid sequence of proteinic domain derived from CCR2
   SEQ ID NO: 35: 
MEDNNMLPQFIHGILSTSHSLFTRSIQELDEGATTPYDYDDGEPCHKTSVKQIGAWILPPLYSLVFIF
GFVGNMLVIIILIGCKKLKSMTDIYLLNLAISDLLFLLTLPFWAHYAANEWVFGNIMCKVFTGLYHIG
YFGGIFFIILLTIDRYLAIVHAVFALKARTVTFGVITSVVTWVVAVFASLPGIIFTKSKQDDHHYTCG
PYFTQLWKNFQTIMRNILSLILPLLVMVICYSGILHTLFRCRNEKKRHRAVRLIFAIMIVYFLFWTPY
NIVLFLTTFQESLGMSNCVIDKHLDQAMQVTETLGMTHCCINPVIYAFVGEKFRRYLSIFFRKHIAKR
LCKQCPVFYRETADRVSSTFTPSTGEQEVSVGL
Amino acid sequence of proteinic domain derived from ITGB2
   SEQ ID NO: 36: 
NARLVECSGRGHCQCNRCICDEGYQPPMCEDCPSCGSHCRDNHTSCAECLKFDKGPFEKNCSVQCAGM
TLQTIPLKKKPCKERDSEGCWITYTLQQKDGRNIYNIHVEDSLECVKGPNVAAIVGGTVVGVVLIGVL
LLVIWKALTHLTDLREYRRFEKEKLKSQWNNDNPLFKSATTTVMNPKFAES
Amino acid sequence of proteinic domain derived from CSF2RB
   SEQ ID NO: 37: 
TQKMAYSFIEHTFQVQYKKKSDSWEDSKTENLDRAHSMDLSQLEPDTSYCARVRVKPISNYDGIWSKW
SEEYTWKTDWVMPTLWIVLILVFLILTLLLILRFGCVSVYRTYRKWKEKIPNPSKSLLFQDGGKGLWP
PGSMAAFATKNPALQGPQSRLLAEQQGESYAHLEDNNVSPLTIEDPNIIRVPPSGPDTTPAASSESTE
QLPNVQVEGPTPNRPRKQLPSFDFNGPYLGPPQSHSLPDLPDQLGSPQVGGSLKPALPGSLEYMCLPP
GGQAQLVPLSQVMGQGQAMDVQCGSSLETSGSPSVEPKENPPVELSMEEQEARDNPVTLPISSGGPEG
SMMASDYVTPGDPVLTLPTGPLSTSLGPSLGLPSAQSPRLCLKLPRVPSGSPALGPPGFEDYVELPPS
VSQAAKSPPGHPAPPVASSPTVIPGEPREEVGPASPHPEGLLVLQQVGDYCFLPGLGPGSLSPHSKPP
SPSLCSETEDLVQDLSVKKFPYQPMPQAPAIQFFKSLKHQDYLSLPPWDNSQSGKVC
Amino acid sequence of proteinic domain derived from CCR1
   SEQ ID NO: 38: 
TPCQKTAVRAFGAGLLPPLYSLVFIIGVVGNVLVILVLMQHRRLQSMTSIYLFNLAVSDLVFLFTLPF
WIDYKLKDDWIFGDAMCKLLSGFYYLGLYSEIFFIILLTIDRYLAIVHAVFALRARTVTFGIITSIIT
WALAILASMPALYFFKAQWEFTHRTCSPHFPYKSLKQWKRFQALKLNLLGLILPLLVMIICYAGIIRI
LLRRPSEKKVKAVRLIFAITLLFFLLWTPYNLSVFVSAFQDVLFTNQCEQSKQLDLAMQVTEVIAYTH
CCVNPIIYVFVGERFWKYLRQLFQRHVAIPLAKWLPFLSVDQLERTSSISPSTGEHELSAGF
Amino acid sequence of proteinic domain derived from CCR5
   SEQ ID NO: 39: 
MSAPCQKINVKQIAAQLLPPLYSLVFIFGFVGNMMVFLILISCKKLKSVTDIYLLNLAISDLLFLLTL
PFWAHYAANEWVFGNIMCKVFTGLYHIGYFGGIFFIILLTIDRYLAIVHAVFALKVRTVNFGVITSVV
TWAVAVFASLPEIIFTRSQKEGFHYTCSPHFPHTQYHFWKSFQTLKMVILSLILPLLVMVICYSGILH
TLFRCRNEKKRHRAVRLIFAIMIVYFLFWTPYNIVLLLTTFQEFFGLNNCSSSNRLDQAMQATETLGM
THCCLNPVIYAFVGEKFRSYLSVFFRKHIVKRFCKRCSIFQQDNPDRASSVYTRSTGEHEVSTGL
Amino acid sequence of proteinic domain derived from CXCR4
   SEQ ID NO: 40: 
FRDENVHFNRIFLPTIYFIIFLTGIVGNGLVILVMGYQKKLRSMTDKYRLHLSVADLLFVITLPFWAV
DAMADWYFGKFLCKAVHIIYTVNLYSSVLILAFISLDRYLAIVHATNSQRPRKLLAEKAVYVGVWIPA
LLLTIPDFIFADVSQGDISQGDDRYICDRLYPDSLWMVVFQFQHIMVGLVLPGIVILSCYCIIISKLS
HSKGHQKRKALKTTVILILAFFACWLPYYVGISIDSFILLGVIKQGCDFESIVHKWISITEALAFFHC
CLNPILYAFLGAKFKSSAQHALNSMSRGSSLKILSKGKRGGHSSVSTESESSSFHSS
Amino acid sequence of proteinic domain derived from SELPLG
   SEQ ID NO: 41: 
IATTDPTAPGTGGTAVGMLSTDSATQWSLTSVETVQPASTEVETSQPAPMEAETSQPAPMEAETSQPA
PMEADTSKPAPTEAETSKPAPTEAETSQPAPNEAETSKPAPTEAETSKPAPTEAETTQLPRIQAVKTL
FTTSAATEVPSTEPTTMETASTESNESTIFLGPSVTHLPDSGLKKGLIVTPGNSPAPTLPGSSDLIPV
KQCLLIILILASLATIFLVCTVVLAVRLSRKTHMYPVRNYSPTEMICISSLLPEGGDGAPVTANGGLP
KVQDLKTEPSGDRDGDDLTLHSFLP
Amino acid sequence of IgK domain
   SEQ ID NO: 42: MDFQVQIFSFLLISASVIMSRG
Nucleic acid sequence of proteinic domain derived from dLNGFR
   SEQ ID NO: 43: 
CTTCTGGGGGTGTCCCTTGGAGGTGCCAAGGAGGCATGCCCCACAGGCCTGTACACACACAGCGGTGA
GTGCTGCAAAGCCTGCAACCTGGGCGAGGGTGTGGCCCAGCCTTGTGGAGCCAACCAGACCGTGTGTG
AGCCCTGCCTGGACAGCGTGACGTTCTCCGACGTGGTGAGCGCGACCGAGCCGTGCAAGCCGTGCACC
 
GAGTGCGTGGGGCTCCAGAGCATGTCGGCGCCGTGCGTGGAGGCCGACGACGCCGTGTGCCGCTGCGC
CTACGGCTACTACCAGGATGAGACGACTGGGCGCTGCGAGGCGTGCCGCGTGTGCGAGGCGGGCTCGG
GCCTCGTGTTCTCCTGCCAGGACAAGCAGAACACCGTGTGCGAGGAGTGCCCCGACGGCACGTATTCC
GACGAGGCCAACCACGTGGACCCGTGCCTGCCCTGCACCGTGTGCGAGGACACCGAGCGCCAGCTCCG
CGAGTGCACACGCTGGGCCGACGCCGAGTGCGAGGAGATCCCTGGCCGTTGGATTACACGGTCCACAC
CCCCAGAGGGCTCGGACAGCACAGCCCCCAGCACCCAGGAGCCTGAGGCACCTCCAGAACAAGACCTC
ATAGCCAGCACGGTGGCAGGTGTGGTGACCACAGTGATGGGCAGCTCCCAGCCCGTGGTGACCCGAGG
CACCACCGACAACCTCATCCCTGTCTATTGCTCCATCCTGGCTGCTGTGGTTGTGGGCCTTGTGGCCT
ACATAGCCTTCAAGAGGTGGAACAGGGGGATCCTCTAG
Nucleic acid sequence of proteinic domain derived from FcγRIIIA
   SEQ ID NO: 44: 
CACGAGAACTCCGAACTGCTGATTCCTAAGGCAACTCACAACGACTCCGGCTCCTATTTCTGTAGAGG
GCTGATTGGACATAACAACAAGAGCTCCGCCTCATTCAGGATTAGCCTGGGCGACCCAGGGTCTCCCA
GTATGTTCCCCCCTTGGCACCAGATCACCTTTTGCCTGCTGATTGGACTGCTGTTCGCTATCGATACA
GTGCTGTACTTTTCTGTCCGGAGAGGCCTGCAGTCACCCGTGGCAGATTACGAAGAACCCAAGATTCA
GTGGAGCAAGGAGCCCCAGGATAAGACGCGTGTCGACTGA
Nucleic acid sequence of proteinic domain derived from FLT3
   SEQ ID NO: 45: 
CCAGGCCCCTTCCCTTTCATCCAAGACAACATCTCCTTCTATGCGACCATTGGGCTCTGTCTCCCCTT
CATTGTTGTTCTCATTGTGTTGATCTGCCACAAATACAAAAAGCAATTTAGGTACGAGAGTCAGCTGC
AGATGATCCAGGTGACTGGCCCCCTGGATAACGAGTACTTCTACGTTGACTTCAGGGACTATGAATAT
GACCTTAAGTGGGAGTTCCCGAGAGAGAACTTAGAGTTTGGGAAGGTCCTGGGGTCTGGCGCTTTCGG
GAGGGTGATGAACGCCACGGCCTATGGCATTAGTAAAACGGGAGTCTCAATTCAGGTGGCGGTGAAGA
TGCTAAAAGAGAAAGCTGACAGCTGTGAAAAAGAAGCTCTCATGTCGGAGCTCAAAATGATGACCCAC
CTGGGACACCATGACAACATCGTGAATCTGCTGGGGGCATGCACACTGTCAGGGCCAGTGTACTTGAT
TTTTGAATATTGTTGCTATGGTGACCTCCTCAACTACCTAAGAAGTAAAAGAGAGAAGTTTCACAGGA
CATGGACAGAGATTTTTAAGGAACATAATTTCAGTTTTTACCCTACTTTCCAGGCACATTCAAATTCC
AGCATGCCTGGTTCACGAGAAGTTCAGTTACACCCGCCCTTGGATCAGCTCTCAGGGTTCAATGGGAA
TTTAATTCATTCTGAAGATGAGATTGAATATGAAAACCAGAAGAGGCTGGCAGAAGAAGAGGAGGAAG
ATTTGAACGTGCTGACGTTTGAAGACCTCCTTTGCTTTGCGTACCAAGTGGCCAAAGGCATGGAATTC
CTGGAGTTCAAGTCGTGTGTCCACAGAGACCTGGCAGCCAGGAATGTGTTGGTCACCCACGGGAAGGT
GGTGAAGATCTGTGACTTTGGACTGGCCCGAGACATCCTGAGCGACTCCAGCTACGTCGTCAGGGGCA
ACGCACGGCTGCCGGTGAAGTGGATGGCACCTGAGAGCTTATTTGAAGGGATCTACACAATCAAGAGT
GACGTCTGGTCCTACGGCATCCTTCTCTGGGAGATATTTTCACTGGGTGTGAACCCTTACCCTGGCAT
TCCTGTCGACGCTAACTTCTATAAACTGATTCAGAGTGGATTTAAAATGGAGCAGCCATTCTATGCCA
CAGAAGGGATATACTTTGTAATGCAATCCTGCTGGGCTTTTGACTCAAGGAAGCGGCCATCCTTCCCC
AACCTGACTTCATTTTTAGGATGTCAGCTGGCAGAGGCAGAAGAAGCGATGTATCAGAACATGGGTGG
CAACGTCCCAGAACATCCATCCATCTACCAAAACAGGCGGCCCCTCAGCAGAGAGGCAGGCTCAGAGC
CGCCATCGCCACAGGCCCAGGTGAAGATTCACGGAGAAAGAAGTTAG
Nucleic acid sequence of proteinic domain derived from TLR4
   SEQ ID NO: 46: 
CAGCTGTATTCCCTCAGCACTCTTGATTGCAGTTTCAATCGCATAGAGACATCTAAAGGAATACTGCA
ACATTTTCCAAAGAGTCTAGCCTTCTTCAATCTTACTAACAATTCTGTTGCTTGTATATGTGAACATC
AGAAATTCCTGCAGTGGGTCAAGGAACAGAAGCAGTTCTTGGTGAATGTTGAACAAATGACATGTGCA
ACACCTGTAGAGATGAATACCTCCTTAGTGTTGGATTTTAATAATTCTACCTGTTATATGTACAAGAC
AATCATCAGTGTGTCAGTGGTCAGTGTGATTGTGGTATCCACTGTAGCATTTCTGATATACCACTTCT
ATTTTCACCTGATACTTATTGCTGGCTGTAAAAAGTACAGCAGAGGAGAAAGCATCTATGATGCATTT
GTGATCTACTCGAGTCAGAATGAGGACTGGGTGAGAAATGAGCTGGTAAAGAATTTAGAAGAAGGAGT
GCCCCGCTTTCACCTCTGCCTTCACTACAGAGACTTTATTCCTGGTGTAGCCATTGCTGCCAACATCA
TCCAGGAAGGCTTCCACAAGAGCCGGAAGGTTATTGTGGTAGTGTCTAGACACTTTATTCAGAGCCGT
TGGTGTATCTTTGAATATGAGATTGCTCAAACATGGCAGTTTCTGAGCAGCCGCTCTGGCATCATCTT
CATTGTCCTTGAGAAGGTTGAGAAGTCCCTGCTGAGGCAGCAGGTGGAATTGTATCGCCTTCTTAGCA
 
GAAACACCTACCTGGAATGGAGGACAATCCTCTGGGGAGGCACATCTTCTGGAGAAGACTTAAAAT
GCCCTATTGGATGGAAAAGCCTCGAATCCTGAGCAAACAGCAGAGGAAGAACAAGAAACGGCAACTTG
GACCTGA
Nucleic acid sequence of proteinic domain derived from CCR2
   SEQ ID NO: 47: 
ATGGAAGACAATAATATGTTACCTCAGTTCATCCATGGCATACTATCAACATCTCATTCTCTATTTAC
ACGAAGTATCCAAGAGCTTGATGAAGGGGCCACCACACCGTATGACTACGATGATGGTGAGCCTTGTC
ATAAAACCAGTGTGAAGCAAATTGGAGCTTGGATCCTGCCTCCACTCTACTCCCTGGTATTCATCTTT
GGTTTTGTGGGCAACATGTTGGTCATTATAATTCTGATAGGCTGTAAAAAGCTGAAGAGCATGACTGA
TATCTATCTGCTCAACCTGGCCATCTCTGACCTGCTCTTCCTGCTCACATTACCATTCTGGGCTCACT
ATGCTGCAAATGAGTGGGTCTTTGGGAATATAATGTGTAAAGTATTCACAGGGCTCTATCACATTGGT
TATTTTGGTGGAATCTTTTTCATTATCCTCCTGACAATTGATAGGTACTTGGCTATTGTTCATGCTGT
GTTTGCTTTAAAAGCCAGGACAGTTACCTTTGGGGTGATAACAAGTGTAGTCACTTGGGTGGTGGCTG
TGTTTGCCTCTCTACCAGGAATCATATTTACTAAATCCAAACAAGATGATCACCATTACACCTGTGGC
CCTTATTTTACACAACTATGGAAGAATTTCCAAACAATAATGAGAAATATCTTGAGCCTGATCCTGCC
TCTACTTGTCATGGTCATCTGCTACTCAGGAATTCTCCACACCCTGTTTCGCTGTAGGAATGAGAAGA
AGAGGCACAGGGCTGTGAGGCTCATCTTTGCCATCATGATTGTCTACTTTCTCTTCTGGACTCCATAC
AATATTGTTCTCTTCTTGACCACCTTCCAGGAATCCTTGGGAATGAGTAACTGTGTGATTGACAAGCA
CTTAGACCAGGCCATGCAGGTGACAGAGACTCTTGGAATGACACACTGCTGCATTAATCCTGTCATTT
ATGCCTTTGTTGGAGAGAAGTTCCGAAGGTATCTCTCCATATTTTTCAGAAAGCACATTGCTAAACGT
CTCTGCAAACAGTGCCCAGTTTTCTATAGGGAGACAGCAGATCGAGTGAGCTCTACATTCACTCCTTC
CACTGGGGAGCAAGAGGTCTCGGTTGGGTTGTAA
Nucleic acid sequence of proteinic domain derived from ITGB2
   SEQ ID NO: 48: 
AATGCACGGCTGGTAGAGTGCAGTGGCCGTGGCCACTGCCAATGCAACAGGTGCATATGTGACGAAGG
CTACCAGCCACCGATGTGTGAGGATTGTCCCAGCTGTGGCTCGCACTGCAGGGACAACCACACCTCTT
GTGCCGAGTGCCTGAAGTTTGATAAGGGCCCTTTTGAGAAGAACTGTAGTGTTCAGTGTGCTGGTATG
ACGCTGCAGACTATCCCTTTGAAGAAAAAGCCCTGCAAGGAGAGGGACTCGGAAGGCTGTTGGATAAC
TTACACTTTGCAGCAGAAGGACGGAAGGAACATTTACAACATCCATGTGGAGGACAGTCTAGAGTGTG
TGAAGGGCCCCAATGTGGCTGCCATCGTAGGGGGCACCGTGGTAGGTGTCGTACTGATTGGTGTCCTC
CTCCTGGTCATCTGGAAGGCCCTGACCCACCTGACTGACCTCAGGGAGTACAGGCGCTTTGAGAAGGA
GAAACTCAAGTCCCAATGGAACAATGACAACCCCCTCTTCAAGAGTGCTACGACAACGGTCATGAACC
CCAAGTTTGCTGAAAGCTAG
Nucleic acid sequence of proteinic domain derived from CSF2RB
   SEQ ID NO: 49: 
ACTCAGAAGATGGCTTACTCATTCATTGAGCACACATTCCAGGTCCAGTACAAGAAGAAATCGGACAG
CTGGGAGGACAGCAAGACAGAGAACCTAGATCGAGCCCATAGCATGGACCTCTCCCAGCTGGAGCCAG
ACACCTCATACTGCGCCAGGGTGAGGGTCAAGCCCATCTCTAACTACGATGGGATCTGGAGCAAGTGG
AGCGAAGAGTACACTTGGAAGACTGACTGGGTGATGCCCACGCTGTGGATAGTCCTCATCCTGGTCTT
TCTCATCCTCACCTTGCTCCTGATCCTTCGCTTTGGCTGTGTCTCTGTATACAGGACGTACAGGAAGT
GGAAGGAAAAGATCCCCAACCCCAGCAAGAGCCTCCTGTTCCAGGATGGAGGTAAAGGTCTCTGGCCT
CCTGGCAGCATGGCAGCCTTCGCCACTAAGAACCCCGCTCTCCAGGGGCCACAGAGCAGGCTTCTTGC
TGAGCAACAGGGGGAGTCATATGCACATTTGGAAGACAACAACGTGTCACCTCTCACTATAGAGGACC
CTAATATAATTCGAGTTCCACCATCCGGGCCTGATACAACCCCAGCTGCCTCATCCGAATCCACAGAG
CAACTTCCCAATGTTCAAGTAGAGGGACCAACTCCTAACAGACCTAGGAAGCAATTACCCAGCTTTGA
CTTCAATGGGCCCTACCTGGGGCCTCCCCAATCCCACTCTCTGCCTGATCTCCCAGACCAGCTGGGTT
CCCCCCAGGTGGGTGGGAGCCTGAAGCCAGCACTGCCAGGCTCCTTGGAGTACATGTGTCTGCCCCCT
GGAGGTCAAGCGCAACTGGTTCCATTGTCCCAGGTGATGGGGCAGGGCCAGGCTATGGATGTGCAGTG
TGGGTCCAGCCTGGAGACCTCAGGGAGCCCTTCTGTGGAGCCAAAGGAGAACCCTCCAGTTGAGCTGA
GCATGGAGGAACAGGAGGCACGGGACAACCCAGTGACTCTGCCCATAAGCTCTGGGGGCCCTGAGGGC
 
AGTATGATGGCCTCTGATTATGTCACTCCTGGAGATCCGGTGCTCACTCTGCCCACAGGCCCCTGTC
TACCTCTCTGGGCCCCTCTCTAGGGTTGCCCTCAGCCCAAAGCCCCCGTCTCTGTCTTAAGCTGCCCA
GGGTCCCCTCTGGAAGCCCAGCTCTAGGGCCACCAGGGTTTGAGGACTATGTGGAGCTGCCTCCAAGT
GTGAGCCAGGCTGCCAAGTCCCCTCCAGGCCATCCTGCTCCTCCTGTGGCAAGCAGCCCCACAGTGAT
CCCAGGAGAGCCCAGGGAGGAAGTGGGCCCAGCATCCCCACATCCCGAAGGCCTCCTTGTTCTTCAGC
AGGTTGGGGACTACTGCTTCCTCCCTGGCCTGGGACCTGGCTCCCTCTCACCACACAGTAAGCCACCC
TCTCCAAGTCTGTGTTCTGAGACTGAGGACCTAGTCCAGGACTTGTCTGTCAAAAAGTTTCCCTATCA
GCCCATGCCCCAGGCGCCAGCCATTCAGTTTTTCAAGTCCCTAAAGCATCAGGACTACCTGTCCCTGC
CCCCTTGGGACAATAGCCAGTCTGGGAAGGTGTGCTGA
Nucleic acid sequence of proteinic domain derived from CCR1
   SEQ ID NO: 50: 
ACTCCATGCCAAAAGACTGCTGTAAGAGCCTTTGGGGCTGGACTCCTGCCCCCCCTGTATTCTCTAGT
GTTCATCATTGGAGTGGTGGGCAATGTCCTAGTGATTCTGGTGCTCATGCAGCATAGGAGGCTTCAAA
GCATGACCAGCATCTACCTGTTCAACCTGGCTGTCTCTGATCTGGTCTTCCTTTTCACTTTACCTTTC
TGGATTGACTACAAGTTGAAAGACGACTGGATTTTTGGTGATGCCATGTGCAAGCTTCTCTCTGGGTT
TTATTACCTGGGTTTATACAGTGAGATCTTCTTTATCATCCTGTTGACGATTGACAGATACCTGGCCA
TTGTCCATGCTGTGTTTGCCCTGAGGGCCCGAACTGTTACTTTTGGCATCATCACCAGTATTATCACC
TGGGCCCTAGCCATCTTAGCTTCCATGCCTGCCTTATACTTTTTTAAGGCCCAGTGGGAGTTCACTCA
CCGTACCTGTAGCCCTCATTTCCCCTACAAGAGCCTGAAGCAGTGGAAGAGGTTTCAAGCTCTAAAGC
TAAACCTTCTTGGACTAATTTTGCCTCTGTTAGTCATGATAATCTGCTATGCAGGGATCATCAGAATT
CTGCTCAGAAGACCCAGTGAGAAGAAGGTCAAAGCCGTGCGTCTGATATTTGCTATTACTCTTCTATT
CTTCCTCCTCTGGACCCCCTACAATCTGAGTGTATTTGTTTCTGCTTTCCAAGATGTTCTATTCACCA
ATCAGTGTGAGCAGAGTAAGCAACTGGACCTGGCCATGCAGGTGACTGAGGTGATTGCCTACACCCAC
TGTTGTGTCAACCCAATCATTTATGTTTTTGTGGGTGAACGGTTCTGGAAGTACCTTCGGCAGCTGTT
TCAAAGGCATGTGGCTATACCACTGGCAAAATGGCTGCCCTTCCTCTCTGTGGACCAACTAGAAAGGA
CCAGTTCTATATCTCCATCCACAGGAGAACATGAGCTCTCTGCTGGCTTCTGA
Nucleic acid sequence of proteinic domain derived from CCR5
   SEQ ID NO: 51: 
ATGTCAGCACCCTGCCAAAAAATCAATGTGAAACAAATTGCGGCTCAGCTCCTGCCCCCACTCTACTC
CCTGGTATTCATCTTTGGTTTTGTGGGTAACATGATGGTCTTCCTCATCTTGATAAGCTGCAAAAAGC
TGAAGAGCGTGACTGATATCTACCTGCTCAACCTGGCCATCTCTGACCTGCTCTTCCTGCTCACACTA
CCATTCTGGGCTCACTATGCTGCAAATGAGTGGGTCTTTGGGAACATAATGTGTAAAGTATTCACAGG
GCTCTATCACATTGGTTATTTTGGTGGAATCTTCTTCATTATCCTCCTGACAATTGATAGGTACTTGG
CTATTGTCCATGCTGTGTTTGCTTTAAAAGTCAGAACGGTCAACTTTGGGGTGATAACAAGTGTAGTC
ACTTGGGCGGTGGCTGTGTTTGCCTCTCTCCCAGAAATAATCTTTACCAGATCTCAGAAAGAAGGTTT
TCATTATACATGCAGTCCTCATTTTCCACACACTCAGTATCATTTCTGGAAGAGTTTCCAAACATTAA
AGATGGTCATCTTGAGCCTGATCCTGCCTCTACTTGTCATGGTCATCTGCTACTCAGGAATTCTCCAC
ACCCTGTTTCGCTGTAGGAATGAGAAGAAGAGGCACAGGGCTGTGAGGCTCATCTTTGCCATCATGAT
TGTCTACTTTCTCTTCTGGACTCCCTACAACATTGTCCTCCTCCTGACCACCTTCCAGGAATTCTTTG
GACTGAATAACTGCAGTAGTTCTAATAGACTAGACCAGGCCATGCAGGCAACAGAGACTCTTGGAATG
ACACACTGCTGCCTAAACCCTGTCATCTATGCCTTTGTTGGAGAGAAGTTCCGGAGTTATCTCTCAGT
GTTCTTCCGAAAACACATTGTCAAACGCTTTTGCAAACGGTGTTCAATTTTCCAGCAAGACAATCCTG
ATCGTGCAAGCTCAGTCTATACCCGATCCACAGGAGAACATGAAGTTTCTACTGGTTTATGA
Nucleic acid sequence of proteinic domain derived from CXCR4
   SEQ ID NO: 52: 
TTCCGGGATGAAAACGTCCATTTCAATAGGATCTTCCTGCCCACCATCTACTTCATCATCTTCTTGAC
TGGCATAGTCGGCAATGGATTGGTGATCCTGGTCATGGGTTACCAGAAGAAGCTAAGGAGCATGACGG
ACAAGTACCGGCTGCACCTGTCAGTGGCTGACCTCCTCTTTGTCATCACACTCCCCTTCTGGGCAGTT
GATGCCATGGCTGACTGGTACTTTGGGAAATTTTTGTGTAAGGCTGTCCATATCATCTACACTGTCAA
 
CCTCTACAGCAGCGTTCTCATCCTGGCCTTCATCAGCCTGGACCGGTACCTCGCTATTGTCCACGCCA
CCAACAGTCAGAGGCCAAGGAAACTGCTGGCTGAAAAGGCAGTCTATGTGGGCGTCTGGATCCCAGCC
CTCCTCCTGACTATACCTGACTTCATCTTTGCCGACGTCAGCCAGGGGGACATCAGTCAGGGGGATGA
CAGGTACATCTGTGACCGCCTTTACCCCGATAGCCTGTGGATGGTGGTGTTTCAATTCCAGCATATAA
TGGTGGGTCTCGTCCTGCCCGGCATCGTCATCCTCTCCTGTTACTGCATCATCATCTCTAAGCTGTCA
CACTCCAAGGGCCACCAGAAGCGCAAGGCCCTCAAGACGACAGTCATCCTCATCCTAGCTTTCTTTGC
CTGCTGGCTGCCATATTATGTGGGGATCAGCATCGACTCCTTCATCCTTTTGGGGGTCATCAAGCAAG
GATGTGACTTCGAGAGCATCGTGCACAAGTGGATCTCCATCACAGAGGCCCTCGCCTTCTTCCACTGT
TGCCTGAACCCCATCCTCTATGCCTTCCTCGGGGCCAAGTTCAAAAGCTCTGCCCAGCATGCACTCAA
CTCCATGAGCAGAGGCTCCAGCCTCAAGATCCTTTCCAAAGGAAAGCGGGGTGGACACTCTTCCGTCT
CCACGGAGTCAGAATCCTCCAGTTTTCACTCCAGCTAA
Nucleic acid sequence of proteinic domain derived from SELPLG
   SEQ ID NO: 53: 
ATTGCCACCACTGACCCTACTGCCCCAGGTACAGGAGGACAGCTGTTGGATGCTGAGCACAGACTC
TGCCACACAGTGGAGTCTAACCTCAGTAGAGACCGTCCAACCAGCATCCACAGAGGTAGAGACCTCGC
AGCCAGCACCCATGGAGGCAGAGACCTCGCAGCCAGCACCCATGGAGGCAGAGACCTCGCAGCCAGCA
CCCATGGAGGCAGACACCTCAAAGCCAGCACCCACGGAGGCAGAGACCTCAAAGCCAGCACCCACGGA
GGCAGAGACCTCTCAGCCAGCACCCAACGAGGCAGAGACCTCAAAACCAGCACCCACGGAGGCAGAGA
CCTCAAAACCAGCACCCACGGAGGCAGAGACCACCCAGCTTCCCAGGATTCAGGCTGTAAAAACTCTG
TTTACAACGTCTGCAGCCACCGAAGTCCCTTCCACAGAACCTACCACCATGGAGACGGCGTCCACAGA
GTCTAACGAGTCTACCATCTTCCTTGGGCCATCCGTGACTCACTTACCTGACAGCGGCCTGAAGAAAG
GGCTGATTGTGACCCCTGGGAATTCACCTGCCCCAACCCTGCCAGGGAGTTCAGATCTCATCCCGGTG
AAGCAATGTCTGCTGATTATCCTCATCTTGGCTTCTCTGGCCACCATCTTCCTCGTGTGCACAGTGGT
GCTGGCGGTCCGTCTGTCCCGTAAGACCCACATGTACCCAGTGCGGAACTACTCCCCCACGGAGATGA
TCTGCATCTCGTCCCTGCTACCTGAGGGGGGAGACGGGGCCCCTGTCACAGCCAATGGGGGCCTGCCC
AAGGTCCAGGACCTGAAGACAGAGCCCAGTGGGGACCGGGATGGGGACGACCTCACCCTGCACAGCTT
CCTCCCTTAG
Amino acid sequence of EVIR-N
   SEQ ID NO: 54: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGLLGVSLGGAKEACPTGLYTHSGECCKACNLGEGVAQPCGANQTVCEPCLDSVTFSDVVSATE
PCKPCTECVGLQSMSAPCVEADDAVCRCAYGYYQDETTGRCEACRVCEAGSGLVFSCQDKQNTVCEEC
PDGTYSDEANHVDPCLPCTVCEDTERQLRECTRWADAECEEIPGRWITRSTPPEGSDSTAPSTQEPEA
PPEQDLIASTVAGVVTTVMGSSQPVVTRGTTDNLIPVYCSILAAVVVGLVAYIAFKRWNRGIL
Amino acid sequence of EVIR-G
   SEQ ID NO: 55: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGHENSELLIPKATHNDSGSYFCRGLIGHNNKSSASFRISLGDPGSPSMFPPWHQITFCLLIGL
LFAIDTVLYFSVRRGLQSPVADYEEPKIQWSKEPQDKTRVD
Amino acid sequence of EVIR-F
   SEQ ID NO: 56: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
 
GHISSSYATSTYNQKFKNKAAFTVDTSSSTAFMQLNSLTSEDSAVYYCVRSGNYEEYAMDYWGQGTSV
TVSSTGPGPFPFIQDNISFYATIGLCLPFIVVLIVLICHKYKKQFRYESQLQMIQVTGPLDNEYFYVD
FRDYEYDLKWEFPRENLEFGKVLGSGAFGRVMNATAYGISKTGVSIQVAVKMLKEKADSCEKEALMSE
LKMMTHLGHHDNIVNLLGACTLSGPVYLIFEYCCYGDLLNYLRSKREKFHRTWTEIFKEHNFSFYPTF
QAHSNSSMPGSREVQLHPPLDQLSGFNGNLIHSEDEIEYENQKRLAEEEEEDLNVLTFEDLLCFAYQV
AKGMEFLEFKSCVHRDLAARNVLVTHGKVVKICDFGLARDILSDSSYVVRGNARLPVKWMAPESLFEG
IYTIKSDVWSYGILLWEIFSLGVNPYPGIPVDANFYKLIQSGFKMEQPFYATEGIYFVMQSCWAFDSR
KRPSFPNLTSFLGCQLAEAEEAMYQNMGGNVPEHPSIYQNRRPLSREAGSEPPSPQAQVKIHGERS
Amino acid sequence of EVIR-T
   SEQ ID NO: 57: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGQLYSLSTLDCSFNRIETSKGILQHFPKSLAFFNLTNNSVACICEHQKFLQWVKEQKQFLVNV
EQMTCATPVEMNTSLVLDFNNSTCYMYKTIISVSVVSVIVVSTVAFLIYHFYFHLILIAGCKKYSRGE
SIYDAFVIYSSQNEDWVRNELVKNLEEGVPRFHLCLHYRDFIPGVAIAANIIQEGFHKSRKVIVVVSR
HFIQSRWCIFEYEIAQTWQFLSSRSGIIFIVLEKVEKSLLRQQVELYRLLSRNTYLEWEDNPLGRHIF
WRRLKNALLDGKASNPEQTAEEEQETATWT
Amino acid sequence of EVIR-C2
   SEQ ID NO: 58: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGMEDNNMLPQFIHGILSTSHSLFTRSIQELDEGATTPYDYDDGEPCHKTSVKQIGAWILPPLY
SLVFIFGFVGNMLVIIILIGCKKLKSMTDIYLLNLAISDLLFLLTLPFWAHYAANEWVFGNIMCKVFT
GLYHIGYFGGIFFIILLTIDRYLAIVHAVFALKARTVTFGVITSVVTWVVAVFASLPGIIFTKSKQDD
HHYTCGPYFTQLWKNFQTIMRNILSLILPLLVMVICYSGILHTLFRCRNEKKRHRAVRLIFAIMIVYF
LFWTPYNIVLFLTTFQESLGMSNCVIDKHLDQAMQVTETLGMTHCCINPVIYAFVGEKFRRYLSIFFR
KHIAKRLCKQCPVFYRETADRVSSTFTPSTGEQEVSVGL
Amino acid sequence of EVIR-I
   SEQ ID NO: 59: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGNARLVECSGRGHCQCNRCICDEGYQPPMCEDCPSCGSHCRDNHTSCAECLKFDKGPFEKNCS
VQCAGMTLQTIPLKKKPCKERDSEGCWITYTLQQKDGRNIYNIHVEDSLECVKGPNVAAIVGGTVVGV
VLIGVLLLVIWKALTHLTDLREYRRFEKEKLKSQWNNDNPLFKSATTTVMNPKFAES
Amino acid sequence of EVIR-C
   SEQ ID NO: 60: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGTQKMAYSFIEHTFQVQYKKKSDSWEDSKTENLDRAHSMDLSQLEPDTSYCARVRVKPISNYD
GIWSKWSEEYTWKTDWVMPTLWIVLILVFLILTLLLILRFGCVSVYRTYRKWKEKIPNPSKSLLFQDG
 
GKGLWPPGSMAAFATKNPALQGPQSRLLAEQQGESYAHLEDNNVSPLTIEDPNIIRVPPSGPDTTPAA
SSESTEQLPNVQVEGPTPNRPRKQLPSFDFNGPYLGPPQSHSLPDLPDQLGSPQVGGSLKPALPGSLE
YMCLPPGGQAQLVPLSQVMGQGQAMDVQCGSSLETSGSPSVEPKENPPVELSMEEQEARDNPVTLPIS
SGGPEGSMMASDYVTPGDPVLTLPTGPLSTSLGPSLGLPSAQSPRLCLKLPRVPSGSPALGPPGFEDY
VELPPSVSQAAKSPPGHPAPPVASSPTVIPGEPREEVGPASPHPEGLLVLQQVGDYCFLPGLGPGSLS
PHSKPPSPSLCSETEDLVQDLSVKKFPYQPMPQAPAIQFFKSLKHQDYLSLPPWDNSQSGKVC
Amino acid sequence of EVIR-C1
   SEQ ID NO: 61: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGTPCQKTAVRAFGAGLLPPLYSLVFIIGVVGNVLVILVLMQHRRLQSMTSIYLFNLAVSDLVF
LFTLPFWIDYKLKDDWIFGDAMCKLLSGFYYLGLYSEIFFIILLTIDRYLAIVHAVFALRARTVTFGI
ITSIITWALAILASMPALYFFKAQWEFTHRTCSPHFPYKSLKQWKRFQALKLNLLGLILPLLVMIICY
AGIIRILLRRPSEKKVKAVRLIFAITLLFFLLWTPYNLSVFVSAFQDVLFTNQCEQSKQLDLAMQVTE
VIAYTHCCVNPIIYVFVGERFWKYLRQLFQRHVAIPLAKWLPFLSVDQLERTSSISPSTGEHELSAGF
Amino acid sequence of EVIR-C5
   SEQ ID NO: 62: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGMSAPCQKINVKQIAAQLLPPLYSLVFIFGFVGNMMVFLILISCKKLKSVTDIYLLNLAISDL
LFLLTLPFWAHYAANEWVFGNIMCKVFTGLYHIGYFGGIFFIILLTIDRYLAIVHAVFALKVRTVNFG
VITSVVTWAVAVFASLPEIIFTRSQKEGFHYTCSPHFPHTQYHFWKSFQTLKMVILSLILPLLVMVIC
YSGILHTLFRCRNEKKRHRAVRLIFAIMIVYFLFWTPYNIVLLLTTFQEFFGLNNCSSSNRLDQAMQA
TETLGMTHCCLNPVIYAFVGEKFRSYLSVFFRKHIVKRFCKRCSIFQQDNPDRASSVYTRSTGEHEVS
TGL
Amino acid sequence of EVIR-CX
   SEQ ID NO: 63: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGFRDENVHFNRIFLPTIYFIIFLTGIVGNGLVILVMGYQKKLRSMTDKYRLHLSVADLLFVIT
LPFWAVDAMADWYFGKFLCKAVHIIYTVNLYSSVLILAFISLDRYLAIVHATNSQRPRKLLAEKAVYV
GVWIPALLLTIPDFIFADVSQGDISQGDDRYICDRLYPDSLWMVVFQFQHIMVGLVLPGIVILSCYCI
IISKLSHSKGHQKRKALKTTVILILAFFACWLPYYVGISIDSFILLGVIKQGCDFESIVHKWISITEA
LAFFHCCLNPILYAFLGAKFKSSAQHALNSMSRGSSLKILSKGKRGGHSSVSTESESSSFHSS
Amino acid sequence of EVIR-S
   SEQ ID NO: 64: 
MDFQVQIFSFLLISASVIMSRGDIVLTQTPSSLPVSVGEKVTMTCKSSQTLLYSNNQKNYLAWYQQKP
GQSPKLLISWAFTRKSGVPDRFTGSGSGTDFTLTIGSVKAEDLAVYYCQQYSNYPWTFGGGTRLEIKR
GGGGSGGGGSGGGGSGGGGSEVQLQQSGPEVVKTGASVKISCKASGYSFTGYFINWVKKNSGKSPEWI
GHISSSYATSTYNQKFKNKAAFTVDTSSSTAFMQLNSLTSEDSAVYYCVRSGNYEEYAMDYWGQGTSV
TVSSTGIATTDPTAPGTGGTAVGMLSTDSATQWSLTSVETVQPASTEVETSQPAPMEAETSQPAPMEA
ETSQPAPMEADTSKPAPTEAETSKPAPTEAETSQPAPNEAETSKPAPTEAETSKPAPTEAETTQLPRI
 
QAVKTLFTTSAATEVPSTEPTTMETASTESNESTIFLGPSVTHLPDSGLKKGLIVTPGNSPAPTLPGS
SDLIPVKQCLLIILILASLATIFLVCTVVLAVRLSRKTHMYPVRNYSPTEMICISSLLPEGGDGAPVT
ANGGLPKVQDLKTEPSGDRDGDDLTLHSFLP
Nucleic acid sequence of EVIR-N
   SEQ ID NO: 65: 
ATGGATTTTCAGGTCCAGATTTTCTCCTTCCTCCTCATTTCAGCCAGCGTCATTATGTCTCGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTCTTCTGGGGGTGTCCCTTGGAGGTGCCAAGGAGGCATGCCCCACAGGCCT
GTACACACACAGCGGTGAGTGCTGCAAAGCCTGCAACCTGGGCGAGGGTGTGGCCCAGCCTTGTGGAG
CCAACCAGACCGTGTGTGAGCCCTGCCTGGACAGCGTGACGTTCTCCGACGTGGTGAGCGCGACCGAG
CCGTGCAAGCCGTGCACCGAGTGCGTGGGGCTCCAGAGCATGTCGGCGCCGTGCGTGGAGGCCGACGA
CGCCGTGTGCCGCTGCGCCTACGGCTACTACCAGGATGAGACGACTGGGCGCTGCGAGGCGTGCCGCG
TGTGCGAGGCGGGCTCGGGCCTCGTGTTCTCCTGCCAGGACAAGCAGAACACCGTGTGCGAGGAGTGC
CCCGACGGCACGTATTCCGACGAGGCCAACCACGTGGACCCGTGCCTGCCCTGCACCGTGTGCGAGGA
CACCGAGCGCCAGCTCCGCGAGTGCACACGCTGGGCCGACGCCGAGTGCGAGGAGATCCCTGGCCGTT
GGATTACACGGTCCACACCCCCAGAGGGCTCGGACAGCACAGCCCCCAGCACCCAGGAGCCTGAGGCA
CCTCCAGAACAAGACCTCATAGCCAGCACGGTGGCAGGTGTGGTGACCACAGTGATGGGCAGCTCCCA
GCCCGTGGTGACCCGAGGCACCACCGACAACCTCATCCCTGTCTATTGCTCCATCCTGGCTGCTGTGG
TTGTGGGCCTTGTGGCCTACATAGCCTTCAAGAGGTGGAACAGGGGGATCCTCTAG
Nucleic acid sequence of EVIR-G
   SEQ ID NO: 66: 
ATGGATTTTCAGGTCCAGATTTTCTCCTTCCTCCTCATTTCAGCCAGCGTCATTATGTCTCGGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTCACGAGAACTCCGAACTGCTGATTCCTAAGGCAACTCACAACGACTCCGG
CTCCTATTTCTGTAGAGGGCTGATTGGACATAACAACAAGAGCTCCGCCTCATTCAGGATTAGCCTGG
GCGACCCAGGGTCTCCCAGTATGTTCCCCCCTTGGCACCAGATCACCTTTTGCCTGCTGATTGGACTG
CTGTTCGCTATCGATACAGTGCTGTACTTTTCTGTCCGGAGAGGCCTGCAGTCACCCGTGGCAGATTA
CGAAGAACCCAAGATTCAGTGGAGCAAGGAGCCCCAGGATAAGACGCGTGTCGACTGA
Nucleic acid sequence of EVIR-F
   SEQ ID NO: 67: 
ATGGATTTTCAGGTCCAGATTTTCTCCTTCCTCCTCATTTCAGCCAGCGTCATTATGTCTCGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGCAGGGGACCTCCGTG
ACTGTCTCAAGCACCGGTCCAGGCCCCTTCCCTTTCATCCAAGACAACATCTCCTTCTATGCGACCAT
TGGGCTCTGTCTCCCCTTCATTGTTGTTCTCATTGTGTTGATCTGCCACAAATACAAAAAGCAATTTA
GGTACGAGAGTCAGCTGCAGATGATCCAGGTGACTGGCCCCCTGGATAACGAGTACTTCTACGTTGAC
TTCAGGGACTATGAATATGACCTTAAGTGGGAGTTCCCGAGAGAGAACTTAGAGTTTGGGAAGGTCCT
GGGGTCTGGCGCTTTCGGGAGGGTGATGAACGCCACGGCCTATGGCATTAGTAAAACGGGAGTCTCAA
TTCAGGTGGCGGTGAAGATGCTAAAAGAGAAAGCTGACAGCTGTGAAAAAGAAGCTCTCATGTCGGAG
CTCAAAATGATGACCCACCTGGGACACCATGACAACATCGTGAATCTGCTGGGGGCATGCACACTGTC
AGGGCCAGTGTACTTGATTTTTGAATATTGTTGCTATGGTGACCTCCTCAACTACCTAAGAAGTAAAA
GAGAGAAGTTTCACAGGACATGGACAGAGATTTTTAAGGAACATAATTTCAGTTTTTACCCTACTTTC
CAGGCACATTCAAATTCCAGCATGCCTGGTTCACGAGAAGTTCAGTTACACCCGCCCTTGGATCAGCT
CTCAGGGTTCAATGGGAATTTAATTCATTCTGAAGATGAGATTGAATATGAAAACCAGAAGAGGCTGG
CAGAAGAAGAGGAGGAAGATTTGAACGTGCTGACGTTTGAAGACCTCCTTTGCTTTGCGTACCAAGTG
GCCAAAGGCATGGAATTCCTGGAGTTCAAGTCGTGTGTCCACAGAGACCTGGCAGCCAGGAATGTGTT
GGTCACCCACGGGAAGGTGGTGAAGATCTGTGACTTTGGACTGGCCCGAGACATCCTGAGCGACTCCA
GCTACGTCGTCAGGGGCAACGCACGGCTGCCGGTGAAGTGGATGGCACCTGAGAGCTTATTTGAAGGG
ATCTACACAATCAAGAGTGACGTCTGGTCCTACGGCATCCTTCTCTGGGAGATATTTTCACTGGGTGT
GAACCCTTACCCTGGCATTCCTGTCGACGCTAACTTCTATAAACTGATTCAGAGTGGATTTAAAATGG
AGCAGCCATTCTATGCCACAGAAGGGATATACTTTGTAATGCAATCCTGCTGGGCTTTTGACTCAAGG
AAGCGGCCATCCTTCCCCAACCTGACTTCATTTTTAGGATGTCAGCTGGCAGAGGCAGAAGAAGCGAT
GTATCAGAACATGGGTGGCAACGTCCCAGAACATCCATCCATCTACCAAAACAGGCGGCCCCTCAGCA
GAGAGGCAGGCTCAGAGCCGCCATCGCCACAGGCCCAGGTGAAGATTCACGGAGAAAGAAGTTAG
Nucleic acid sequence of EVIR-T
   SEQ ID NO: 68: 
ATGGATTTTCAGGTCCAGATTTTCTCCTTCCTCCTCATTTCAGCCAGCGTCATTATGTCTCGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAACTGGAGCTTCAGTCAAAATCAGCTGTAAGGCATCTG
GGTACAGCTTCACCGGCTACTTCATCAACTGGGTGAAGAAAAATTCAGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTCAGCTGTATTCCCTCAGCACTCTTGATTGCAGTTTCAATCGCATAGAGAC
 
ATCTAAAGGAATACTGCAACATTTTCCAAAGAGTCTAGCCTTCTTCAATCTTACTAACAATTCTGTTG
CTTGTATATGTGAACATCAGAAATTCCTGCAGTGGGTCAAGGAACAGAAGCAGTTCTTGGTGAATGTT
GAACAAATGACATGTGCAACACCTGTAGAGATGAATACCTCCTTAGTGTTGGATTTTAATAATTCTAC
CTGTTATATGTACAAGACAATCATCAGTGTGTCAGTGGTCAGTGTGATTGTGGTATCCACTGTAGCAT
TTCTGATATACCACTTCTATTTTCACCTGATACTTATTGCTGGCTGTAAAAGTACAGCAGAGGAGAA
AGCATCTATGATGCATTTGTGATCTACTCGAGTCAGAATGAGGACTGGGTGAGAAATGAGCTGGTAAA
GAATTTAGAAGAAGGAGTGCCCCGCTTTCACCTCTGCCTTCACTACAGAGACTTTATTCCTGGTGTAG
CCATTGCTGCCAACATCATCCAGGAAGGCTTCCACAAGAGCCGGAAGGTTATTGTGGTAGTGTCTAGA
CACTTTATTCAGAGCCGTTGGTGTATCTTTGAATATGAGATTGCTCAAACATGGCAGTTTCTGAGCAG
CCGCTCTGGCATCATCTTCATTGTCCTTGAGAAGGTTGAGAAGTCCCTGCTGAGGCAGCAGGTGGAAT
TGTATCGCCTTCTTAGCAGAAACACCTACCTGGAATGGGAGGACAATCCTCTGGGGAGGCACATCTTC
TGGAGAAGACTTAAAAATGCCCTATTGGATGGAAAAGCCTCGAATCCTGAGCAAACAGCAGAGGAAGA
ACAAGAAACGGCAACTTGGACCTGA
Nucleic acid sequence of EVIR-C2
   SEQ ID NO: 69: 
ATGGATTTTCAGGTCCAGATTTTCTCCTTCCTCCTCATTTCAGCCAGCGTCATTATGTCTCGGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGCAGGGGACCTCCGTG
ACTGTCTCAAGCACCGGTATGGAAGACAATAATATGTTACCTCAGTTCATCCATGGCATACTATCAAC
ATCTCATTCTCTATTTACACGAAGTATCCAAGAGCTTGATGAAGGGGCCACCACACCGTATGACTACG
ATGATGGTGAGCCTTGTCATAAAACCAGTGTGAAGCAAATTGGAGCTTGGATCCTGCCTCCACTCTAC
TCCCTGGTATTCATCTTTGGTTTTGTGGGCAACATGTTGGTCATTATAATTCTGATAGGCTGTAAAAA
GCTGAAGAGCATGACTGATATCTATCTGCTCAACCTGGCCATCTCTGACCTGCTCTTCCTGCTCACAT
TACCATTCTGGGCTCACTATGCTGCAAATGAGTGGGTCTTTGGAATATAATGTGTAAAGTATTCACA
GGGCTCTATCACATTGGTTATTTTGGTGGAATCTTTTTCATTATCCTCCTGACAATTGATAGGTACTT
GGCTATTGTTCATGCTGTGTTTGCTTTAAAAGCCAGGACAGTTACCTTTGGGGTGATAACAAGTGTAG
TCACTTGGGTGGTGGCTGTGTTTGCCTCTCTACCAGGAATCATATTTACTAAATCCAAACAAGATGAT
CACCATTACACCTGTGGCCCTTATTTTACACAACTATGGAAGAATTTCCAAACAATAATGAGAAATAT
CTTGAGCCTGATCCTGCCTCTACTTGTCATGGTCATCTGCTACTCAGGAATTCTCCACACCCTGTTTC
GCTGTAGGAATGAGAAGAAGAGGCACAGGGCTGTGAGGCTCATCTTTGCCATCATGATTGTCTACTTT
CTCTTCTGGACTCCATACAATATTGTTCTCTTCTTGACCACCTTCCAGGAATCCTTGGGAATGAGTAA
CTGTGTGATTGACAAGCACTTAGACCAGGCCATGCAGGTGACAGAGACTCTTGGAATGACACACTGCT
GCATTAATCCTGTCATTTATGCCTTTGTTGGAGAGAAGTTCCGAAGGTATCTCTCCATATTTTTCAGA
AAGCACATTGCTAAACGTCTCTGCAAACAGTGCCCAGTTTTCTATAGGGAGACAGCAGATCGAGTGAG
CTCTACATTCACTCCTTCCACTGGGGAGCAAGAGGTCTCGGTTGGGTTGTAA
Nucleic acid sequence of EVIR-I
   SEQ ID NO: 70: 
ATGGATTTTCAGGTCCAGATTTTCTCCTTCCTCCTCATTTCAGCCAGCGTCATTATGTCTCGGGGGGA
TATTGTCCTCACACAGACTCCCAGCTCCCTGCCTGTGTCCGTCGGAGAGAAAGTGACCATGACATGCA
 
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTAATGCACGGCTGGTAGAGTGCAGTGGCCGTGGCCACTGCCAATGCAACAG
GTGCATATGTGACGAAGGCTACCAGCCACCGATGTGTGAGGATTGTCCCAGCTGTGGCTCGCACTGCA
GGGACAACCACACCTCTTGTGCCGAGTGCCTGAAGTTTGATAAGGGCCCTTTTGAGAAGAACTGTAGT
GTTCAGTGTGCTGGTATGACGCTGCAGACTATCCCTTTGAAGAAAAAGCCCTGCAAGGAGAGGGACTC
GGAAGGCTGTTGGATAACTTACACTTTGCAGCAGAAGGACGGAAGGAACATTTACAACATCCATGTGG
AGGACAGTCTAGAGTGTGTGAAGGGCCCCAATGTGGCTGCCATCGTAGGGGGCACCGTGGTAGGTGTC
GTACTGATTGGTGTCCTCCTCCTGGTCATCTGGAAGGCCCTGACCCACCTGACTGACCTCAGGGAGTA
CAGGCGCTTTGAGAAGGAGAAACTCAAGTCCCAATGGAACAATGACAACCCCCTCTTCAAGAGTGCTA
CGACAACGGTCATGAACCCCAAGTTTGCTGAAAGCTAG
Nucleic acid sequence of EVIR-C
   SEQ ID NO: 71: 
ATGGATTTTCAGGTCCAGATTTTCTCCTTCCTCCTCATTTCAGCCAGCGTCATTATGTCTCGGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTACTCAGAAGATGGCTTACTCATTCATTGAGCACACATTCCAGGTCCAGTA
CAAGAAGAAATCGGACAGCTGGGAGGACAGCAAGACAGAGAACCTAGATCGAGCCCATAGCATGGACC
TCTCCCAGCTGGAGCCAGACACCTCATACTGCGCCAGGGTGAGGGTCAAGCCCATCTCTAACTACGAT
GGGATCTGGAGCAAGTGGAGCGAAGAGTACACTTGGAAGACTGACTGGGTGATGCCCACGCTGTGGAT
AGTCCTCATCCTGGTCTTTCTCATCCTCACCTTGCTCCTGATCCTTCGCTTTGGCTGTGTCTCTGTAT
ACAGGACGTACAGGAAGTGGAAGGAAAAGATCCCCAACCCCAGCAAGAGCCTCCTGTTCCAGGATGGA
GGTAAAGGTCTCTGGCCTCCTGGCAGCATGGCAGCCTTCGCCACTAAGAACCCCGCTCTCCAGGGGCC
ACAGAGCAGGCTTCTTGCTGAGCAACAGGGGGAGTCATATGCACATTTGGAAGACAACAACGTGTCAC
CTCTCACTATAGAGGACCCTAATATAATTCGAGTTCCACCATCCGGGCCTGATACAACCCCAGCTGCC
TCATCCGAATCCACAGAGCAACTTCCCAATGTTCAAGTAGAGGGACCAACTCCTAACAGACCTAGGAA
GCAATTACCCAGCTTTGACTTCAATGGGCCCTACCTGGGGCCTCCCCAATCCCACTCTCTGCCTGATC
TCCCAGACCAGCTGGGTTCCCCCCAGGTGGGTGGGAGCCTGAAGCCAGCACTGCCAGGCTCCTTGGAG
TACATGTGTCTGCCCCCTGGAGGTCAAGCGCAACTGGTTCCATTGTCCCAGGTGATGGGGCAGGGCCA
GGCTATGGATGTGCAGTGTGGGTCCAGCCTGGAGACCTCAGGGAGCCCTTCTGTGGAGCCAAAGGAGA
ACCCTCCAGTTGAGCTGAGCATGGAGGAACAGGAGGCACGGGACAACCCAGTGACTCTGCCCATAAGC
 
TCTGGGGGCCCTGAGGGCAGTATGATGGCCTCTGATTATGTCACTCCTGGAGATCCGGTGCTCACTCT
GCCCACAGGGCCCCTGTCTACCTCTCTGGGCCCCTCTCTAGGGTTGCCCTCAGCCCAAAGCCCCCGTC
TCTGTCTTAAGCTGCCCAGGGTCCCCTCTGGAAGCCCAGCTCTAGGGCCACCAGGGTTTGAGGACTAT
GTGGAGCTGCCTCCAAGTGTGAGCCAGGCTGCCAAGTCCCCTCCAGGCCATCCTGCTCCTCCTGTGGC
AAGCAGCCCCACAGTGATCCCAGGAGAGCCCAGGGAGGAAGTGGGCCCAGCATCCCCACATCCCGAAG
GCCTCCTTGTTCTTCAGCAGGTTGGGGACTACTGCTTCCTCCCTGGCCTGGGACCTGGCTCCCTCTCA
CCACACAGTAAGCCACCCTCTCCAAGTCTGTGTTCTGAGACTGAGGACCTAGTCCAGGACTTGTCTGT
CAAAAAGTTTCCCTATCAGCCCATGCCCCAGGCGCCAGCCATTCAGTTTTTCAAGTCCCTAAAGCATC
AGGACTACCTGTCCCTGCCCCCTTGGGACAATAGCCAGTCTGGGAAGGTGTGCTGA
Nucleic acid sequence of EVIR-C1
   SEQ ID NO: 72: 
ATGGATTTTCAGGTCCAGATTTTCTCCTTCCTCCTCATTTCAGCCAGCGTCATTATGTCTCGGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTACTCCATGCCAAAAGACTGCTGTAAGAGCCTTTGGGGCTGGACTCCTGCC
CCCCCTGTATTCTCTAGTGTTCATCATTGGAGTGGTGGGCAATGTCCTAGTGATTCTGGTGCTCATGC
AGCATAGGAGGCTTCAAAGCATGACCAGCATCTACCTGTTCAACCTGGCTGTCTCTGATCTGGTCTTC
CTTTTCACTTTACCTTTCTGGATTGACTACAAGTTGAAAGACGACTGGATTTTTGGTGATGCCATGTG
CAAGCTTCTCTCTGGGTTTTATTACCTGGGTTTATACAGTGAGATCTTCTTTATCATCCTGTTGACGA
TTGACAGATACCTGGCCATTGTCCATGCTGTGTTTGCCCTGAGGGCCCGAACTGTTACTTTTGGCATC
ATCACCAGTATTATCACCTGGGCCCTAGCCATCTTAGCTTCCATGCCTGCCTTATACTTTTTTAAGGC
CCAGTGGGAGTTCACTCACCGTACCTGTAGCCCTCATTTCCCCTACAAGAGCCTGAAGCAGTGGAAGA
GGTTTCAAGCTCTAAAGCTAAACCTTCTTGGACTAATTTTGCCTCTGTTAGTCATGATAATCTGCTAT
GCAGGGATCATCAGAATTCTGCTCAGAAGACCCAGTGAGAAGAAGGTCAAAGCCGTGCGTCTGATATT
TGCTATTACTCTTCTATTCTTCCTCCTCTGGACCCCCTACAATCTGAGTGTATTTGTTTCTGCTTTCC
AAGATGTTCTATTCACCAATCAGTGTGAGCAGAGTAAGCAACTGGACCTGGCCATGCAGGTGACTGAG
GTGATTGCCTACACCCACTGTTGTGTCAACCCAATCATTTATGTTTTTGTGGGTGAACGGTTCTGGAA
GTACCTTCGGCAGCTGTTTCAAAGGCATGTGGCTATACCACTGGCAAAATGGCTGCCCTTCCTCTCTG
TGGACCAACTAGAAAGGACCAGTTCTATATCTCCATCCACAGGAGAACATGAGCTCTCTGCTGGCTTC
TGA
Nucleic acid sequence of EVIR-C5
   SEQ ID NO: 73: 
ATGGATTTTCAGGTCCAGATTTTCTCCTTCCTCCTCATTTCAGCCAGCGTCATTATGTCTCGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
 
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGTATGTCAGCACCCTGCCAAAAAATCAATGTGAAACAAATTGCGGCTCAGCT
CCTGCCCCCACTCTACTCCCTGGTATTCATCTTTGGTTTTGTGGGTAACATGATGGTCTTCCTCATCT
TGATAAGCTGCAAAAAGCTGAAGAGCGTGACTGATATCTACCTGCTCAACCTGGCCATCTCTGACCTG
CTCTTCCTGCTCACACTACCATTCTGGGCTCACTATGCTGCAAATGAGTGGGTCTTTGGGAACATAAT
GTGTAAAGTATTCACAGGGCTCTATCACATTGGTTATTTTGGTGGAATCTTCTTCATTATCCTCCTGA
CAATTGATAGGTACTTGGCTATTGTCCATGCTGTGTTTGCTTTAAAAGTCAGAACGGTCAACTTTGGG
GTGATAACAAGTGTAGTCACTTGGGCGGTGGCTGTGTTTGCCTCTCTCCCAGAAATAATCTTTACCAG
ATCTCAGAAAGAAGGTTTTCATTATACATGCAGTCCTCATTTTCCACACACTCAGTATCATTTCTGGA
AGAGTTTCCAAACATTAAAGATGGTCATCTTGAGCCTGATCCTGCCTCTACTTGTCATGGTCATCTGC
TACTCAGGAATTCTCCACACCCTGTTTCGCTGTAGGAATGAGAAGAAGAGGCACAGGGCTGTGAGGCT
CATCTTTGCCATCATGATTGTCTACTTTCTCTTCTGGACTCCCTACAACATTGTCCTCCTCCTGACCA
CCTTCCAGGAATTCTTTGGACTGAATAACTGCAGTAGTTCTAATAGACTAGACCAGGCCATGCAGGCA
ACAGAGACTCTTGGAATGACACACTGCTGCCTAAACCCTGTCATCTATGCCTTTGTTGGAGAGAAGTT
CCGGAGTTATCTCTCAGTGTTCTTCCGAAAACACATTGTCAAACGCTTTTGCAAACGGTGTTCAATTT
TCCAGCAAGACAATCCTGATCGTGCAAGCTCAGTCTATACCCGATCCACAGGAGAACATGAAGTTTCT
ACTGGTTTATGA
Nucleic acid sequence of EVIR-CX
   SEQ ID NO: 74: 
ATGGATTTTCAGGTCCAGATTTTCTCCTTCCTCCTCATTTCAGCCAGCGTCATTATGTCTCGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGGCAGGGGACCTCCGTG
ACTGTCTCAAGCACCGGGTTCCGGGATGAAAACGTCCATTTCAATAGGATCTTCCTGCCCACCATCTA
CTTCATCATCTTCTTGACTGGCATAGTCGGCAATGGATTGGTGATCCTGGTCATGGGTTACCAGAAGA
AGCTAAGGAGCATGACGGACAAGTACCGGCTGCACCTGTCAGTGGCTGACCTCCTCTTTGTCATCACA
CTCCCCTTCTGGGCAGTTGATGCCATGGCTGACTGGTACTTTGGGAAATTTTTGTGTAAGGCTGTCCA
TATCATCTACACTGTCAACCTCTACAGCAGCGTTCTCATCCTGGCCTTCATCAGCCTGGACCGGTACC
TCGCTATTGTCCACGCCACCAACAGTCAGAGGCCAAGGAAACTGCTGGCTGAAAAGGCAGTCTATGTG
GGCGTCTGGATCCCAGCCCTCCTCCTGACTATACCTGACTTCATCTTTGCCGACGTCAGCCAGGGGGA
CATCAGTCAGGGGGATGACAGGTACATCTGTGACCGCCTTTACCCCGATAGCCTGTGGATGGTGGTGT
TTCAATTCCAGCATATAATGGTGGGTCTCGTCCTGCCCGGCATCGTCATCCTCTCCTGTTACTGCATC
ATCATCTCTAAGCTGTCACACTCCAAGGGCCACCAGAAGCGCAAGGCCCTCAAGACGACAGTCATCCT
CATCCTAGCTTTCTTTGCCTGCTGGCTGCCATATTATGTGGGGATCAGCATCGACTCCTTCATCCTTT
TGGGGGTCATCAAGCAAGGATGTGACTTCGAGAGCATCGTGCACAAGTGGATCTCCATCACAGAGGCC
CTCGCCTTCTTCCACTGTTGCCTGAACCCCATCCTCTATGCCTTCCTCGGGGCCAAGTTCAAAAGCTC
TGCCCAGCATGCACTCAACTCCATGAGCAGAGGCTCCAGCCTCAAGATCCTTTCCAAAGGAAAGCGGG
GTGGACACTCTTCCGTCTCCACGGAGTCAGAATCCTCCAGTTTTCACTCCAGCTAA
Nucleic acid sequence of EVIR-S
   SEQ ID NO: 75: 
ATGGATTTTCAGGTCCAGATTTTCTCCTTCCTCCTCATTTCAGCCAGCGTCATTATGTCTCGGGGGGA
TATTGTCCTCACACAGACTCCCAGCTCCCTGCCTGTGTCCGTCGGAGAGAAAGTGACCATGACATGCA
AGTCTAGTCAGACACTGCTCTACTCTAACAATCAGAAGAACTACCTCGCATGGTATCAGCAGAAACCA
GGACAGAGCCCCAAGCTGCTCATCTCCTGGGCTTTCACCCGGAAATCCGGGGTGCCTGACCGCTTCAC
AGGTAGCGGCTCCGGAACTGATTTTACTCTGACCATTGGATCTGTGAAGGCAGAGGACCTCGCCGTCT
ACTATTGCCAGCAGTACAGTAATTATCCATGGACTTTTGGCGGAGGGACCAGGCTGGAAATCAAGAGA
GGTGGAGGAGGGTCCGGTGGAGGAGGGTCTGGTGGAGGAGGGAGTGGTGGAGGAGGGTCAGAGGTGCA
GCTGCAGCAGTCTGGCCCCGAAGTGGTCAAAACTGGAGCTTCAGTCAAAATCAGCTGTAAGGCATCTG
GGTACAGCTTCACCGGCTACTTCATCAACTGGGTGAAGAAAAATTCAGGGAAGAGCCCTGAGTGGATC
GGCCACATTTCAAGCTCCTACGCCACAAGCACTTACAACCAGAAGTTCAAAAATAAGGCCGCTTTTAC
CGTGGACACATCTAGTTCAACCGCCTTCATGCAGCTGAACTCCCTCACATCTGAAGATAGTGCTGTGT
ACTATTGTGTCAGGAGCGGCAACTACGAAGAATATGCTATGGATTACTGGGGCAGGGGACCTCCGTG
ACTGTCTCAAGCACCGGTATTGCCACCACTGACCCTACTGCCCCAGGTACAGGAGGGACAGCTGTTGG
GATGCTGAGCACAGACTCTGCCACACAGTGGAGTCTAACCTCAGTAGAGACCGTCCAACCAGCATCCA
CAGAGGTAGAGACCTCGCAGCCAGCACCCATGGAGGCAGAGACCTCGCAGCCAGCACCCATGGAGGCA
GAGACCTCGCAGCCAGCACCCATGGAGGCAGACACCTCAAAGCCAGCACCCACGGAGGCAGAGACCTC
AAAGCCAGCACCCACGGAGGCAGAGACCTCTCAGCCAGCACCCAACGAGGCAGAGACCTCAAAACCAG
CACCCACGGAGGCAGAGACCTCAAAACCAGCACCCACGGAGGCAGAGACCACCCAGCTTCCCAGGATT
CAGGCTGTAAAAACTCTGTTTACAACGTCTGCAGCCACCGAAGTCCCTTCCACAGAACCTACCACCAT
GGAGACGGCGTCCACAGAGTCTAACGAGTCTACCATCTTCCTTGGGCCATCCGTGACTCACTTACCTG
ACAGCGGCCTGAAGAAAGGGCTGATTGTGACCCCTGGGAATTCACCTGCCCCAACCCTGCCAGGGAGT
TCAGATCTCATCCCGGTGAAGCAATGTCTGCTGATTATCCTCATCTTGGCTTCTCTGGCCACCATCTT
CCTCGTGTGCACAGTGGTGCTGGCGGTCCGTCTGTCCCGTAAGACCCACATGTACCCAGTGCGGAACT
ACTCCCCCACGGAGATGATCTGCATCTCGTCCCTGCTACCTGAGGGGGGAGACGGGGCCCCTGTCACA
GCCAATGGGGGCCTGCCCAAGGTCCAGGACCTGAAGACAGAGCCCAGTGGGGACCGGGATGGGGACGA
CCTCACCCTGCACAGCTTCCTCCCTTAG
Nucleic acid sequence of anti-HER2 scFv Trastuzumab
   SEQ ID NO: 76: 
GATATTCAGATGACCCAGTCCCCCAGCTCCCTGTCAGCAAGCGTGGGCGACCGAGTCACTATCACCTG
CCGAGCTAGCCAGGATGTGAACACCGCAGTCGCCTGGTACCAGCAGAAGCCAGGGAAAGCACCCAAGC
TGCTCATCTACTCCGCCTCTTTCCTGTATTCAGGAGTGCCAAGCAGGTTTAGTGGCTCAAGAAGCGGA
ACTGACTTCACACTGACTATCTCTAGTCTCCAGCCCGAGGATTTTGCAACCTACTATTGCCAGCAGCA
CTATACCACACCCCCTACCTTCGGTCAGGGCACAAAAGTGGAAATTAAGCGGACCGGCTCCACATCTG
GAAGTGGGAAGCCCGGTTCCGGCGAGGGATCTGAAGTGCAGCTGGTCGAGTCCGGAGGAGGACTCGTG
CAGCCTGGTGGCAGTCTGAGGCTCTCATGTGCCGCTAGCGGCTTCAACATCAAAGACACATACATTCA
TTGGGTGCGCCAGGCTCCTGGGAAGGGTCTGGAATGGGTCGCACGAATCTATCCAACTAATGGGTACA
CCCGATATGCTGACTCTGTGAAAGGCAGGTTCACAATTTCCGCCGATACATCTAAGAACACTGCTTAC
CTGCAGATGAATAGTCTCAGAGCTGAGGATACTGCAGTCTACTATTGTAGCCGGTGGGGAGGGGATGG
CTTCTATGCTATGGATGTCTGGGGGCAGGGGACTCTGGTGACTGTCTCAAGTGGTACCGGTACGCGTG
Nucleic acid sequence of anti-HER2 scFv Pertuzumab
   SEQ ID NO: 77: 
GATATTCAGATGACCCAGAGCCCAAGCTCCCTGTCAGCTAGCGTGGGCGACCGAGTCACCATCACATG
CAAAGCCAGTCAGGATGTGTCAATTGGCGTCGCTTGGTACCAGCAGAAGCCCGGAAAAGCTCCTAAGC
TGCTCATCTATTCCGCATCTTACAGGTACACAGGCGTGCCCTCTCGCTTCAGTGGTTCAGGCAGCGGA
ACTGACTTTACTCTGACCATTTCTAGTCTCCAGCCTGAGGATTTCGCAACCTACTATTGTCAGCAGTA
CTATATCTACCCATATACCTTTGGGCAGGGTACAAAAGTGGAAATTAAGAGAACAGTCGCAGCTCCAG
GAGGAGGAGGTAGCGGAGGAGGGGGTTCCGGCGGAGGGGGTTCTGGCGGAGGGGGTAGTGAGGTGCAG
 
CTGGTCGAAAGCGGAGGAGGACTCGTGCAGCCTGGTGGCAGCCTGAGACTCTCCTGCGCAGCCTCTGG
CTTCACCTTCACCGACTACACCATGGATTGGGTGCGGCAGGCACCAGGAAAGGGACTGGAGTGGGTGG
CAGACGTCAACCCCAATTCCGGAGGGTCTATCTACAACCAGAGGTTCAAAGGAAGGTTCACCCTGAGT
GTGGATCGATCAAAGAACACCCTGTATCTCCAGATGAATTCCCTGAGGGCCGAAGATACAGCCGTCTA
TTATTGTGCAAGAAACCTGGGTCCATCATTTTATTTTGACTATTGG
Nucleic acid sequence of anti-HER2 scFv FRP5
   SEQ ID NO: 78: 
CAGGTCCAGCTCCAGCAGTCAGGTCCAGAACTCAAGAAGCCAGGGGAAACAGTCAAAATCTCATGTAA
AGCCTCAGGATACCCATTCACTAACTATGGGATGAATTGGGTGAAGCAGGCACCTGGCCAGGGACTGA
AATGGATGGGTTGGATCAACACTAGCACCGGGGAGTCCACATTCGCCGACGATTTTAAGGGCCGGTTC
GACTTTTCTCTCGAAACCAGTGCAAATACAGCCTATCTGCAGATTAACAATCTCAAATCCGAGGATAT
GGCCACCTACTTCTGCGCTCGCTGGGAAGTGTACCACGGATATGTCCCATACTGGGGGCAGGGTACCA
CAGTGACAGTCAGCTCCGGAGGAGGAGGTTCAGGAGGAGGAGGTAGCGGAGGAGGAGGTTCCGACATC
CAGCTGACACAGTCTCATAAGTTTCTCTCCACTTCTGTGGGCGACAGGGTCTCTATTACCTGTAAAGC
TAGTCAGGATGTGTATAACGCCGTCGCTTGGTACCAGCAGAAGCCCGGCCAGAGCCCTAAACTGCTCA
TCTATAGCGCCTCTAGTAGGTACACTGGAGTGCCAAGCAGATTCACCGGCAGTGGATCAGGGCCCGAC
TTCACCTTCACCATTTCAAGCGTGCAGGCTGAGGATCTGGCAGTCTACTTTTGCCAGCAGCATTTTCG
CACCCCTTTCACCTTTGGAAGCGGGACTAAACTGGAGATTAAGAGGA
Amino acid sequence of hinge domain derived from dLNGFR
   SEQ ID NO: 79: 
LLGVSLGGAKEACPTGLYTHSGECCKACNLGEGVAQPCGANQTVCEPCLDSVTFSDVVSATEPCKPCT
ECVGLQSMSAPCVEADDAVCRCAYGYYQDETTGRCEACRVCEAGSGLVFSCQDKQNTVCEECPDGTYS
DEANHVDPCLPCTVCEDTERQLRECTRWADAECEEIPGRWITRSTPPEGSDSTAPSTQEPEAPPEQDL
IASTVAGVVTTVMGSSQPVVTRGTTDN
Amino acid sequence of a transmembrane domain derived from dLNGFR
   SEQ ID NO: 80:
   LIPVYCSILAAVVVGLVAYIAF
Amino acid sequence of an intracellular domain derived from dLNGFR
   SEQ ID NO: 81:
   KRWNRGIL
Amino acid sequence of hinge domain derived from FcγRIIIA
   SEQ ID NO: 82:
   HENSELLIPKATHNDSGSYFCRGLIGHNNKSSASFRISLGDPGSPSMFPP
Amino acid sequence of a transmembrane domain derived from FcγRIIIA
   SEQ ID NO: 83:
   WHQITFCLLIGLLFAIDTVLYF
Amino acid sequence of an intracellular domain derived from FcγRIIIA
   SEQ ID NO: 84:
   SVRRGLQSPVADYEEPKIQWSKEPQDKTRVD
Amino acid sequence of hinge domain derived from FLT3
   SEQ ID NO: 85:
   PGPFPFIQDN
Amino acid sequence of a transmembrane domain derived from FLT3
   SEQ ID NO: 86:
   ISFYATIGLCLPFIVVLIVLIC
Amino acid sequence of an intracellular domain derived from FLT3
   SEQ ID NO: 87: 
HKYKKQFRYESQLQMIQVTGPLDNEYFYVDFRDYEYDLKWEFPRENLEFGKVLGSGAFGRVMNATAYG
ISKTGVSIQVAVKMLKEKADSCEKEALMSELKMMTHLGHHDNIVNLLGACTLSGPVYLIFEYCCYGDL
LNYLRSKREKFHRTWTEIFKEHNFSFYPTFQAHSNSSMPGSREVQLHPPLDQLSGFNGNLIHSEDEIE
YENQKRLAEEEEEDLNVLTFEDLLCFAYQVAKGMEFLEFKSCVHRDLAARNVLVTHGKVVKICDFGLA
RDILSDSSYVVRGNARLPVKWMAPESLFEGIYTIKSDVWSYGILLWEIFSLGVNPYPGIPVDANFYKL
IQSGFKMEQPFYATEGIYFVMQSCWAFDSRKRPSFPNLTSFLGCQLAEAEEAMYQNMGGNVPEHPSIY
QNRRPLSREAGSEPPSPQAQVKIHGERS
Amino acid sequence of hinge domain derived from TLR4
   SEQ ID NO: 88: 
QLYSLSTLDCSFNRIETSKGILQHFPKSLAFFNLTNNSVACICEHQKFLQWVKEQKQFLVNVEQMTCA
TPVEMNTSLVLDFNNSTCYMYKTIISVSVVS
Amino acid sequence of a transmembrane domain derived from TLR4
   SEQ ID NO: 89:
   VIVVSTVAFLIYHFYFHLILI
Amino acid sequence of an intracellular domain derived from TLR4
   SEQ ID NO: 90: 
AGCKKYSRGESIYDAFVIYSSQNEDWVRNELVKNLEEGVPRFHLCLHYRDFIPGVAIAANIIQEGFHK
SRKVIVVVSRHFIQSRWCIFEYEIAQTWQFLSSRSGIIFIVLEKVEKSLLRQQVELYRLLSRNTYLEW
EDNPLGRHIFWRRLKNALLDGKASNPEQTAEEEQETATWT
Amino acid sequence of hinge domain derived from CCR2
   SEQ ID NO: 91:
   MEDNNMLPQFIHGILSTSHSLFTRSIQELDEGATTPYDYDDGEPCHKTSVKQIGA
Amino acid sequence of hinge domain derived from ITGB2
   SEQ ID NO: 92: 
NARLVECSGRGHCQCNRCICDEGYQPPMCEDCPSCGSHCRDNHTSCAECLKFDKGPFEKNCSVQCAGM
TLQTIPLKKKPCKERDSEGCWITYTLQQKDGRNIYNIHVEDSLECVKGPN
Amino acid sequence of a transmembrane domain derived from ITGB2
   SEQ ID NO: 93:
   VAAIVGGTVVGVVLIGVLLLVIW
Amino acid sequence of an intracellular domain derived from ITGB2
   SEQ ID NO: 94:
   KALTHLTDLREYRRFEKEKLKSQWNNDNPLFKSATTTVMNPKFAES
Amino acid sequence of hinge domain derived from CSF2RB
   SEQ ID NO: 95: 
TQKMAYSFIEHTFQVQYKKKSDSWEDSKTENLDRAHSMDLSQLEPDTSYCARVRVKPISNYDGIWSKW
SEEYTWKTDW
Amino acid sequence of a transmembrane domain derived from CSF2RB
   SEQ ID NO: 96:
   VMPTLWIVLILVFLILTLLLIL
Amino acid sequence of an intracellular domain derived from CSF2RB
   SEQ ID NO: 97: 
RFGCVSVYRTYRKWKEKIPNPSKSLLFQDGGKGLWPPGSMAAFATKNPALQGPQSRLLAEQQGESYAH
LEDNNVSPLTIEDPNIIRVPPSGPDTTPAASSESTEQLPNVQVEGPTPNRPRKQLPSFDFNGPYLGPP
 
QSHSLPDLPDQLGSPQVGGSLKPALPGSLEYMCLPPGGQAQLVPLSQVMGQGQAMDVQCGSSLETSGS
PSVEPKENPPVELSMEEQEARDNPVTLPISSGGPEGSMMASDYVTPGDPVLTLPTGPLSTSLGPSLGL
PSAQSPRLCLKLPRVPSGSPALGPPGFEDYVELPPSVSQAAKSPPGHPAPPVASSPTVIPGEPREEVG
PASPHPEGLLVLQQVGDYCFLPGLGPGSLSPHSKPPSPSLCSETEDLVQDLSVKKFPYQPMPQAPAIQ
FFKSLKHQDYLSLPPWDNSQSGKVC
Amino acid sequence of hinge domain derived from CCR1
   SEQ ID NO: 98:
   TPCQKTAVRAFGA
Amino acid sequence of hinge domain derived from CCR5
   SEQ ID NO: 99:
   MSAPCQKINVKQIAA
Amino acid sequence of hinge domain derived from CXCR4
   SEQ ID NO: 100:
   FRDENVHFNR
Amino acid sequence of hinge domain derived from SELPLG
   SEQ ID NO: 101: 
IATTDPTAPGTGGTAVGMLSTDSATQWSLTSVETVQPASTEVETSQPAPMEAETSQPAPMEAETSQPA
PMEADTSKPAPTEAETSKPAPTEAETSQPAPNEAETSKPAPTEAETSKPAPTEAETTQLPRIQAVKTL
FTTSAATEVPSTEPTTMETASTESNESTIFLGPSVTHLPDSGLKKGLIVTPGNSPAPTLPGSSDLIPV
KQC
Amino acid sequence of a transmembrane domain derived from SELPLG
   SEQ ID NO: 102:
   LLIILILASLATIFLVCTVVL
Amino acid sequence of an intracellular domain derived from SELPLG
   SEQ ID NO: 103: 
AVRLSRKTHMYPVRNYSPTEMICISSLLPEGGDGAPVTANGGLPKVQDLKTEPSGDRDGDDLTLHSFL
P
Amino acid sequence of peptide that facilitates DNA engineering
   SEQ ID NO: 104:
   TG
Nucleic acid sequence of IgK domain (for FRP5 and trastuzumab)
   SEQ ID NO: 105:
   ATGGATTTTCAGGTGCAGATTTTCTCTTTCCTCCTCATTTCCGCCTCAGTGATTATGTCAAGGGGG
Nucleic acid sequence of IgK domain (for pertuzumab)
   SEQ ID NO: 106:
   ATGGATTTTCAGGTGCAGATTTTCTCCTTTCTCCTCATTTCAGCCAGCGTGATTATGTCTCGGGGG
Nucleic acid sequence of a peptide that facilitates DNA engineering
   SEQ ID NO: 107:
   ACCGGT
Nucleic acid sequence of a peptide that facilitates DNA engineering
   SEQ ID NO: 108:
   ACCGGG
Nucleic acid sequence of IgK domain (for EVIR-N1)
   SEQ ID NO: 109:
   ATGGACTTCCAGGTGCAGATCTTCAGCTTCCTGCTGATCTCCGCCAGCGTGATCATGAGCAGAGGC
Nucleic acid sequence of IgK domain (for EVIR-N2)
   SEQ ID NO: 110:
   ATGGATTTTCAGGTGCAGATCTTCAGCTTCCTGCTGATCTCCGCCAGCGTGATCATGAGCAGAGGC
Nucleic acid sequence of anti-TYRP1 scFv TA99
   SEQ ID NO: 111: 
GACATCCAGATGAGCCAGAGCCCTGCCAGCCTGTCTGCCTCTGTGGGCGAGACAGTGACCATCACCTG
TAGAGCCAGCGGCAACATCTACAACTACCTGGCCTGGTATCAGCAGAAGCAGGGCAAGAGCCCCCATC
TGCTGGTGTACGACGCCAAGACACTGGCCGACGGCGTGCCCTCTAGATTCTCTGGCAGCGGCTCCGGC
ACCCAGTACAGCCTGAAGATCAGCTCCCTGCAGACCGAGGACTCCGGCAACTACTACTGCCAGCACTT
CTGGTCCCTGCCCTTCACCTTCGGCAGCGGCACCAAGCTGGAAATCAAGAGAGGCGGCGGAGGCTCTG
GCGGAGGCGGATCTGGGGGCGGAGGAAGTGGCGGGGGAGGATCTGAAGTGCAGCTGCAGCAGTCTGGC
GCTGAGCTCGTGCGACCTGGCGCTCTCGTGAAGCTGAGCTGCAAGACCAGCGGCTTCAATATCAAGGA
CTACTTCCTGCACTGGGTGCGACAGAGGCCTGACCAGGGCCTGGAATGGATCGGCTGGATCAACCCCG
ACAACGGCAACACCGTGTACGACCCTAAGTTCCAGGGCACCGCCAGCCTGACAGCCGACACAAGCTCC
AACACAGTGTACCTGCAGCTGAGCGGCCTGACCTCCGAGGATACCGCCGTGTACTTCTGCACCAGAAG
AGACTACACCTACGAGAAGGCCGCCCTGGACTACTGGGGCCAGGGAACAACCGTGACCGTGTCC
Nucleic acid sequence of anti-GD2 scFv 14G2a
   SEQ ID NO: 112: 
GAAGTTCAGCTGCTGCAGAGCGGACCCGAACTGGAAAAACCTGGCGCCTCCGTGATGATCAGCTGCAA
GGCCTCTGGCAGCTCCTTCACCGGCTACAACATGAACTGGGTCCGACAGAACATCGGCAAGAGCCTGG
AATGGATCGGCGCCATCGATCCTTACTACGGCGGCACCAGCTACAACCAGAAGTTCAAGGGCAGAGCC
ACACTGACCGTGGACAAGAGCAGCAGCACAGCCTACATGCATCTGAAGTCCCTGACCAGCGAGGACAG
CGCCGTGTACTACTGTGTGTCCGGCATGGAATACTGGGGCCAGGGCACAAGCGTGACAGTCTCTTCTG
GCGGCGGTGGATCTGGCGGAGGCGGAAGTGGTGGCGGCGGATCTGATGTGGTCATGACACAGACCCCT
CTGAGCCTGCCTGTGTCTCTGGGAGATCAGGCCAGCATCAGCTGTAGAAGCAGCCAGAGCCTGGTGCA
CAGAAACGGCAACACCTACCTGCACTGGTATCTGCAGAAGCCCGGCCAGTCTCCTAAGCTGCTGATCC
ACAAGGTGTCCAACAGATTCAGCGGCGTGCCCGACAGATTCTCTGGCTCTGGAAGCGGCACCGACTTC
ACCCTGAAGATTAGCAGAGTGGAAGCCGAGGACCTGGGCGTGTACTTCTGTAGCCAGAGCACACACGT
GCCACCTCTGACATTTGGCGCTGGCACCAAGCTGGAACTG
Amino acid sequence of TA99-based anti-TYRP1 scFv
   SEQ ID NO: 113: 
DIQMSQSPASLSASVGETVTITCRASGNIYNYLAWYQQKQGKSPHLLVYDAKTLADGVPSRFSGSGSG
TQYSLKISSLQTEDSGNYYCQHFWSLPFTFGSGTKLEIKRGGGGSGGGGSGGGGSGGGGSEVQLQQSG
AELVRPGALVKLSCKTSGFNIKDYFLHWVRQRPDQGLEWIGWINPDNGNTVYDPKFQGTASLTADTSS
NTVYLQLSGLTSEDTAVYFCTRRDYTYEKAALDYWGQGTTVTVS
Amino acid sequence of 14G2a-based anti-GD2 scFv
   SEQ ID NO: 114: 
EVQLLQSGPELEKPGASVMISCKASGSSFTGYNMNWVRQNIGKSLEWIGAIDPYYGGTSYNQKFKGRA
TLTVDKSSSTAYMHLKSLTSEDSAVYYCVSGMEYWGQGTSVTVSSGGGGSGGGGSGGGGSDVVMTQTP
LSLPVSLGDQASISCRSSQSLVHRNGNTYLHWYLQKPGQSPKLLIHKVSNRFSGVPDRFSGSGSGTDF
TLKISRVEAEDLGVYFCSQSTHVPPLTFGAGTKLEL
Nucleic acid sequence of Cxcl9_Fw_XmaI
   SEQ ID NO: 115: AAAAACCCGGGTCACTCCAACACAGTGACTC
Nucleic acid sequence of Cxcl9_Rv_SalI/NheI
   SEQ ID NO: 116: AAAAAGTCGACGCTAGCCAGGGTGCTTGTTGGTAAAGT
Nucleic acid sequence of Cxcl9
   SEQ ID NO: 117: 
tcactccaacacagtgactcaatagaactcagctctgccatgaagtccgctgttcttttcctcttggg
catcatcttcctggagcagtgtggagttcgaggaaccctagtgataaggaatgcacgatgctcctgca
tcagcaccagccgaggcacgatccactacaaatccctcaaagacctcaaacagtttgccccaagcccc
aattgcaacaaaactgaaatcattgctacactgaagaacggagatcaaacctgcctagatccggactc
ggcaaatgtgaagaagctgatgaaagaatgggaaaagaagatcagccaaaagaaaaagcaaaagaggg
ggaaaaaacatcaaaagaacatgaaaaacagaaacccaaaacaccccaaagtcgtcgtcgttcaagg
aagactacataagagaccattactttaccaacaagcaccctg
Nucleic acid sequence of GM-CSF_Fw_XmaI
   SEQ ID NO: 118: AAAAACCCGGGCAGAGAGAAAGGCTAAGGTCC
Nucleic acid sequence of GM-CSF_Rv_SalI/NheI
   SEQ ID NO: 119: AAAAAGTCGACGCTAGCAGTCTGAGAAGCTGGATT
Nucleic acid sequence of GM-CSF
   SEQ ID NO: 120: 
CAGAGAGAAAGGCTAAGGTCCTGAGGAGGATGTGGCTGCAGAATTTACTTTTCCTGGGCATTGTGGTC
TACAGCCTCTCAGCACCCACCCGCTCACCCATCACTGTCACCCGGCCTTGGAAGCATGTAGAGGCCAT
CAAAGAAGCCCTGAACCTCCTGGATGACATGCCTGTCACGTTGAATGAAGAGGTAGAAGTCGTCTCTA
ACGAGTTCTCCTTCAAGAAGCTAACATGTGTGCAGACCCGCCTGAAGATATTCGAGCAGGGTCTACGG
GGCAATTTCACCAAACTCAAGGGCGCCTTGAACATGACAGCCAGCTACTACCAGACATACTGCCCCCC
AACTCCGGAAACGGACTGTGAAACACAAGTTACCACCTATGCGGATTTCATAGACAGCCTTAAAACCT
TTCTGACTGATATCCCCTTTGAATGCAAAAAACCAGGCCAAAAATGAGGAAGCCCAGGCCAGCTCTGA
ATCCAGCTTCTCAGACT
Nucleic acid sequence of IFNγ_Fw_XmaI
   SEQ ID NO: 121: AAAAACCCGGGAGTTCTGGGCTTCTCCTCCT
Nucleic acid sequence of IFNγ_Rv_SalI/NheI
   SEQ ID NO: 122: AAAAAGTCGACGCTAGCGACAATCTCTTCCCCACCCC
Nucleic acid sequence of IFNγ
   SEQ ID NO: 123: 
AGTTCTGGGCTTCTCCTCCTGCGGCCTAGCTCTGAGACAATGAACGCTACACACTGCATCTTGGCTTT
GCAGCTCTTCCTCATGGCTGTTTCTGGCTGTTACTGCCACGGCACAGTCATTGAAAGCCTAGAAAGTC
TGAATAACTATTTTAACTCAAGTGGCATAGATGTGGAAGAAAAGAGTCTCTTCTTGGATATCTGGAGG
AACTGGCAAAAGGATGGTGACATGAAAATCCTGCAGAGCCAGATTATCTCTTTCTACCTCAGACTCTT
TGAAGTCTTGAAAGACAATCAGGCCATCAGCAACAACATAAGCGTCATTGAATCACACCTGATTACTA
CCTTCTTCAGCAACAGCAAGGCGAAAAAGGATGCATTCATGAGTATTGCCAAGTTTGAGGTCAACAAC
CCACAGGTCCAGCGCCAAGCATTCAATGAGCTCATCCGAGTGGTCCACCAGCTGTTGCCGGAATCCAG
CCTCAGGAAGCGGAAAAGGAGTCGCTGCTGATTCGGGGTGGGGAAGAGATTGTC
Nucleic acid sequence of LIN28_Fw_BamHI
   SEQ ID NO: 124: AAAAAGGATCCCTTTGCCTCCGGACTTCTCTGG
Nucleic acid sequence of LIN28_Rv_SalI
   SEQ ID NO: 125: AAAAAGTCGACAAAGACAGGGTGACACTGGGA
Nucleic acid sequence of LIN28 (PstI and SmaI- mouse trophoblast cells)
   SEQ ID NO: 126: 
CTTTGCCTCCGGACTTCTCTGGGGCCAGCAGCCGCCCGACCTGGGGCCCGGGCCACGGCTCAGCAG
ACGACCATGGGCTCGGTGTCCAACCAGCAGTTTGCAGGTGGCTGCGCCAAGGCAGCGGAGAAGGCGCC
AGAGGAGGCGCCGCCTGACGCGGCCCGAGCGGCAGACGAGCCGCAGCTGCTGCACGGGGCCGGCATCT
GTAAGTGGTTCAACGTGCGCATGGGGTTCGGCTTCCTGTCTATGACCGCCCGCGCTGGGGTCGCGCTC
 
GACCCCCCGGTGGACGTCTTTGTGCACCAGAGCAAGCTGCACATGGAAGGGTTCCGAAGCCTCAAGGA
GGGTGAGGCGGTGGAGTTCACCTTTAAGAAGTCTGCCAAGGGTCTGGAATCCATCCGTGTCACTGGCC
CTGGTGGTGTGTTCTGTATTGGAAGTGAGCGGCGGCCAAAGGGGAAGAACATGCAGAAGCGAAGATCC
AAAGGAGACAGGTGCTACAACTGCGGTGGGCTAGACCATCATGCCAAGGAATGCAAGCTGCCACCCCA
GCCCAAGAAGTGCCACTTTTGCCAAAGCATCAACCATATGGTGGCCTCGTGTCCACTGAAGGCCCAGC
AGGGCCCCAGTTCTCAGGGAAAGCCTGCCTACTTCCGGGAGGAAGAGGAAGAGATCCACAGCCCTGCC
CTGCTCCCAGAAGCCCAGAATTGAGGCCCAGGAGTCAGGGTTATTCTTTGGCTAATGGGGAGTTTAAG
GAAAGAGGCATCAATCTGCAGAGTGGAGAAAGTGGGGGTAAGGGTGGGTTGCGTGGGTAGCTTGCACT
GCCGTGTCTCAGGCCGGGGTTCCCAGTGTCACCCTGTCTTT
Nucleic acid sequence of CD40(GeneArt CD40 Blunt sites (SmaI - AgeI-blunted) in AfeI/NheI-blunted bidirectional)
   SEQ ID NO: 127: 
GCCACCATGGTCTCTCCCTCGGCTGTGTGCTCTGTGGGGTTGTCTGCTCACCGCTGTGCATCTCGG
CCAGTGTGTGACTTGTTCTGATAAACAGTACCTGCATGACGGCAGTGCTGTGATCTGTGCCAGCCCG
GTTCTAGGCTCACCAGTCATTGTACAGCCCTGGAGAAGACTCAGTGCCACCCTTGTGACTCAGGGGAG
TTCAGCGCTCAGTGGAACCGAGAAATTAGGTGCCACCAGCATAGACACTGTGAGCCTAATCAGGGGCT
GCGGGTGAAGAAAGAGGGTACCGCAGAAAGTGACACTGTCTGCACCTGTAAGGAGGGCCAGCATTGCA
CCTCAAAAGATTGCGAAGCTTGTGCACAGCACACACCTTGTATCCCAGGCTTCGGAGTGATGGAGATG
GCTACTGAAACCACAGACACCGTGTGCCACCCATGTCCCGTCGGATTCTTTTCTAACCAGAGCTCCCT
CTTTGAGAAGTGCTATCCATGGACAAGCTGTGAGGATAAGAACCTGGAAGTGCTCCAGAAAGGCACAT
CCCAGACTAATGTCATTTGCGGACTGAAATCTCGGATGCGCGCCCTGCTCGTGATCCCAGTGGTCATG
GGCATCCTCATTACTATCTTCGGAGTGTTTCTGTACATTAAGAAAGTGGTCAAGAAACCCAAGGACAA
CGAGATCCTCCCACCTGCAGCTAGGAGACAGGACCCCCAGGAGATGGAAGATTATCCTGGACATAATA
CAGCAGCCCCAGTGCAGGAAACTCTGCACGGGTGTCAGCCCGTCACCCAGGAGGATGGCAAGGAAAGC
AGAATCTCCGTCCAGGAAAGGCAGGTCACTGATAGCATCGCACTCCGCCCACTCGTCTGA
Nucleic acid sequence of anti-HER2 scFv CHA21 (1)
   SEQ ID NO: 128: 
GATATTGTCCTCACACAGACTCCCAGCTCCCTGCCTGTGTCCGTCGGAGAGAAAGTGACCATGACATG
CAAGTCTAGTCAGACACTGCTCTACTCTAACAATCAGAAGAACTACCTCGCATGGTATCAGCAGAAAC
CAGGACAGAGCCCCAAGCTGCTCATCTCCTGGGCTTTCACCCGGAAATCCGGGGTGCCTGACCGCTTC
ACAGGTAGCGGCTCCGGAACTGATTTTACTCTGACCATTGGATCTGTGAAGGCAGAGGACCTCGCCGT
CTACTATTGCCAGCAGTACAGTAATTATCCATGGACTTTTGGCGGAGGGACCAGGCTGGAAATCAAGA
GAGGTGGAGGAGGGTCCGGTGGAGGAGGGTCTGGTGGAGGAGGGAGTGGTGGAGGAGGGTCAGAGGTG
CAGCTGCAGCAGTCTGGCCCCGAAGTGGTCAAAACTGGAGCTTCAGTCAAAATCAGCTGTAAGGCATC
TGGGTACAGCTTCACCGGCTACTTCATCAACTGGGTGAAGAAAAATTCAGGGAAGAGCCCTGAGTGGA
TCGGCCACATTTCAAGCTCCTACGCCACAAGCACTTACAACCAGAAGTTCAAAAATAAGGCCGCTTTT
ACCGTGGACACATCTAGTTCAACCGCCTTCATGCAGCTGAACTCCCTCACATCTGAAGATAGTGCTGT
GTACTATTGTGTCAGGAGCGGCAACTACGAAGAATATGCTATGGATTACTGGGGGCAGGGGACCTCCG
TGACTGTCTCAAGC
Nucleic acid sequence of IgK domain (1)
   SEQ ID NO: 129:
   ATGGATTTTCAGGTCCAGATTTTCTCCTTCCTCCTCATTTCAGCCAGCGTCATTATGTCTCGGGGG
Nucleic acid sequence of EVIR-N1
   SEQ ID NO: 130: 
ATGGACTTCCAGGTGCAGATCTTCAGCTTCCTGCTGATCTCCGCCAGCGTGATCATGAGCAGAGGCGA
CATCCAGATGAGCCAGAGCCCTGCCAGCCTGTCTGCCTCTGTGGGCGAGACAGTGACCATCACCTGTA
GAGCCAGCGGCAACATCTACAACTACCTGGCCTGGTATCAGCAGAAGCAGGGCAAGAGCCCCCATCTG
CTGGTGTACGACGCCAAGACACTGGCCGACGGCGTGCCCTCTAGATTCTCTGGCAGCGGCTCCGGCAC
CCAGTACAGCCTGAAGATCAGCTCCCTGCAGACCGAGGACTCCGGCAACTACTACTGCCAGCACTTCT
GGTCCCTGCCCTTCACCTTCGGCAGCGGCACCAAGCTGGAAATCAAGAGAGGCGGCGGAGGCTCTGGC
GGAGGCGGATCTGGGGGCGGAGGAAGTGGCGGGGGAGGATCTGAAGTGCAGCTGCAGCAGTCTGGCGC
TGAGCTCGTGCGACCTGGCGCTCTCGTGAAGCTGAGCTGCAAGACCAGCGGCTTCAATATCAAGGACT
 
ACTTCCTGCACTGGGTGCGACAGAGGCCTGACCAGGGCCTGGAATGGATCGGCTGGATCAACCCCGAC
AACGGCAACACCGTGTACGACCCTAAGTTCCAGGGCACCGCCAGCCTGACAGCCGACACAAGCTCCAA
CACAGTGTACCTGCAGCTGAGCGGCCTGACCTCCGAGGATACCGCCGTGTACTTCTGCACCAGAAGAG
ACTACACCTACGAGAAGGCCGCCCTGGACTACTGGGGCCAGGGAACAACCGTGACCGTGTCCACCGGT
CTTCTGGGGGTGTCCCTTGGAGGTGCCAAGGAGGCATGCCCCACAGGCCTGTACACACACAGCGGTGA
GTGCTGCAAAGCCTGCAACCTGGGCGAGGGTGTGGCCCAGCCTTGTGGAGCCAACCAGACCGTGTGTG
AGCCCTGCCTGGACAGCGTGACGTTCTCCGACGTGGTGAGCGCGACCGAGCCGTGCAAGCCGTGCACC
GAGTGCGTGGGGCTCCAGAGCATGTCGGCGCCGTGCGTGGAGGCCGACGACGCCGTGTGCCGCTGCGC
CTACGGCTACTACCAGGATGAGACGACTGGGCGCTGCGAGGCGTGCCGCGTGTGCGAGGCGGGCTCGG
GCCTCGTGTTCTCCTGCCAGGACAAGCAGAACACCGTGTGCGAGGAGTGCCCCGACGGCACGTATTCC
GACGAGGCCAACCACGTGGACCCGTGCCTGCCCTGCACCGTGTGCGAGGACACCGAGCGCCAGCTCCG
CGAGTGCACACGCTGGGCCGACGCCGAGTGCGAGGAGATCCCTGGCCGTTGGATTACACGGTCCACAC
CCCCAGAGGGCTCGGACAGCACAGCCCCCAGCACCCAGGAGCCTGAGGCACCTCCAGAACAAGACCTC
ATAGCCAGCACGGTGGCAGGTGTGGTGACCACAGTGATGGGCAGCTCCCAGCCCGTGGTGACCCGAGG
CACCACCGACAACCTCATCCCTGTCTATTGCTCCATCCTGGCTGCTGTGGTTGTGGGCCTTGTGGCCT
ACATAGCCTTCAAGAGGTGGAACAGGGGGATCCTCTAG
Nucleic acid sequence of EVIR-N2
   SEQ ID NO: 131: 
ATGGATTTTCAGGTGCAGATCTTCAGCTTCCTGCTGATCTCCGCCAGCGTGATCATGAGCAGAGGCGA
AGTTCAGCTGCTGCAGAGCGGACCCGAACTGGAAAAACCTGGCGCCTCCGTGATGATCAGCTGCAAGG
CCTCTGGCAGCTCCTTCACCGGCTACAACATGAACTGGGTCCGACAGAACATCGGCAAGAGCCTGGAA
TGGATCGGCGCCATCGATCCTTACTACGGCGGCACCAGCTACAACCAGAAGTTCAAGGGCAGAGCCAC
ACTGACCGTGGACAAGAGCAGCAGCACAGCCTACATGCATCTGAAGTCCCTGACCAGCGAGGACAGCG
CCGTGTACTACTGTGTGTCCGGCATGGAATACTGGGGCCAGGGCACAAGCGTGACAGTCTCTTCTGGC
GGCGGTGGATCTGGCGGAGGCGGAAGTGGTGGCGGCGGATCTGATGTGGTCATGACACAGACCCCTCT
GAGCCTGCCTGTGTCTCTGGGAGATCAGGCCAGCATCAGCTGTAGAAGCAGCCAGAGCCTGGTGCACA
GAAACGGCAACACCTACCTGCACTGGTATCTGCAGAAGCCCGGCCAGTCTCCTAAGCTGCTGATCCAC
AAGGTGTCCAACAGATTCAGCGGCGTGCCCGACAGATTCTCTGGCTCTGGAAGCGGCACCGACTTCAC
CCTGAAGATTAGCAGAGTGGAAGCCGAGGACCTGGGCGTGTACTTCTGTAGCCAGAGCACACACGTGC
CACCTCTGACATTTGGCGCTGGCACCAAGCTGGAACTGACCGGTCTTCTGGGGGTGTCCCTTGGAGGT
GCCAAGGAGGCATGCCCCACAGGCCTGTACACACACAGCGGTGAGTGCTGCAAAGCCTGCAACCTGGG
CGAGGGTGTGGCCCAGCCTTGTGGAGCCAACCAGACCGTGTGTGAGCCCTGCCTGGACAGCGTGACGT
TCTCCGACGTGGTGAGCGCGACCGAGCCGTGCAAGCCGTGCACCGAGTGCGTGGGGCTCCAGAGCATG
TCGGCGCCGTGCGTGGAGGCCGACGACGCCGTGTGCCGCTGCGCCTACGGCTACTACCAGGATGAGAC
GACTGGGCGCTGCGAGGCGTGCCGCGTGTGCGAGGCGGGCTCGGGCCTCGTGTTCTCCTGCCAGGACA
AGCAGAACACCGTGTGCGAGGAGTGCCCCGACGGCACGTATTCCGACGAGGCCAACCACGTGGACCCG
TGCCTGCCCTGCACCGTGTGCGAGGACACCGAGCGCCAGCTCCGCGAGTGCACACGCTGGGCCGACGC
CGAGTGCGAGGAGATCCCTGGCCGTTGGATTACACGGTCCACACCCCCAGAGGGCTCGGACAGCACAG
CCCCCAGCACCCAGGAGCCTGAGGCACCTCCAGAACAAGACCTCATAGCCAGCACGGTGGCAGGTGTG
GTGACCACAGTGATGGGCAGCTCCCAGCCCGTGGTGACCCGAGGCACCACCGACAACCTCATCCCTGT
CTATTGCTCCATCCTGGCTGCTGTGGTTGTGGGCCTTGTGGCCTACATAGCCTTCAAGAGGTGGAACA
GGGGGATCCTCTAG
Amino acid sequence of EVIR-N1
   SEQ ID NO: 132: 
MDFQVQIFSFLLISASVIMSRGDIQMSQSPASLSASVGETVTITCRASGNIYNYLAWYQQKQGKSPHL
LVYDAKTLADGVPSRFSGSGSGTQYSLKISSLQTEDSGNYYCQHFWSLPFTFGSGTKLEIKRGGGGSG
GGGSGGGGSGGGGSEVQLQQSGAELVRPGALVKLSCKTSGFNIKDYFLHWVRQRPDQGLEWIGWINPD
NGNTVYDPKFQGTASLTADTSSNTVYLQLSGLTSEDTAVYFCTRRDYTYEKAALDYWGQGTTVTVSTG
LLGVSLGGAKEACPTGLYTHSGECCKACNLGEGVAQPCGANQTVCEPCLDSVTFSDVVSATEPCKPCT
ECVGLQSMSAPCVEADDAVCRCAYGYYQDETTGRCEACRVCEAGSGLVFSCQDKQNTVCEECPDGTYS
DEANHVDPCLPCTVCEDTERQLRECTRWADAECEEIPGRWITRSTPPEGSDSTAPSTQEPEAPPEQDL
IASTVAGVVTTVMGSSQPVVTRGTTDNLIPVYCSILAAVVVGLVAYIAFKRWNRGIL
Amino acid sequence of EVIR-N2
   SEQ ID NO: 133: 
MDFQVQIFSFLLISASVIMSRGEVQLLQSGPELEKPGASVMISCKASGSSFTGYNMNWVRQNIGKSLE
WIGAIDPYYGGTSYNQKFKGRATLTVDKSSSTAYMHLKSLTSEDSAVYYCVSGMEYWGQGTSVTVSSG
GGGSGGGGSGGGGSDVVMTQTPLSLPVSLGDQASISCRSSQSLVHRNGNTYLHWYLQKPGQSPKLLIH
KVSNRFSGVPDRFSGSGSGTDFTLKISRVEAEDLGVYFCSQSTHVPPLTFGAGTKLELTGLLGVSLGG
AKEACPTGLYTHSGECCKACNLGEGVAQPCGANQTVCEPCLDSVTFSDVVSATEPCKPCTECVGLQSM
SAPCVEADDAVCRCAYGYYQDETTGRCEACRVCEAGSGLVFSCQDKQNTVCEECPDGTYSDEANHVDP
CLPCTVCEDTERQLRECTRWADAECEEIPGRWITRSTPPEGSDSTAPSTQEPEAPPEQDLIASTVAGV
VTTVMGSSQPVVTRGTTDNLIPVYCSILAAVVVGLVAYIAFKRWNRGIL
Nucleic acid sequence of TYRP1 Fw
   SEQ ID NO: 134: AAAAAAACCGGTGACCTGTGTTCTGAACTCTTGC
Nucleic acid sequence of TYRP1 Rv
   SEQ ID NO: 135: AAAAAAGTCGACACTGTCATCACTGGAGAGCA
Nucleic acid sequence of the anti-B2m gRNA
   SEQ ID NO: 136:

GGTCGTCAGCATGGCTCGCTGTTTTAGAGCTAGAAATAGCAAGTTAAAATAAGGCTAGTCCGTTATCA
ACTTGAAAAAGTGGCACCGAGTCGGTGCTTTTTTT

## Claims

1. A recombinant extra-cellular vesicle internalizing receptor (EVIR) comprising:
(i) an extracellular antibody domain specific for a membrane molecule of a cancer cell, wherein said membrane molecule is a cancer-associated antigen;
(ii) a proteinic domain to anchor the EVIR to the membrane of an antigen-presenting cell when expressed in said cell, said proteinic domain comprising at least one transmembrane and an intracellular domain of a protein being selected from the nerve growth factor receptor (NGFR), C-C chemokine receptor type 2 (CCR2), C-C chemokine receptor type 5 (CCR5), and P-selectin glycoprotein-1 ligand receptor (SELPLG); and
(iii) optionally, a cell membrane export domain increasing the export of the EVIR to the cellular membrane of an antigen-presenting cell when expressed in said cell.

2. An EVIR according to claim 1 wherein the extracellular antibody domain is a scFv.

3. An EVIR according to claims 1 or 2, wherein the extracellular antibody domain comprises a sequence of an antibody, or a fragment thereof, specific for human epidermal growth factor receptor 2 (HER2).

4. An EVIR according to claims 1 or 2, wherein the extracellular antibody domain comprises a sequence of an antibody, or a fragment thereof, specific for tyrosinase-related protein-1 (TYRP1), carcinoembryonic antigen (CEA), mesothelin, PMEL (gp100), gangliosides (GD2, GD3), or mucins.

5. An EVIR according to any one of claims 1 to 3, wherein the extracellular antibody domain comprises a sequence selected from SEQ ID NO: 27, SEQ ID NO: 28, SEQ ID NO: 29 and SEQ ID NO: 30 or a homologous variant thereof wherein said variant has an amino acid sequence which is at least 90% identical to the original amino acid sequence and which binds to and has specificity for said tumor-associated antigen.

6. An EVIR according to claim 4 wherein the extracellular antibody domain comprises a sequence of an antibody, or a fragment thereof, specific for tyrosinase-related protein-1 (TYRP1) or gangliosides (GD2).

7. An EVIR according to claim 6, wherein the extracellular antibody domain comprises a sequence selected from SEQ ID NO: 113 and SEQ ID NO: 114.

8. An EVIR according to any one of claims 1 to 7 wherein the proteinic domain is selected from sequences of SEQ ID NO: 31, SEQ ID NO: 35, SEQ ID NO: 39 and SEQ ID NO: 41 or a homologous variant thereof, wherein said variant has an amino acid sequence which is at least 90% identical to the original amino acid sequence, wherein said homologuous variants have unaffected anchoring ability to the cell membranes compared to the non-modified sequence.

9. An EVIR according to any one of claims 1 to 7 comprising a cell membrane export domain comprising an IgK domain.

10. An EVIR according to claim 8, comprising a cell membrane export domain comprising an IgK domain of SEQ ID NO 42 or a homologous derivative thereof, wherein said variant has an amino acid sequence which is at least 90% identical to the original amino acid sequence, wherein said homologuous variants have unaffected ability of sorting the EVIR to the cell membrane compared to the non-modified sequence.

11. An EVIR according to anyone of claims 1 to 3, 5, or 8 to 9 comprising an amino acid sequence selected from SEQ ID NO: 54, SEQ ID NO: 58, SEQ ID NO: 62and SEQ ID NO: 64 or a homologous variant thereof, wherein said variant has an amino acid sequence which is at least 90% identical to the original amino acid sequence.

12. An EVIR according to anyone of claims 1 to 2, 4, 6 to 7 or 8 to 9 comprising an amino acid sequence selected from SEQ ID NO: 132 and SEQ ID NO: 133 or a homologous variant thereof, wherein said variant has an amino acid sequence which is at least 90% identical to the original amino acid sequence.

13. An isolated nucleic acid sequence encoding an EVIR according to any one of claims 1 to 12.

14. An isolated nucleic acid according to claim 13 comprising a sequence selected from SEQ ID NO: 65, SEQ ID NO: 69, SEQ ID NO: 73, and SEQ ID NO: 75.

15. An isolated nucleic acid according to claim 14 comprising a sequence selected from SEQ ID NO: 130 and SEQ ID NO: 131.

16. A vector comprising at least one nucleic acid sequence of any one of claims 13 to 15.

17. A vector according to claim 16 further comprising a least one nucleic acid encoding for a functional protein that promotes survival, differentiation, proliferation, activation, maturation, phagocytosis, endocytosis, antigen-processing and presentation, T-cell recruitment in monocyte, macrophage and/or dendritic cells or a protein capable of inducing antigen presenting cell (APC) differentiation, survival, activation and/or cross-presentation or attracting and/or activating T cells.

18. An *ex vivo* method of inducing expression of at least one EVIR according to any of claims 1 to 12, in an antigen-presenting cell (APC) or a stem/progenitor cell thereof, comprising the steps of:
(i) *ex vivo* transducing said cell with a vector encoding at least one EVIR according to any of any one of claims 1 to 12; and
(ii) optionally inducing cell differentiation, maturation or activation.

19. A method according to claim 18 wherein the vector comprises a nucleic acid sequence according to claim 13 to 15 or is a vector according to claim 16 or 17.

20. An isolated antigen presenting cell (APC) or a stem or progenitor cell thereof expressing at least one EVIR according to any one of claims 1 to 12.

21. An *ex vivo* method of preparing EVIR-expressing, TAAs-presenting cells, comprising the steps of:
(i) providing at least one cancer cell or at least one cancer cell-derived extracellular vesicles (EV) obtained from a cancer subject;
(ii) providing an EVIR-expressing cell according to claim 20;
(iii)contacting, *ex vivo*, an EVIR-expressing cell provided under (ii) with said at least one cancer cell or EV provided under (i);
(iv)collecting the said EVIR-expressing cells from step (iii);
wherein cells obtained under (iv) have an enhanced ability to present T-cells tumor associated antigens (TAAs) from said cancer subject as compared to a cell not expressing an EVIR and treated as in (iii), once administered to said subject.

22. A pharmaceutical composition comprising at least one vector encoding at least one EVIR according to any of any one of claims 1 to 12 or cells according to claim 20 or cells obtainable from a method according to claims 18 or 21 and at least one pharmaceutically acceptable carrier, diluent or excipient thereof.

23. A pharmaceutical composition according to claim 22, wherein said composition is a vaccine composition.

24. A pharmaceutical composition according to claim 22 or 23 for use in the prevention and/or treatment of a cancer.

25. A kit comprising at least one EVIR according to any one of claims 1 to 12, or at least one recombinant expression vector according to claim 16 or 17, or at least one EVIR-expressing cell according to claim 20.

## Patentansprüche

1. Rekombinanter, extrazelluläre Vesikel internalisierender Rezeptor (EVIR), umfassend:
(i) eine extrazelluläre Antikörperdomäne, die für ein Membranmolekül einer Krebszelle spezifisch ist, wobei das Membranmolekül ein krebsassoziiertes Antigen ist;
(ii) eine Proteindomäne, um den EVIR an der Membran einer antigenpräsentierenden Zelle zu verankern, wenn er in der Zelle exprimiert wird, wobei die Proteindomäne mindestens eine Transmembran und eine intrazelluläre Domäne eines Proteins umfasst, die aus dem Nervenwachstumsfaktor-Rezeptor (NGFR), C-C-Chemokinrezeptor Typ 2 (CCR2), C-C-Chemokinrezeptor Typ 5 (CCR5) und P-Selektin-Glykoprotein-1-Ligand-Rezeptor (SELPLG) umfasst; und
(iii) optional eine Zellmembranexportdomäne, die den Export des EVIR zu der Zellmembran einer antigenpräsentierenden Zelle erhöht, wenn er in der Zelle exprimiert wird.

2. EVIR nach Anspruch 1, wobei die extrazelluläre Antikörperdomäne scFv ist.

3. EVIR nach den Ansprüchen 1 oder 2, wobei die extrazelluläre Antikörperdomäne eine Sequenz eines Antikörpers oder ein Fragment davon umfasst, die bzw. das für humanen epidermalen Wachstumsfaktor-Rezeptor 2 (HER2) spezifisch ist.

4. EVIR nach den Ansprüchen 1 oder 2, wobei die extrazelluläre Antikörperdomäne eine Sequenz eines Antikörpers oder ein Fragment davon umfasst, die bzw. das für Tyrosinase-bezogenes Protein-1 (TYRP1), karzinoembryonales Antigen (CEA), Mesothelin, PMEL (gp100), Ganglioside (GD2, GD3) oder Muzine spezifisch ist.

5. EVIR nach einem der Ansprüche 1 bis 3, wobei die extrazelluläre Antikörperdomäne eine Sequenz umfasst, die aus SEQ ID Nr. 27, SEQ ID Nr. 28, SEQ ID Nr. 29 und SEQ ID Nr. 30 oder einer homologen Variante davon ausgewählt ist, wobei die Variante eine Aminosäuresequenz aufweist, die zu mindestens 90 % zu der ursprünglichen Aminosäuresequenz identisch ist und die an das tumorassoziierte Antigen bindet und Spezifität für dieses aufweist.

6. EVIR nach Anspruch 4, wobei die extrazelluläre Antikörperdomäne eine Sequenz eines Antikörpers oder ein Fragment davon umfasst, die bzw. das für Tyrosinase-bezogenes Protein-1 (TYRP1) oder Ganglioside (GD2) spezifisch ist.

7. EVIR nach Anspruch 6, wobei die extrazelluläre Antikörperdomäne eine Sequenz umfasst, die aus SEQ ID Nr. 113 und SEQ ID Nr. 114 ausgewählt ist.

8. EVIR nach einem der Ansprüche 1 bis 7, wobei die Proteindomäne aus Sequenzen von SEQ ID Nr. 31, SEQ ID Nr. 35, SEQ ID Nr. 39 und SEQ ID Nr. 41 oder einer homologen Variante davon ausgewählt ist, wobei die Variante eine Aminosäuresequenz aufweist, die zu mindestens 90 % zu der ursprünglichen Aminosäuresequenz identisch ist, wobei die homologen Varianten eine unbeeinträchtigte Verankerungsfähigkeit an die Zellmembranen im Vergleich zu der nichtmodifizierten Sequenz aufweisen.

9. EVIR nach einem der Ansprüche 1 bis 7, umfassend eine Zellmembranexportdomäne, umfassend eine IgK-Domäne.

10. EVIR nach Anspruch 8, umfassend eine Zellmembranexportdomäne, umfassend eine IgK-Domäne von SEQ ID Nr. 42 oder ein homologes Derivat davon, wobei die Variante eine Aminosäuresequenz aufweist, die zu mindestens 90 % zu der ursprünglichen Aminosäuresequenz identisch ist, wobei die homologen Varianten eine unbeeinträchtigte Fähigkeit zum Sortieren des EVIR an der Zellmembran im Vergleich zu der nichtmodifizierten Sequenz aufweisen.

11. EVIR nach einem der Ansprüche 1 bis 3, 5 oder 8 bis 9, umfassend eine Aminosäuresequenz, die aus SEQ ID Nr. 54, SEQ ID Nr. 58, SEQ ID Nr. 62 und SEQ ID Nr. 64 oder einer homologen Variante davon ausgewählt ist, wobei die Variante eine Aminosäuresequenz aufweist, die zu mindestens 90 % zu der ursprünglichen Aminosäuresequenz identisch ist.

12. EVIR nach einem der Ansprüche 1 bis 2, 4, 6 bis 7 oder 8 bis 9, umfassend eine Aminosäuresequenz, die aus SEQ ID Nr. 132 und SEQ ID Nr. 133 oder einer homologen Variante davon ausgewählt ist, wobei die Variante eine Aminosäuresequenz aufweist, die zu mindestens 90 % zu der ursprünglichen Aminosäuresequenz identisch ist.

13. Isolierte Nukleinsäuresequenz, die einen EVIR nach einem der Ansprüche 1 bis 12 codiert.

14. Isolierte Nukleinsäure nach Anspruch 13, umfassend eine Sequenz, die aus SEQ ID Nr. 65, SEQ ID Nr. 69, SEQ ID Nr. 73 und SEQ ID Nr. 75 ausgewählt ist.

15. Isolierte Nukleinsäure nach Anspruch 14, umfassend eine Sequenz, die aus SEQ ID Nr. 130 und SEQ ID Nr. 131 ausgewählt ist.

16. Vektor, umfassend mindestens eine Nukleinsäuresequenz nach einem der Ansprüche 13 bis 15.

17. Vektor nach Anspruch 16, ferner umfassend mindestens eine Nukleinsäure, die für ein funktionales Protein, das Überleben, Differenzierung, Proliferation, Aktivierung, Reifung, Phagozytose, Endozytose, Antigenverarbeitung und - präsentation, T-Zell-Rekrutierung in Monozyten-, Makrophagen- und/oder dendritischen Zellen fördert, oder ein Protein codiert, das dazu in der Lage ist, Differenzierung, Überleben, Aktivierung und/oder Kreuzpräsentation von antigenpräsentierenden Zellen (APZ) oder Anziehung und/oder Aktivierung von T-Zellen zu induzieren.

18. *Ex-vivo*-Verfahren zur Induktion einer Expression mindestens eines EVIR nach einem der Ansprüche 1 bis 12 in einer antigenpräsentierenden Zelle (APZ) oder einer Stamm-/Vorläuferzelle davon, umfassend die Schritte:
(i) Transduzieren der Zelle *ex vivo* mit einem Vektor, der mindestens einen EVIR nach einem der Ansprüche 1 bis 12 codiert; und
(ii) optionales Induzieren von Zelldifferenzierung, - reifung oder -aktivierung.

19. Verfahren nach Anspruch 18, wobei der Vektor eine Nukleinsäuresequenz nach Anspruch 13 bis 15 umfasst oder ein Vektor nach Anspruch 16 oder 17 ist.

20. Isolierte antigenpräsentierende Zelle (APZ) oder Stamm- oder Vorläuferzelle davon, die mindestens einen EVIR nach einem der Ansprüche 1 bis 12 exprimiert.

21. *Ex-vivo*-Verfahren zur Herstellung von EVIR-exprimierenden, TAA-präsentierenden Zellen, umfassend die Schritte:
(i) Bereitstellen mindestens einer Krebszelle oder mindestens eines von Krebszellen abgeleiteten extrazellulären Vesikels (EV), die bzw. das von einem Krebsprobanden erhalten wird;
(ii) Bereitstellen einer EVIR-exprimierenden Zelle nach Anspruch 20;
(iii) Inkontaktbringen, *ex-vivo*, einer unter (ii) bereitgestellten EVIR-exprimierenden Zelle mit der bzw. dem mindestens einen unter (i) bereitgestellten Krebszelle oder EV;
(iv) Sammeln der besagten EVIR-exprimierenden Zellen aus Schritt (iii);
wobei unter (iv) erhaltene Zellen eine verstärkte Fähigkeit zum Präsentieren von tumorassoziierten Antigenen (TAA) von T-Zellen von dem Krebsprobanden im Vergleich zu einer Zelle, die keinen EVIR exprimiert und wie in (iii) behandelt wird, einmal an den Probanden verabreicht.

22. Pharmazeutische Zusammensetzung, umfassend mindestens einen Vektor, der mindestens einen EVIR nach einem der Ansprüche 1 bis 12 codiert, oder Zellen nach Anspruch 20 oder Zellen, die aus einem Verfahren nach den Ansprüchen 18 oder 21 erhalten werden können, und mindestens einen pharmazeutisch unbedenklichen Trägerstoff, ein pharmazeutisch unbedenkliches Verdünnungsmittel oder einen pharmazeutisch unbedenklichen Hilfsstoff davon.

23. Pharmazeutische Zusammensetzung nach Anspruch 22, wobei die Zusammensetzung eine Impfstoffzusammensetzung ist.

24. Pharmazeutische Zusammensetzung nach Anspruch 22 oder 23 zur Verwendung bei der Prävention und/oder Behandlung eines Krebses.

25. Kit, umfassend mindestens einen EVIR nach einem der Ansprüche 1 bis 12 oder mindestens einen rekombinanten Expressionsvektor nach Anspruch 16 oder 17 oder mindestens eine EVIR-exprimierende Zelle nach Anspruch 20.

## Revendications

1. Récepteur d'internalisation de vésicule extracellulaire (EVIR) recombinant comprenant :
(i) un domaine d'anticorps extracellulaire spécifique d'une molécule de membrane d'une cellule cancéreuse, dans lequel ladite molécule de membrane est un antigène associé à un cancer ;
(ii) un domaine protéinique pour ancrer l'EVIR à la membrane d'une cellule présentatrice d'antigène lorsqu'il est exprimé dans ladite cellule, ledit domaine protéinique comprenant au moins un domaine transmembranaire ainsi qu'un domaine intracellulaire d'une protéine qui est choisie parmi le récepteur du facteur de croissance du tissu nerveux (NGFR), le récepteur de chimiokine C-C de type 2 (CCR2), le récepteur de chimiokine C-C de type 5 (CCR5) et le récepteur-ligand 1 glycoprotéique 1 de la sélectine P (SELPLG) ; et
(iii) éventuellement un domaine d'exportation vers la membrane cellulaire augmentant l'exportation de l'EVIR vers la membrane cellulaire d'une cellule présentatrice d'antigène lorsqu'il est exprimé dans ladite cellule.

2. EVIR selon la revendication 1, dans lequel le domaine d'anticorps extracellulaire est un scFv.

3. EVIR selon la revendication 1 ou 2, dans lequel le domaine d'anticorps extracellulaire comprend une séquence d'un anticorps, ou d'un fragment de celui-ci, spécifique du récepteur 2 du facteur de croissance de l'épiderme humain (HER2).

4. EVIR selon la revendication 1 ou 2, dans lequel le domaine d'anticorps extracellulaire comprend une séquence d'un anticorps, ou d'un fragment de celui-ci, spécifique de la protéine 1 apparentée à la tyrosinase (TYRP1), d'un antigène carcinoembryonnaire (CEA), de la mésothéline, de la PMEL (gp100), des gangliosides (GD2, GD3), ou des mucines.

5. EVIR selon l'une quelconque des revendications 1 à 3, dans lequel le domaine d'anticorps extracellulaire comprend une séquence choisie parmi les SEQ ID NO : 27, SEQ ID NO : 28, SEQ ID NO : 29 et SEQ ID NO : 30 ou un variant homologue d'une telle séquence, dans lequel ledit variant a une séquence d'acides aminés qui est identique à au moins 90 % à la séquence d'acides aminés d'origine et qui se lie à et a une spécificité pour ledit antigène associé à une tumeur.

6. EVIR selon la revendication 4, dans lequel le domaine d'anticorps extracellulaire comprend une séquence d'un anticorps, ou d'un fragment de celui-ci, spécifique de la protéine 1 apparentée à la tyrosinase (TYRP1) ou des gangliosides (GD2).

7. EVIR selon la revendication 6, dans lequel le domaine d'anticorps extracellulaire comprend une séquence choisie parmi les SEQ ID NO : 113 et SEQ ID NO : 114.

8. EVIR selon l'une quelconque des revendications 1 à 7, dans lequel le domaine protéinique est choisi parmi les séquences des SEQ ID NO : 31, SEQ ID NO : 35, SEQ ID NO : 39 et SEQ ID NO : 41 ou un variant homologue d'une telle séquence, dans lequel ledit variant a une séquence d'acides aminés qui est identique à au moins 90 % à la séquence d'acides aminés d'origine, dans lequel lesdits variants homologues ont une aptitude d'ancrage aux membranes cellulaires non affectée en comparaison avec la séquence non modifiée.

9. EVIR selon l'une quelconque des revendications 1 à 7, comprenant un domaine d'exportation vers la membrane cellulaire comprenant un domaine d'IgK.

10. EVIR selon la revendication 8, comprenant un domaine d'exportation vers la membrane cellulaire comprenant un domaine d'IgK de la SEQ ID NO : 42 ou un dérivé homologue de celui-ci, dans lequel ledit variant a une séquence d'acides aminés qui est identique à au moins 90 % à la séquence d'acides aminés d'origine, dans lequel lesdits variants homologues ont une aptitude d'adressage de l'EVIR à la membrane cellulaire non affectée en comparaison avec la séquence non modifiée.

11. EVIR selon l'une quelconque des revendications 1 à 3, 5, 8 et 9, comprenant une séquence d'acides aminés choisie parmi les SEQ ID NO : 54, SEQ ID NO : 58, SEQ ID NO : 62 et SEQ ID NO : 64 ou un variant homologue d'une telle séquence, dans lequel ledit variant a une séquence d'acides aminés qui est identique à au moins 90 % à la séquence d'acides aminés d'origine.

12. EVIR selon l'une quelconque des revendications 1, 2, 4, 6, 7, 8 et 9, comprenant une séquence d'acides aminés choisie parmi les SEQ ID NO : 132 et SEQ ID NO : 133 ou un variant homologue d'une telle séquence, dans lequel ledit variant a une séquence d'acides aminés qui est identique à au moins 90 % à la séquence d'acides aminés d'origine.

13. Séquence d'acide nucléique isolé codant un EVIR selon l'une quelconque des revendications 1 à 12.

14. Acide nucléique isolé selon la revendication 13, comprenant une séquence choisie parmi les SEQ ID NO : 65, SEQ ID NO : 69, SEQ ID NO : 73 et SEQ ID NO : 75.

15. Acide nucléique isolé selon la revendication 14, comprenant une séquence choisie parmi les SEQ ID NO : 130 et SEQ ID NO : 131.

16. Vecteur comprenant au moins une séquence d'acide nucléique selon l'une quelconque des revendications 13 à 15.

17. Vecteur selon la revendication 16, comprenant en outre au moins un acide nucléique codant une protéine fonctionnelle qui favorise la survie, la différenciation, la prolifération, l'activation, la maturation, la phagocytose, l'endocytose, l'apprêtement et la présentation d'antigène, le recrutement de cellules T au sein des monocytes, macrophages et/ou cellules dendritiques ou une protéine capable d'induire la différenciation, la survie, l'activation et/ou la présentation croisée des cellules présentatrices d'antigène (APC), ou d'attirer et/ou d'activer des cellules T.

18. Méthode *ex vivo* d'induction de l'expression d'au moins un EVIR selon l'une quelconque des revendications 1 à 12, dans une cellule présentatrice d'antigène (APC) ou une cellule souche/progénitrice de celle-ci, comprenant les étapes suivantes :
(i) transduction *ex vivo* de ladite cellule avec un vecteur codant au moins un EVIR selon l'une quelconque des revendications 1 à 12 ; et
(ii) éventuellement induction d'une différenciation, maturation ou activation de la cellule.

19. Méthode selon la revendication 18, dans laquelle le vecteur comprend une séquence d'acides nucléiques selon les revendications 13 à 15 ou est un vecteur selon la revendication 16 ou 17.

20. Cellule présentatrice d'antigène (APC) isolée ou une cellule souche ou progénitrice de celle-ci exprimant au moins un EVIR selon l'une quelconque des revendications 1 à 12.

21. Méthode *ex vivo* de préparation de cellules présentatrices de TAA exprimant un EVIR, comprenant les étapes suivantes :
(i) fourniture d'au moins une cellule cancéreuse ou d'au moins une vésicule extracellulaire (EV) dérivée de cellule cancéreuse obtenue auprès d'un sujet cancéreux ;
(ii) fourniture d'une cellule exprimant un EVIR selon la revendication 20 ;
(iii) mise en contact, *ex vivo*, d'une cellule exprimant un EVIR obtenue en (ii) avec ladite au moins une cellule cancéreuse ou EV obtenue en (i) ;
(iv) collecte desdites cellules exprimant un EVIR de l'étape (iii) ;
dans laquelle les cellules obtenues en (iv) ont une aptitude à présenter à des cellules T des antigènes associés à une tumeur (TAA) provenant dudit sujet cancéreux, meilleure que celle d'une cellule n'exprimant pas d'EVIR et traitée comme en (iii), une fois qu'elles ont été administrées audit sujet.

22. Composition pharmaceutique comprenant au moins un vecteur codant au moins un EVIR selon l'une quelconque des revendications 1 à 12 ou des cellules selon la revendication 20 ou des cellules pouvant être obtenues par une méthode selon la revendication 18 ou 21 et au moins un véhicule, diluant ou excipient pour ceux-ci, pharmaceutiquement acceptable.

23. Composition pharmaceutique selon la revendication 22, laquelle composition est une composition de vaccin.

24. Composition pharmaceutique selon la revendication 22 ou 23, pour une utilisation dans la prévention et/ou le traitement d'un cancer.

25. Trousse comprenant au moins un EVIR selon l'une quelconque des revendications 1 à 12, ou au moins un vecteur d'expression recombinant selon la revendication 16 ou 17, ou au moins une cellule exprimant un EVIR selon la revendication 20.
